# EUROPÄISCHE PATENTSCHRIFT

(11) **EP 3 941 920 B1**
(45) Veröffentlichungstag und Bekanntmachung des Hinweises auf die Patenterteilung: **20.03.2024**
(21) Anmeldenummer: 20711173.3
(22) Anmeldetag: 17.03.2020
(51) Int. Cl.: C07D 471/04, C07D 471/14, C07D 487/04, C07D 491/14, C07D 495/14, C07D 513/14, C07F 5/02, C07F 9/576, C09K 11/06

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 20.03.2019 EP 19163999
(43) Veröffentlichungstag der Anmeldung: 26.01.2022
(73) Patentinhaber: Merck Patent GmbH, 64293 Darmstadt (DE)
(72) Erfinder: PARHAM, Amir Hossain, 60486 FRANKFURT AM MAIN (DE); KROEBER, Jonas Valentin, 60311 FRANKFURT AM MAIN (DE); ENGELHART, Jens, 64285 DARMSTADT (DE); JATSCH, Anja, 60489 FRANKFURT AM MAIN (DE); EICKHOFF, Christian, 68259 MANNHEIM (DE); EHRENREICH, Christian, 64285 DARMSTADT (DE)
(74) Vertreter: Merck Patent Association
(86) Internationale Anmeldenummer: PCT/EP2020/057170
(87) Internationale Veröffentlichungsnummer: WO 2020/187865

(56) Entgegenhaltungen:
- WO-A1-2005/111034
- WO-A1-2017/078182
- WO-A1-2018/080068
- WO-A1-2019/081391
- Wolfgang Stadlbauer ET AL: "Monatshefte fiir Chemie Chemical Monthly Synthese yon Indolen und Isochinolonen aus Phenylmalonylheteroeyclen", ~onatshefte fiir Chemie, 1984, Seiten 467-475, XP055693657, Gefunden im Internet: URL:https://link.springer.com/content/pdf/ 10.1007/BF00810008.pdf [gefunden am 2020-05-12]
- Christa E Mü ET AL: "Chiral Pyrrolo[2,3-d]pyrimidine and Pyrimido[4,5-b]indole Derivatives: Structure-Activity Relationships of Potent, Highly Stereoselective A 1 -Adenosine Receptor Antagonists", J. Med. Chem., 1996, Seiten 2482-2491, XP055693672, Gefunden im Internet: URL:https://pubs.acs.org/doi/pdf/10.1021/j m960011w [gefunden am 2020-05-11]

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig phosphoreszierende metallorganische Komplexe eingesetzt. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter oder als Elektronentransportmaterial, und dort zu verbesserten Eigenschaften führen. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen und sich gut für die Verwendung in OLEDs eignen. Dabei weisen die OLEDs insbesondere eine lange Lebensdauer, eine hohe Effizienz und eine geringe Betriebsspannung auf. Diese Verbindungen sowie elektronische Vorrichtungen, insbesondere organische Elektrolumineszenzvorrichtungen, welche diese Verbindungen enthalten, sind daher der Gegenstand der vorliegenden Erfindung.

Gegenstand der vorliegenden Erfindung ist eine Verbindung gemäß Formel (1), 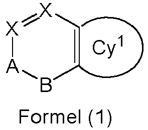 wobei für die verwendeten Symbole gilt:
A, B sind jeweils ausgewählt aus der Gruppe bestehend aus NAr¹, C=O, C=S, C=NR, BR, PR, P(=O)R, SO und SO₂, mit der Maßgabe, dass eines der Symbole A und B für NAr¹ steht und das andere der Symbole A und B für C=O, C=S, C=NR, BR, PR, P(=O)R, SO oder SO₂ steht;
Cy¹ ist zusammen mit den beiden explizit eingezeichneten Kohlenstoffatomen eine Gruppe der folgenden Formel (2), 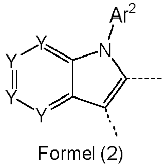 wobei die gestrichelten Bindungen die Verknüpfung dieser Gruppe in der Formel (1) kennzeichnen;
X ist gleich oder verschieden bei jedem Auftreten CR' oder N; oder die beiden Gruppen X stehen für eine Gruppe der folgenden Formel (3), 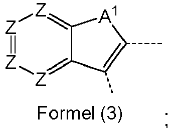
   - Y, Z: ist gleich oder verschieden bei jedem Auftreten CR oder N;
   - A¹: ist gleich oder verschieden bei jedem Auftreten NAr³, O, S oder C(R)₂;
   - Ar¹, Ar², Ar³: ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann; dabei kann Ar¹ mit einem benachbarten Rest R` ein Ringsystem bilden;
   - R, R`: ist bei jedem Auftreten gleich oder verschieden H, D, F, Cl, Br, I, N(Ar')₂, N(R¹)₂, OAr', SAr', CN, NO₂, OR¹, SR¹, C(=O)N(R¹)₂ Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann; dabei können zwei Reste R auch miteinander ein aliphatisches, heteroaliphatisches, aromatisches oder heteroaromatisches Ringsystem bilden; weiterhin können zwei Reste R' auch miteinander ein aliphatisches oder heteroaliphatisches Ringsystem bilden; weiterhin kann R' mit einem benachbarten Rest Ar¹ ein Ringsystem bilden;
   - Ar': ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
   - R¹: ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein aliphatisches Ringsystem bilden;
   - R²: ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome, bevorzugt 6 bis 40 C-Atome im Ringsystem. Ein heteroaromatisches Ringsystem im Sinne dieser Erfindung enthält 2 bis 60 C-Atome, bevorzugt 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. Ebenso sollen hierunter Systeme verstanden werden, in denen zwei oder mehr Aryl- bzw. Heteroarylgruppen direkt miteinander verknüpft sind, wie z. B. Biphenyl, Terphenyl, Bipyridin oder Phenylpyridin. So sollen beispielsweise auch Systeme wie Fluoren, 9,9`-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugte aromatische bzw. heteroaromatische Ringsysteme sind einfache Aryl- bzw. Heteroarylgruppen sowie Gruppen, in denen zwei oder mehr Aryl- bzw. Heteroarylgruppen direkt miteinander verknüpft sind, beispielsweise Biphenyl oder Bipyridin, sowie Fluoren oder Spirobifluoren.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe OR¹ mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe SR¹ mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, besonders bevorzugt F oder CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter der Formulierung, dass zwei oder mehr Reste miteinander ein Ringsystem bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

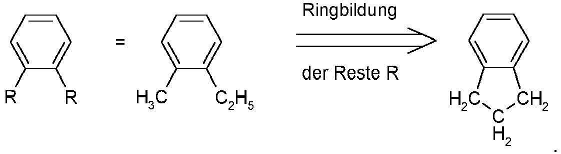

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

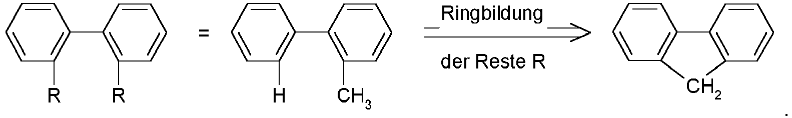

Je nach Orientierung der Gruppe der Formel (2) ergeben sich unterschiedliche Isomere, wie nachfolgend durch die Formeln (4) und (5) dargestellt, 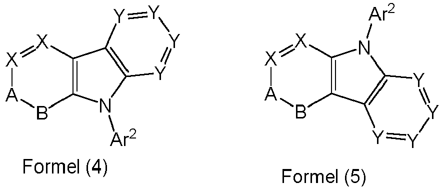 wobei die verwendeten Symbole die oben genannten Bedeutungen aufweisen.

In einer bevorzugten Ausführungsform der Erfindung steht eine der Gruppen A und B für NAr¹, und die andere der Gruppen A und B steht für C=O, P(=O)R, BR oder SO₂. Besonders bevorzugt steht eine der Gruppen A und B für NAr¹, und die andere der Gruppen A und B steht für C=O.

Bevorzugte Ausführungsformen der Verbindungen der Formel (4) sind somit die Verbindungen der folgenden Formeln (4a) und (4b), und bevorzugte Ausführungsformen der Verbindungen der Formel (5) sind die Verbindungen der folgenden Formeln (5a) und (5b), 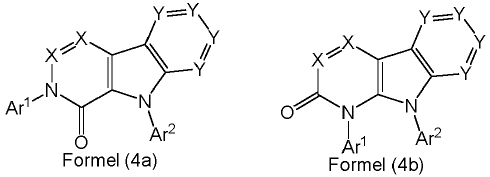 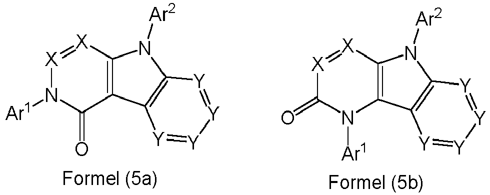 wobei die verwendeten Symbole die oben genannten Bedeutungen aufweisen.

In einer bevorzugten Ausführungsform der Erfindung steht maximal ein Symbol X für N und das andere Symbol X steht für CR`. In einer besonders bevorzugten Ausführungsform der Erfindung stehen beide Symbole X gleich oder verschieden für CR`. Besonders bevorzugt sind also die Verbindungen der folgenden Formeln (4a-1), (4b-1), (5a-1) und (5b-1), 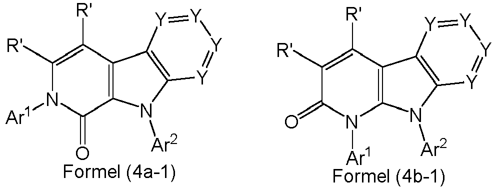 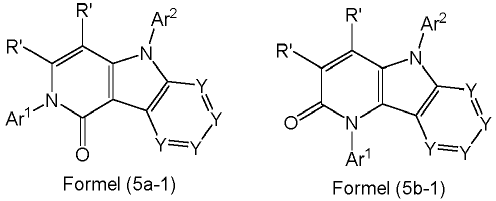 wobei die verwendeten Symbole die oben genannten Bedeutungen aufweisen.

In einer weiteren bevorzugten Ausführungsform der Erfindung steht maximal ein Symbol Y für N und die weiteren Symbole Y stehen für CR. In einer besonders bevorzugten Ausführungsform der Erfindung stehen alle Symbole Y für CR. Besonders bevorzugt sind also die Verbindungen der folgenden Formeln (4a-2), (4b-2), (5a-2) und (5b-2), 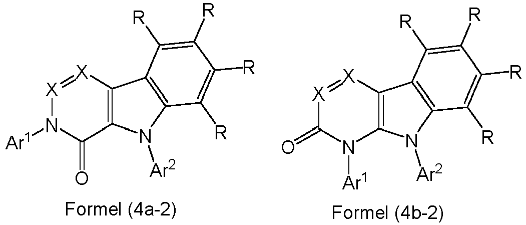 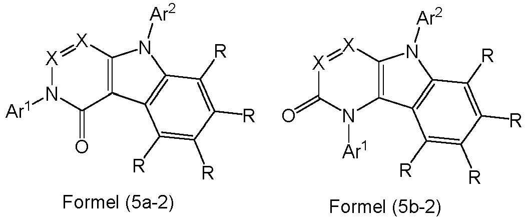 wobei die verwendeten Symbole die oben genannten Bedeutungen aufweisen.

Besonders bevorzugt treten die oben genannten Bevorzugungen für X und Y gleichzeitig auf, so dass Strukturen der folgenden Formeln (4a-3), (4b-3), (5a-3) und (5b-3) besonders bevorzugt sind, 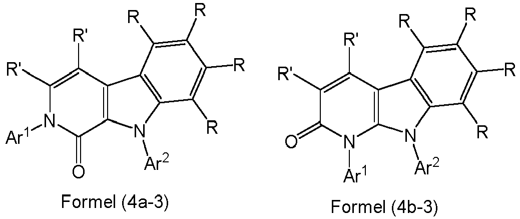 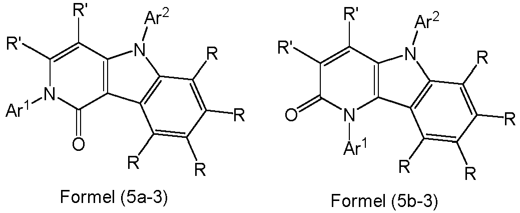 wobei die verwendeten Symbole die oben genannten Bedeutungen aufweisen.

In einer bevorzugten Ausführungsform der Erfindung stehen maximal zwei Reste R, besonders bevorzugt maximal ein Rest R in der Verbindung der Formel (1) bzw. den oben aufgeführten bevorzugten Strukturen für eine Gruppe ungleich Wasserstoff. Ganz besonders bevorzugt sind die Verbindungen der folgenden Formeln (4a-4), (4b-4), (5a-4) und (5b-4), 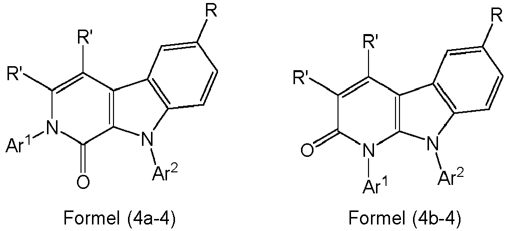 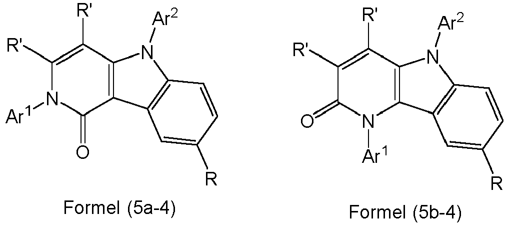 wobei die verwendeten Symbole die oben genannten Bedeutungen aufweisen.

In einer weiteren Ausführungsform der Erfindung stehen die beiden Gruppen X für eine Gruppe der Formel (3). Dabei steht in der Gruppe der Formel (3) das Symbol A¹ bevorzugt für NAr³. Wenn die beiden Gruppen X für eine Gruppe der Formel (3) stehen, sind bevorzugte Ausführungsformen der Formel (4) die Verbindungen der folgenden Formeln (6) und (7) und bevorzugte Ausführungsformen der Formel (5) die Verbindungen der folgenden Formeln (8) und (9), 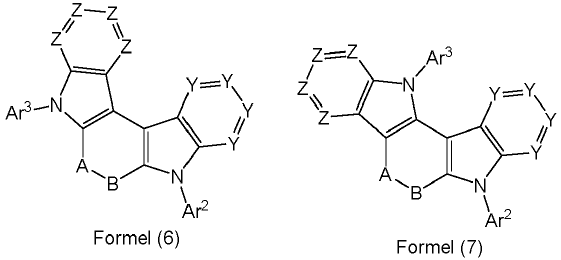 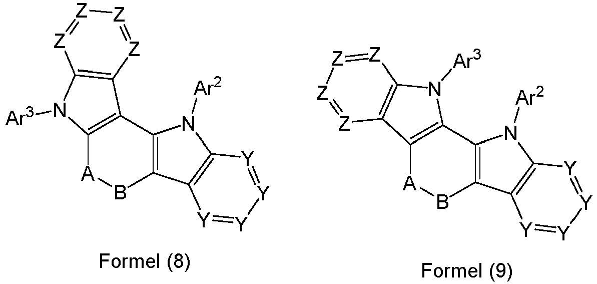 wobei die verwendeten Symbole die oben genannten Bedeutungen aufweisen.

Dabei steht in Formel (6) bis (9) bevorzugt maximal eine Gruppe Y für N und die anderen Gruppen Y stehen gleich oder verschieden für CR. Besonders bevorzugt stehen alle Gruppen Y gleich oder verschieden für CR.

In einer weiteren bevorzugten Ausführungsform der Erfindung steht maximal eine Gruppe Z für N, und die anderen Gruppen Z stehen gleich oder verschieden für CR. Besonders bevorzugt stehen alle Gruppen Z gleich oder verschieden für CR.

Besonders bevorzugt treten die oben genannten Bevorzugungen für Y und Z in den Formeln (6) bis (9) gleichzeitig auf, so dass die Verbindungen der folgenden Formeln (6-1) bis (9-1) besonders bevorzugt sind, 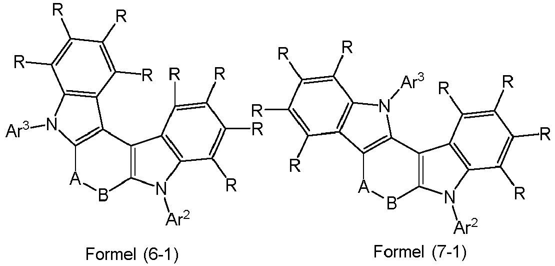 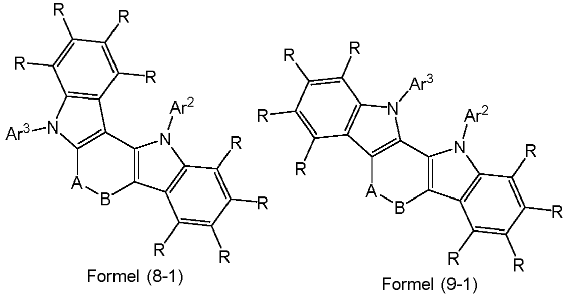 wobei die verwendeten Symbole die oben genannten Bedeutungen aufweisen.

Dabei gilt für die Formeln (6) bis (9) und (6-1) bis (9-1), dass bevorzugt eine der Gruppen A und B für NAr¹ steht und die andere der Gruppen A und B für C=O steht. Besonders bevorzugt sind daher die Strukturen der folgenden Formeln (6-2) bis (9-2), 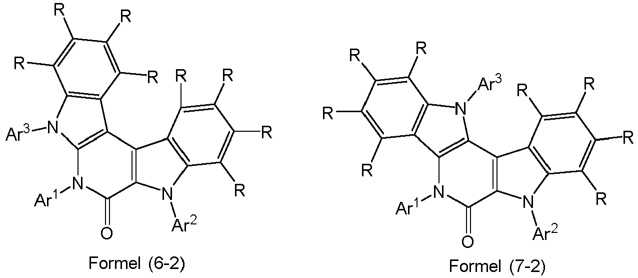 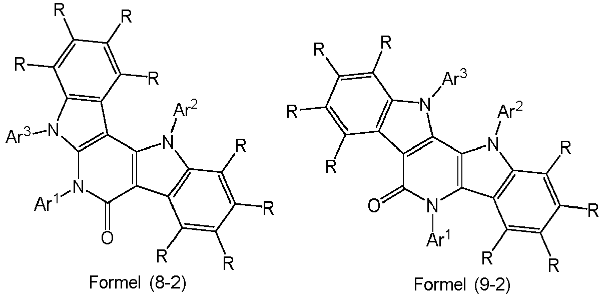 wobei die verwendeten Symbole die oben genannten Bedeutungen aufweisen.

In einer bevorzugten Ausführungsform der Erfindung stehen insgesamt maximal drei Reste R, besonders bevorzugt maximal zwei Reste R und ganz besonders bevorzugt maximal ein Rest R für eine Gruppe ungleich Wasserstoff. Besonders bevorzugt sind die Verbindungen der folgenden Formeln (6-3) bis (9-3), 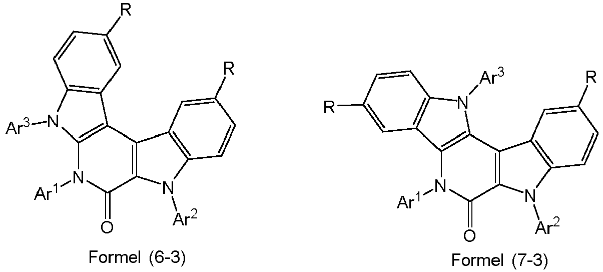 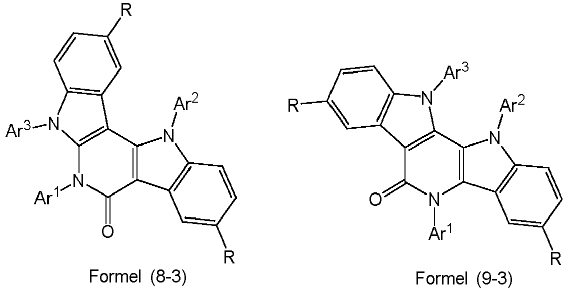 wobei die verwendeten Symbole die oben genannten Bedeutungen aufweisen.

In einer weiteren Ausführungsform der Erfindung steht der Rest Ar¹ für eine Phenylgruppe und der Substituent R', der benachbart zu der Gruppe Ar¹ steht, ist ebenfalls eine Phenylgruppe, wobei die beiden Phenylgruppen miteinander ein Ringsystem bilden. Entsprechende Ausführungsformen der Formeln (4) und (5) sind somit die Verbindungen der folgenden Formeln (10) und (11), 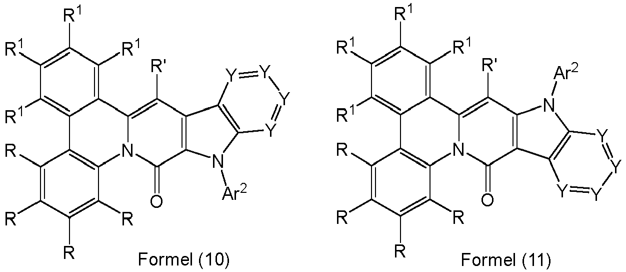 wobei die verwendeten Symbole die oben genannten Bedeutungen aufweisen.

In einer bevorzugten Ausführungsform der Formeln (10) und (11) steht maximal ein Symbol Y für N und die anderen Symbole Y stehen gleich oder verschieden für CR. Besonders bevorzugt stehen alle Symbole Y gleich oder verschieden für CR. Besonders bevorzugt sind somit die Verbindungen der folgenden Formeln (10-1) und (11-1), 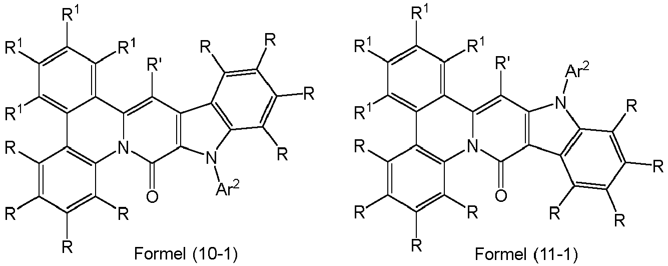 wobei die verwendeten Symbole die oben genannten Bedeutungen aufweisen.

Besonders bevorzugt sind die Verbindungen der folgenden Formeln (10-2) und (11-2), 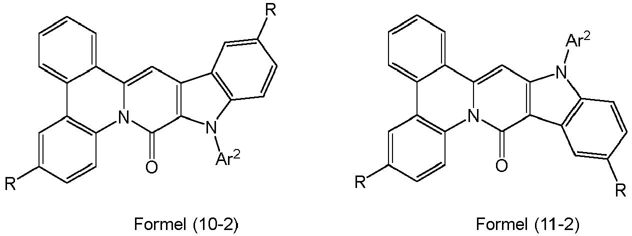 wobei die verwendeten Symbole die oben genannten Bedeutungen aufweisen.

Im Folgenden werden bevorzugte Substituenten Ar¹, Ar², Ar³, R, R', Ar`, R¹ und R² an den erfindungsgemäßen Verbindungen beschrieben. In einer besonders bevorzugten Ausführungsform der Erfindung treten die nachfolgend genannten Bevorzugungen für Ar¹, Ar², Ar³, R, R', Ar', R¹ und R² gleichzeitig auf und gelten für die Strukturen der Formel (1) sowie für alle oben aufgeführten bevorzugten Ausführungsformen.

In einer bevorzugten Ausführungsform der Erfindung sind Ar¹, Ar² und Ar³ bei jedem Auftreten gleich oder verschieden ein aromatisches oder heteroaromatisches Ringsystem mit 6 bis 30 aromatischen Ringatomen, das durch einen oder mehrere Reste R substituiert sein kann. Besonders bevorzugt sind Ar¹, Ar² und Ar³ bei jedem Auftreten gleich oder verschieden ein aromatisches oder heteroaromatisches Ringsystem mit 6 bis 24 aromatischen Ringatomen, insbesondere mit 6 bis 12 aromatischen Ringatomen, das durch einen oder mehrere, bevorzugt nicht-aromatische, Reste R substituiert sein kann. Wenn Ar¹, Ar² bzw. Ar³ für eine Heteroarylgruppe, insbesondere für Triazin, Pyrimidin, Chinazolin oder Carbazol steht, können auch aromatische oder heteroaromatische Substituenten R an dieser Heteroarylgruppe bevorzugt sein. Weiterhin kann es bevorzugt sein, wenn Ar¹, Ar² bzw. Ar³ mit einer Gruppe N(Ar')₂ substituiert ist, so dass der Substituent Ar¹, Ar² bzw. Ar³ insgesamt eine Triarylamin- bzw. Triheteroarylamin-Gruppe darstellt.

Geeignete aromatische bzw. heteroaromatische Ringsysteme Ar¹, Ar² bzw. Ar³ sind bei jedem Auftreten gleich oder verschieden ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R, bevorzugt nichtaromatischen Resten R, substituiert sein können. Wenn Ar¹, Ar² bzw. Ar³ für eine Heteroarylgruppe, insbesondere für Triazin, Pyrimidin, Chinazolin oder Carbazol steht, können auch aromatische oder heteroaromatische Reste R an dieser Heteroarylgruppe bevorzugt sein.

Dabei sind Ar¹, Ar² und Ar³ bevorzugt bei jedem Auftreten gleich oder verschieden gewählt aus den Gruppen der folgenden Formeln Ar-1 bis Ar-83, 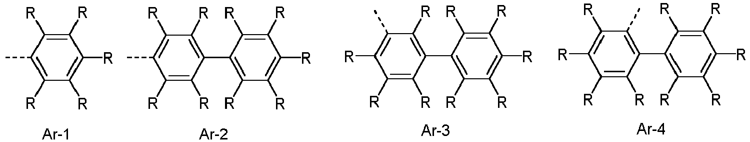 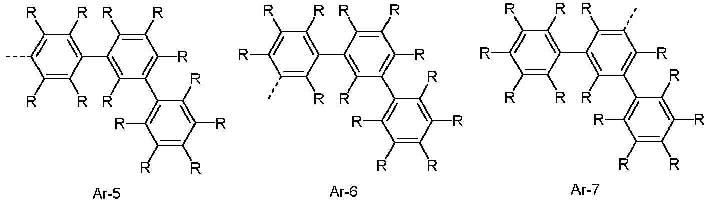 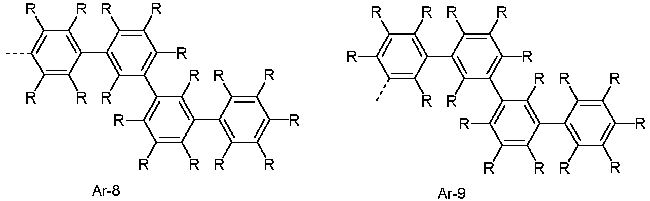 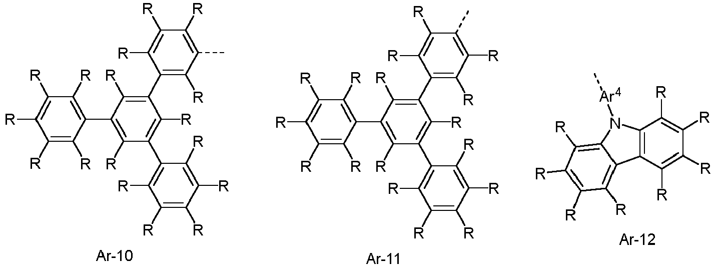 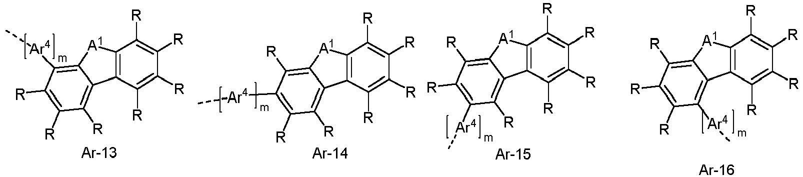 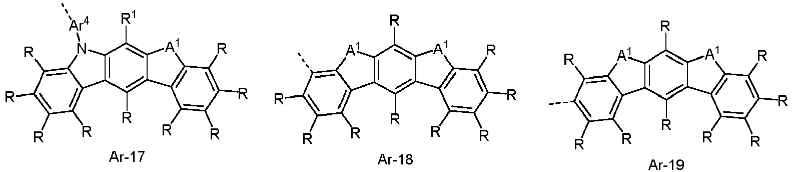 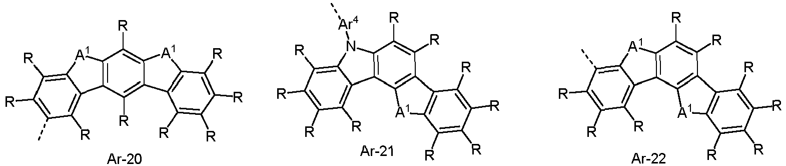 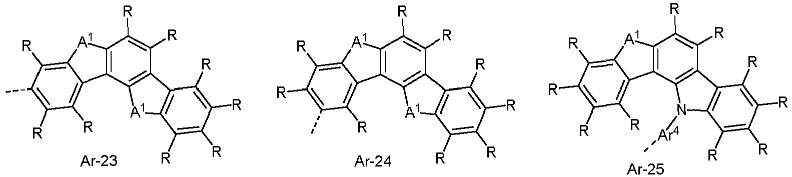 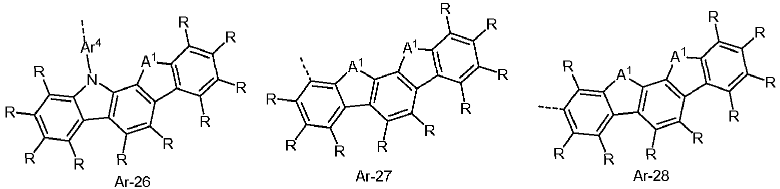 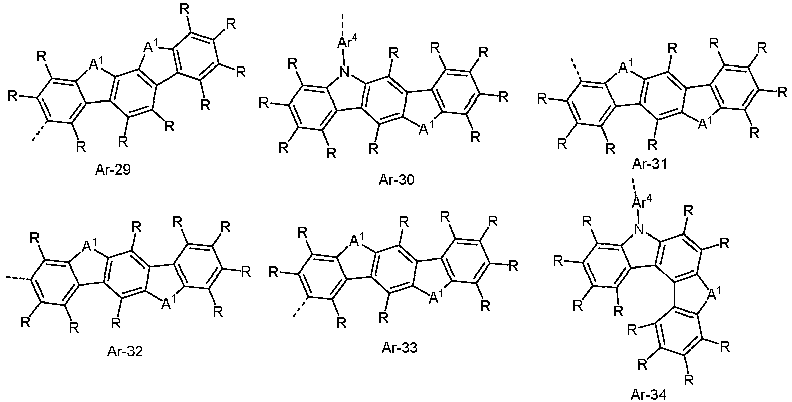 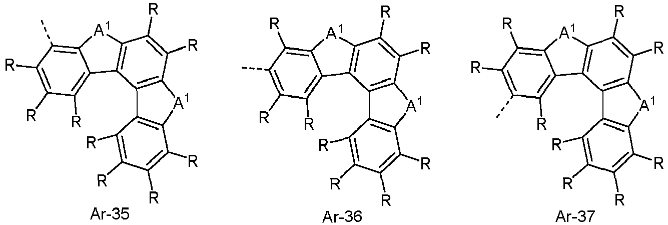 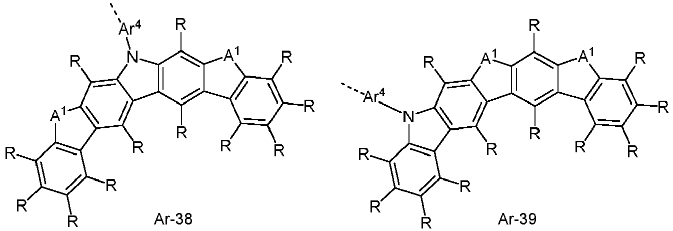 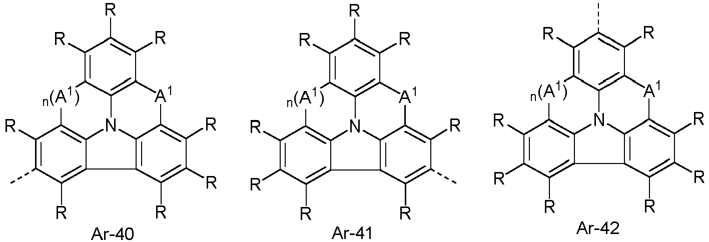 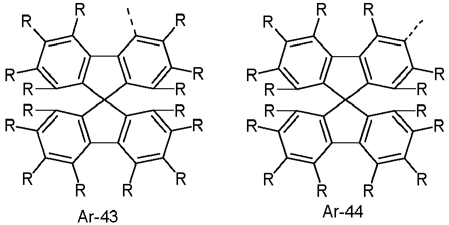 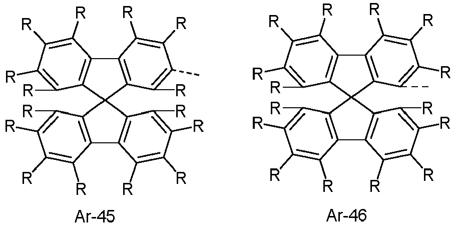 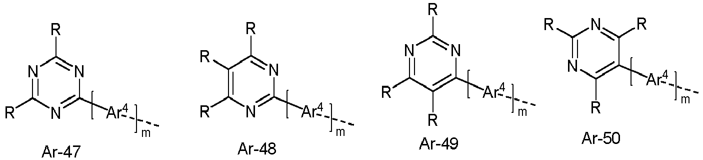 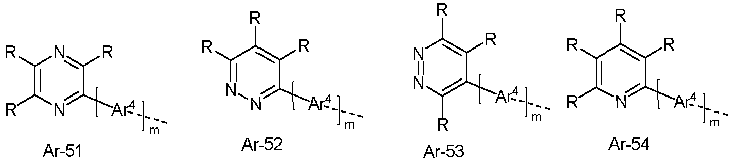 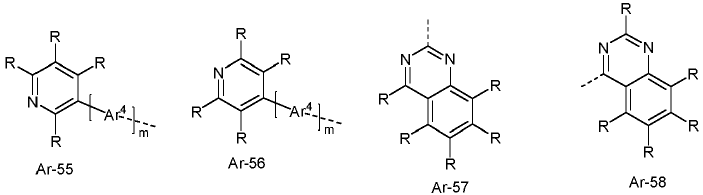 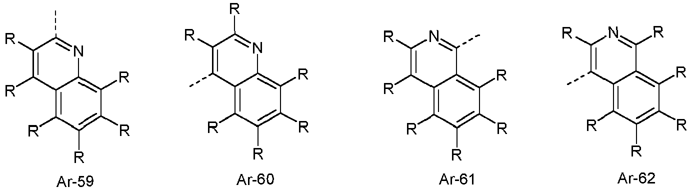 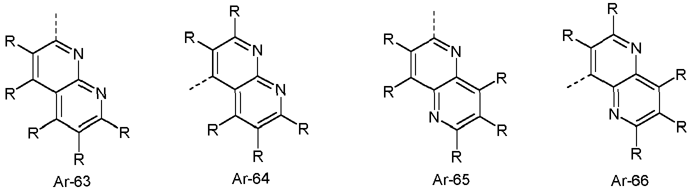 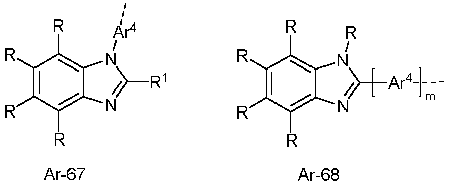 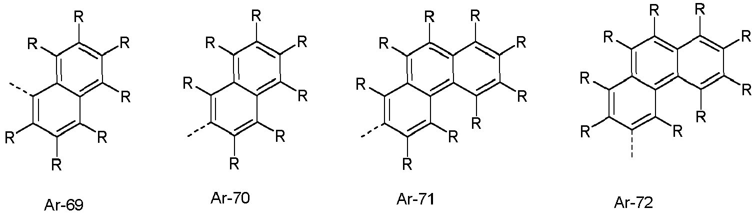 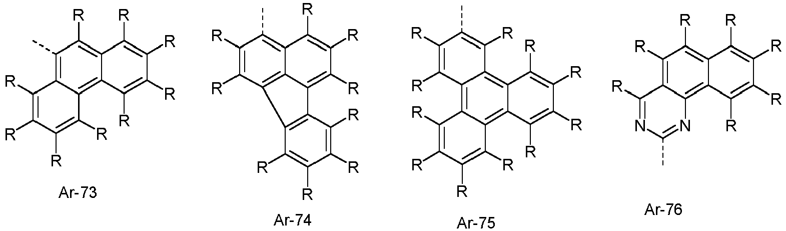 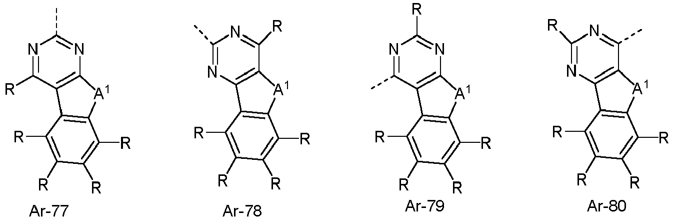 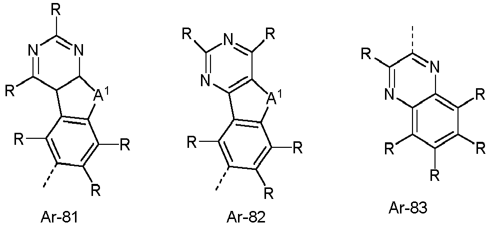 wobei R und A¹ die oben genannten Bedeutungen aufweisen, die gestrichelte Bindung die Bindung an das Stickstoffatom darstellt und weiterhin gilt:
- Ar⁴: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;
- n: ist 0 oder 1, wobei n = 0 bedeutet, dass an dieser Position keine Gruppe A¹ gebunden ist und an die entsprechenden Kohlenstoffatome statt dessen Reste R gebunden sind;
- m: ist 0 oder 1, wobei m = 0 bedeutet, dass die Gruppe Ar⁴ nicht vorhanden ist und dass die entsprechende aromatische bzw. heteroaromatische Gruppe direkt an das Stickstoffatom gebunden ist.

In einer bevorzugten Ausführungsform der Erfindung sind R und R` bei jedem Auftreten gleich oder verschieden ausgewählt aus der Gruppe bestehend aus H, D, F, N(Ar')₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches, aromatisches oder heteroaromatisches Ringsystem bilden; weiterhin können zwei Reste R` auch miteinander ein aliphatisches Ringsystem bilden; weiterhin kann R` mit Ar¹ ein Ringsystem bilden. Besonders bevorzugt sind R und R` bei jedem Auftreten gleich oder verschieden ausgewählt aus der Gruppe bestehend aus H, N(Ar')₂, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Ganz besonders bevorzugt sind R und R` bei jedem Auftreten gleich oder verschieden ausgewählt aus der Gruppe bestehend aus H oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Weiterhin kann es bevorzugt sein, wenn R bzw. R` für eine Triaryl- bzw. -heteroarylamingruppe steht, welche durch einen oder mehrere Reste R¹ substituiert sein kann. Diese Gruppe ist eine Ausführungsform eines aromatischen oder heteroaromatischen Ringsystems, wobei dann mehrere Aryl- bzw. Heteroarylgruppen durch ein Stickstoffatom miteinander verknüpft sind. Wenn R bzw. R` für eine Triaryl- bzw. -heteroarylamingruppe steht, weist diese Gruppe bevorzugt 18 bis 30 aromatische Ringatome auf und kann durch einen oder mehrere Reste R¹, bevorzugt nicht-aromatische Reste R¹, substituiert sein.

In einer weiteren bevorzugten Ausführungsform der Erfindung ist Ar' ein aromatisches oder heteroaromatisches Ringsystem mit 6 bis 30 aromatischen Ringatomen, das durch einen oder mehrere Reste R¹ substituiert sein kann. In einer besonders bevorzugten Ausführungsform der Erfindung ist Ar' ein aromatisches oder heteroaromatisches Ringsystem mit 6 bis 24 aromatischen Ringatomen, insbesondere mit 6 bis 13 aromatischen Ringatomen, das durch einen oder mehrere, bevorzugt nicht-aromatische, Reste R¹ substituiert sein kann.

Geeignete aromatische bzw. heteroaromatische Ringsysteme R, R` bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R¹ substituiert sein können. Wenn R, R` bzw. Ar' für eine Heteroarylgruppe, insbesondere für Triazin, Pyrimidin, Chinazolin oder Carbazol steht, können auch aromatische oder heteroaromatische Reste R¹ an dieser Heteroarylgruppe bevorzugt sein.

Dabei sind die Gruppen R bzw. R', wenn sie für ein aromatisches bzw. heteroaromatisches Ringsystem stehen, bzw. Ar' bevorzugt gewählt aus den Gruppen der folgenden Formeln R-1 bis R-83, 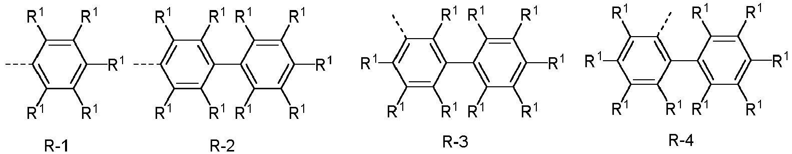 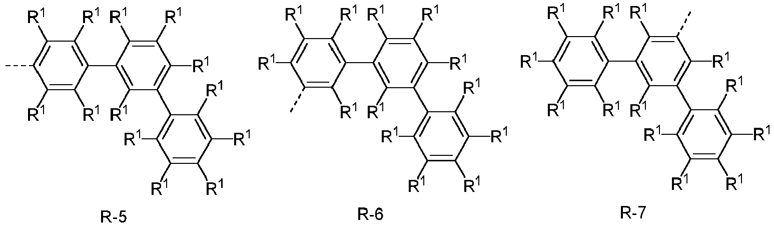 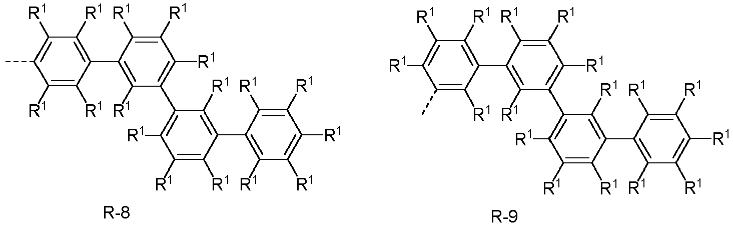 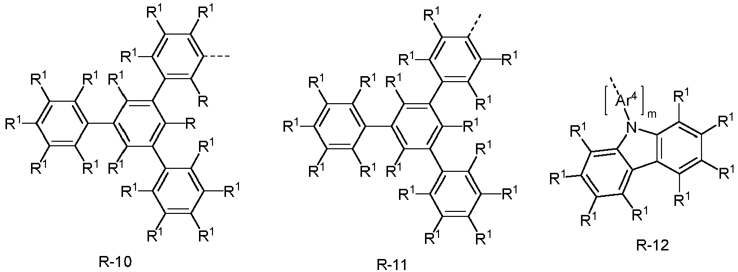 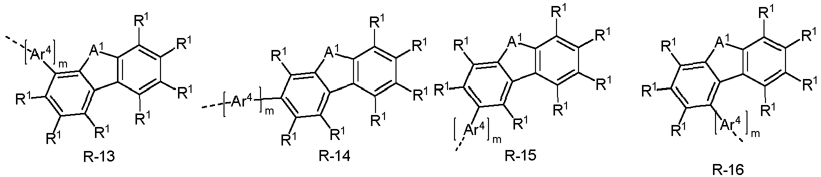 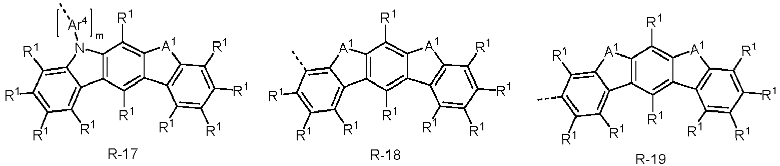 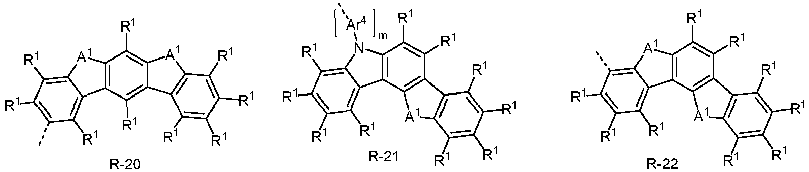 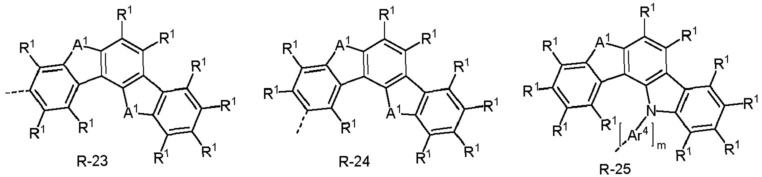 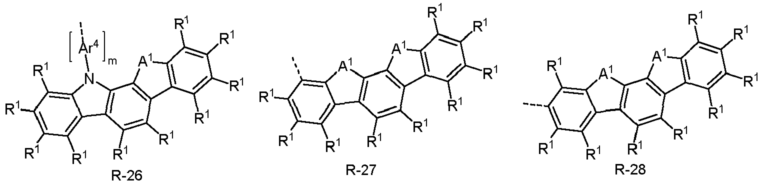 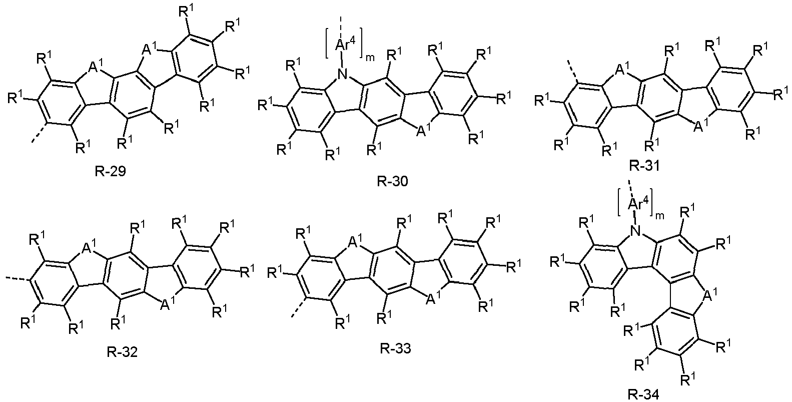 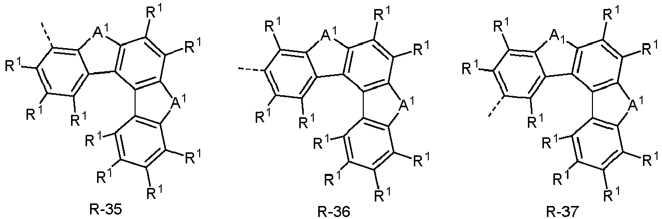 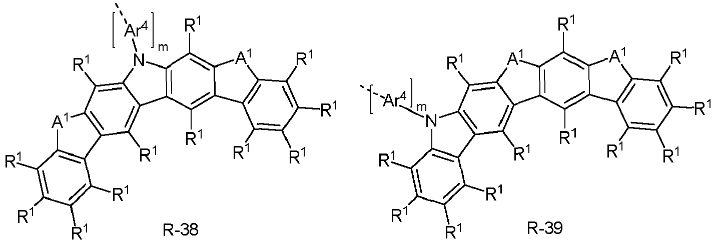 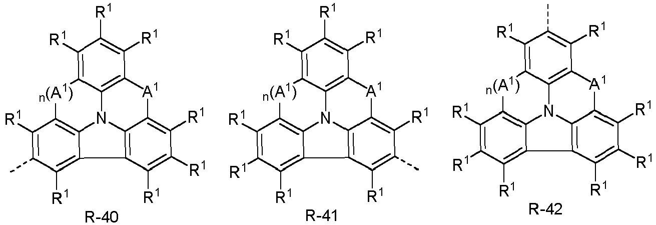 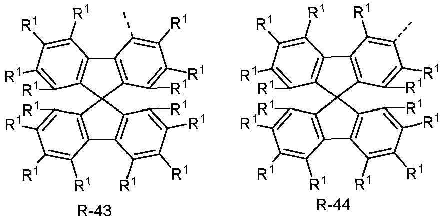 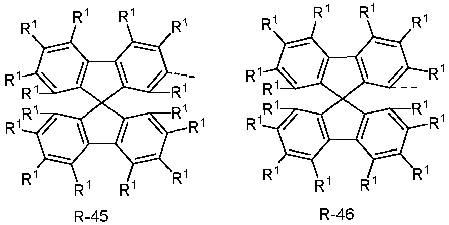 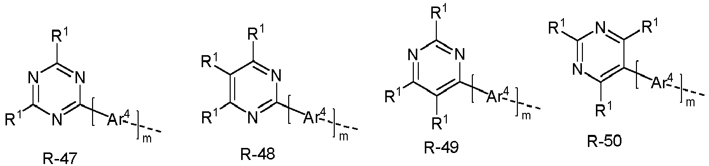 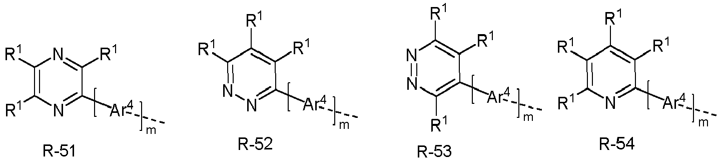 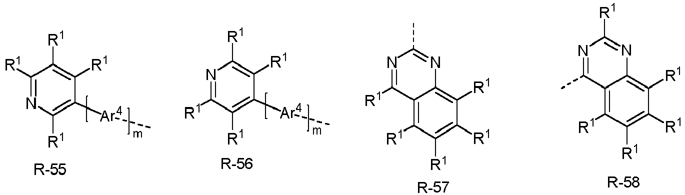 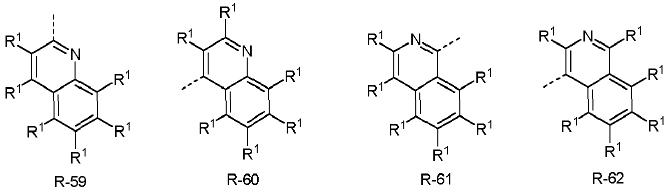 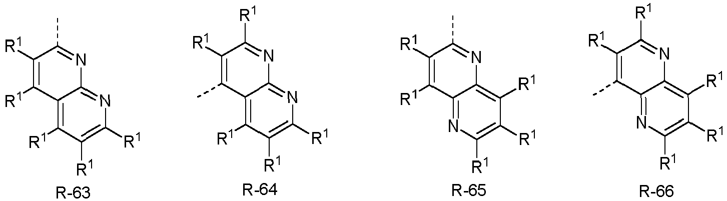 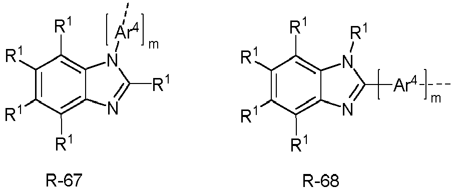 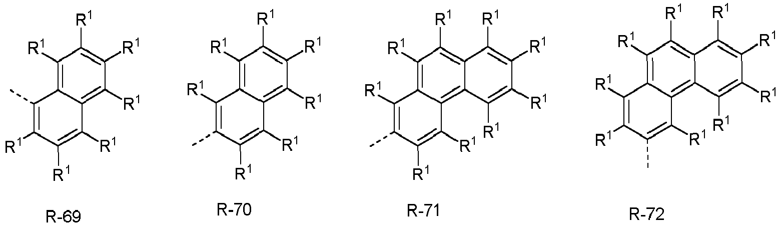 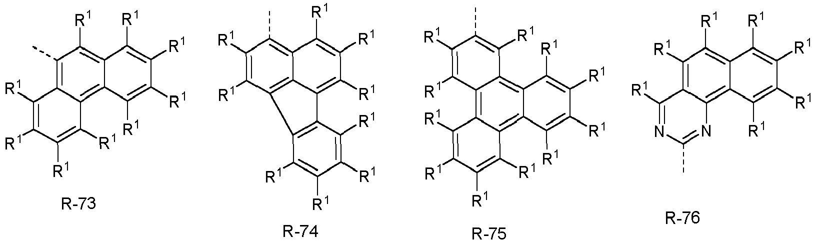 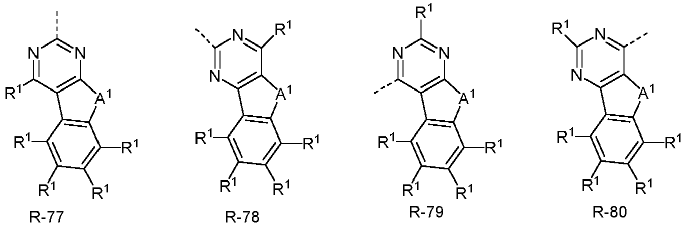 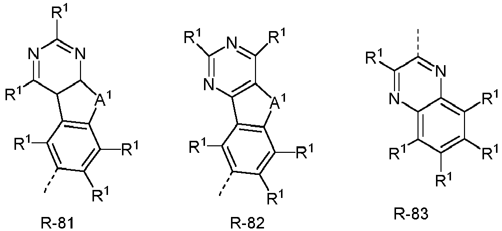 wobei R' die oben genannten Bedeutungen aufweist, die gestrichelte Bindung die Bindung an ein Kohlenstoffatom des Grundgerüsts in Formel (1), (2) und (3) bzw. in den bevorzugten Ausführungsformen bzw. an das Stickstoffatom in der Gruppe N(Ar')₂ darstellt und weiterhin gilt:
- Ar⁴: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A¹: ist bei jedem Auftreten gleich oder verschieden C(R¹)₂, NR¹, O oder S;
- n: ist 0 oder 1, wobei n = 0 bedeutet, dass an dieser Position keine Gruppe A¹ gebunden ist und an den entsprechenden Kohlenstoffatomen statt dessen Reste R¹ gebunden sind;
- m: ist 0 oder 1, wobei m = 0 bedeutet, dass die Gruppe Ar⁴ nicht vorhanden ist und dass die entsprechende aromatische bzw. heteroaromatische Gruppe direkt an ein Kohlenstoffatom des Grundgerüsts in Formel (1) bzw. in den bevorzugten Ausführungsformen bzw. an das Stickstoffatom in der Gruppe N(Ar')₂ gebunden ist; mit der Maßgabe, dass m = 1 ist für die Strukturen (R-12), (R-17), (R-21), (R-25), (R-26), (R-30), (R-34), (R-38) und (R-39), wenn es sich bei diesen Gruppen um Ausführungsformen von Ar' handelt.

Wenn die oben genannten Gruppen Ar-1 bis Ar-83 für Ar¹, Ar² bzw. Ar³ und R-1 bis R-83 für R bzw. Ar' mehrere Gruppen A¹ aufweisen, so kommen hierfür alle Kombinationen aus der Definition von A¹ in Frage. Bevorzugte Ausführungsformen sind dann solche, in denen eine Gruppe A¹ für NR bzw. NR¹ und die andere Gruppe A¹ für C(R)₂ bzw. C(R¹)₂ steht oder in denen beide Gruppen A¹ für NR bzw. NR¹ stehen oder in denen beide Gruppen A¹ für O stehen. In einer besonders bevorzugten Ausführungsform der Erfindung steht in Gruppen Ar¹, Ar², Ar³, R bzw. Ar`, die mehrere Gruppen A¹ aufweisen, mindestens eine Gruppe A¹ für C(R)₂ bzw. C(R¹)₂ oder für NR bzw. NR¹.

Wenn A¹ für NR bzw. NR¹ steht, steht der Substituent R bzw. R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R¹ bzw. R² substituiert sein kann. In einer besonders bevorzugten Ausführungsform steht dieser Substituent R bzw. R¹ gleich oder verschieden bei jedem Auftreten für ein aromatisches oder heteroaromatisches Ringsystem mit 6 bis 24 aromatischen Ringatomen, bevorzugt mit 6 bis 12 aromatischen Ringatomen, welches keine kondensierten Arylgruppen oder Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R¹ bzw. R² substituiert sein kann. Besonders bevorzugt sind Phenyl, Biphenyl, Terphenyl und Quaterphenyl mit Verknüpfungsmustern, wie vorne für Ar-1 bis Ar-11 bzw. R-1 bis R-11 aufgeführt, wobei diese Strukturen durch einen oder mehrere Reste R¹ bzw. R² substituiert sein können, bevorzugt aber unsubstituiert sind.

Wenn A¹ für C(R)₂ bzw. C(R¹)₂ steht, stehen die Substituenten R bzw. R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R¹ bzw. R² substituiert sein kann. Ganz besonders bevorzugt steht R bzw. R¹ für eine Methylgruppe oder für eine Phenylgruppe. Dabei können die Reste R bzw. R¹ auch miteinander ein Ringsystem bilden, was zu einem Spirosystem führt.

In einer bevorzugten Ausführungsform der Erfindung weist die Verbindung mindestens einen Rest R oder R` auf, der für ein heteroaromatisches Ringsystem steht, und/oder mindestens eine Gruppe Ar¹ oder Ar² oder, wenn vorhanden, Ar³ steht für ein heteroaromatisches Ringsystem.

In einer Ausführungsform der Erfindung steht mindestens ein Rest R bzw. R` für ein elektronenreiches heteroaromatisches Ringsystem. Dabei ist das elektronenreiche heteroaromatische Ringsystem bevorzugt gewählt aus den oben abgebildeten Gruppen R-13 bis R-42, wobei in den Gruppen R-13 bis R-16, R-18 bis R-20, R-22 bis R-24, R-27 bis R-29, R-31 bis R-33 und R-35 bis R-37 mindestens eine Gruppe A¹ für NR¹ steht, wobei R¹ bevorzugt für ein aromatisches oder heteroaromatisches Ringsystem steht, inbesondere für ein aromatisches Ringsystem.

In einer weiteren Ausführungsform der Erfindung steht mindestens ein Rest R bzw. R` für ein elektronenarmes heteroaromatisches Ringsystem. Dabei ist das elektronenarme heteroaromatische Ringsystem bevorzugt gewählt aus den oben abgebildeten Gruppen R-47 bis R-50, R-57, R-58 und R-76 bis R-83.

In einer weiteren Ausführungsform der Erfindung steht Ar¹ und/oder Ar² und/oder, falls vorhanden, Ar³ für ein elektronenarmes heteroaromatisches Ringsystem. Dabei ist das elektronenarme heteroaromatische Ringsystem bevorzugt gewählt aus den oben abgebildeten Gruppen Ar-47 bis Ar-50, Ar-57, Ar-58 und Ar-76 bis Ar-83.

In einer weiteren bevorzugten Ausführungsform der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, F, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Weitere geeignete Gruppen Ar¹, Ar², Ar³, R, R` bzw. Ar' sind Gruppen der Formel -Ar⁷-N(Ar⁵)(Ar⁶), wobei Ar⁵, Ar⁶ und Ar⁷ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Für Ar ergibt sich eine solche Gruppe, indem die Gruppe Ar mit einer Gruppe N(Ar')₂ substituiert ist. Dabei beträgt die Gesamtzahl der aromatischen Ringatome von Ar⁵, Ar⁶ und Ar⁷ maximal 60 und bevorzugt maximal 40.

Dabei können Ar⁷ und Ar⁵ miteinander und/oder Ar⁵ und Ar⁶ miteinander auch durch eine Gruppe, ausgewählt aus C(R¹)₂, NR¹, O oder S, verbunden sein. Bevorzugt erfolgt die Verknüpfung von Ar⁷ und Ar⁵ miteinander bzw. von Ar⁵ und Ar⁶ miteinander jeweils ortho zur Position der Verknüpfung mit dem Stickstoffatom. In einer weiteren Ausführungsform der Erfindung sind keine der Gruppen Ar⁵, Ar⁶ bzw. Ar⁷ miteinander verbunden.

Bevorzugt ist Ar⁷ ein aromatisches oder heteroaromatisches Ringsystem mit 6 bis 24 aromatischen Ringatomen, insbesondere mit 6 bis 12 aromatischen Ringatomen, das jeweils mit einem oder mehreren Resten R¹ substituiert sein kann. Besonders bevorzugt ist Ar⁷ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁷ eine unsubstituierte Phenylengruppe. Dies gilt insbesondere, wenn Ar⁷ mit Ar⁵ durch einen Einfachbindung verbunden ist.

Bevorzugt sind Ar⁵ und Ar⁶ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar⁵ bzw. Ar⁶ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar⁵ und Ar⁶ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben die Alkylgruppen in erfindungsgemäßen Verbindungen, die durch Vakuumverdampfung verarbeitet werden, bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste Ar¹, Ar², Ar³, R, R', Ar`, R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für geeignete Verbindungen gemäß den oben aufgeführten Ausführungsformen sind die in der folgenden Tabelle aufgeführten Verbindungen.

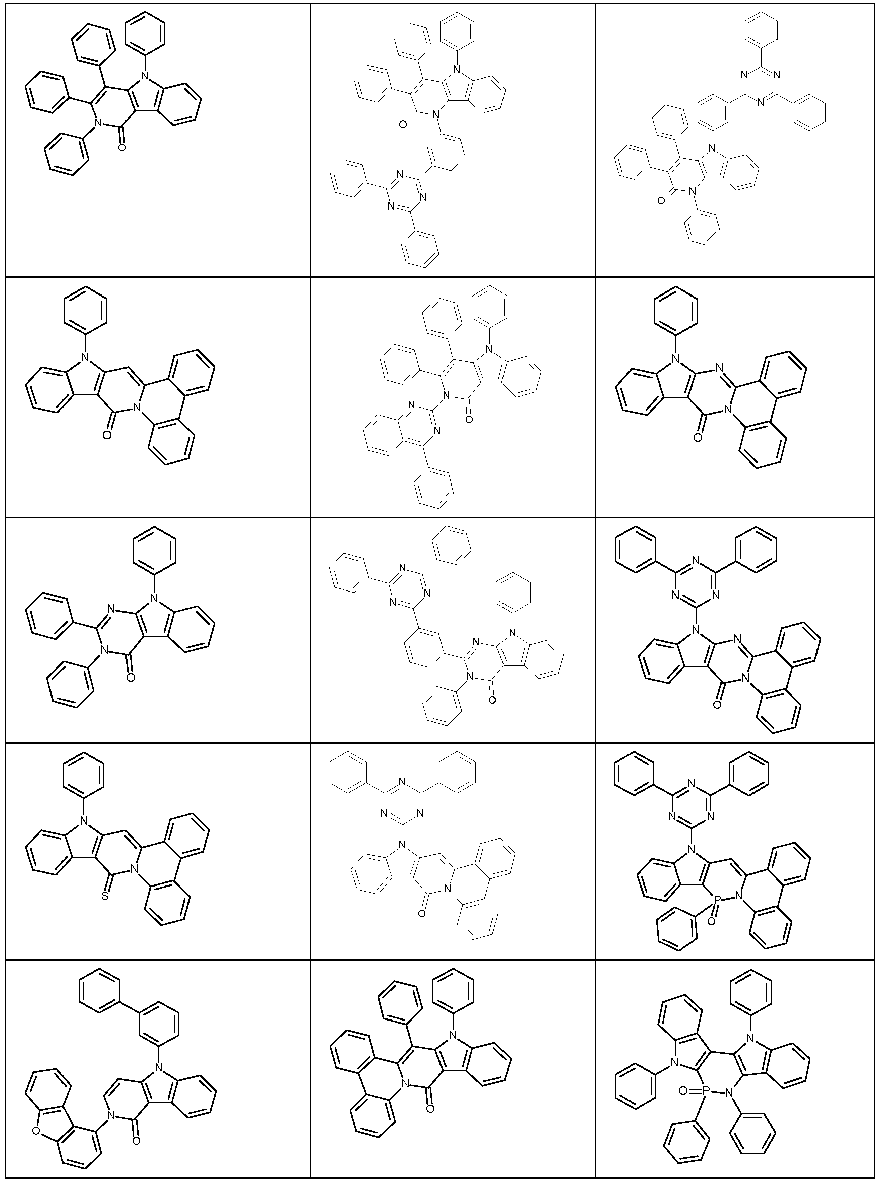

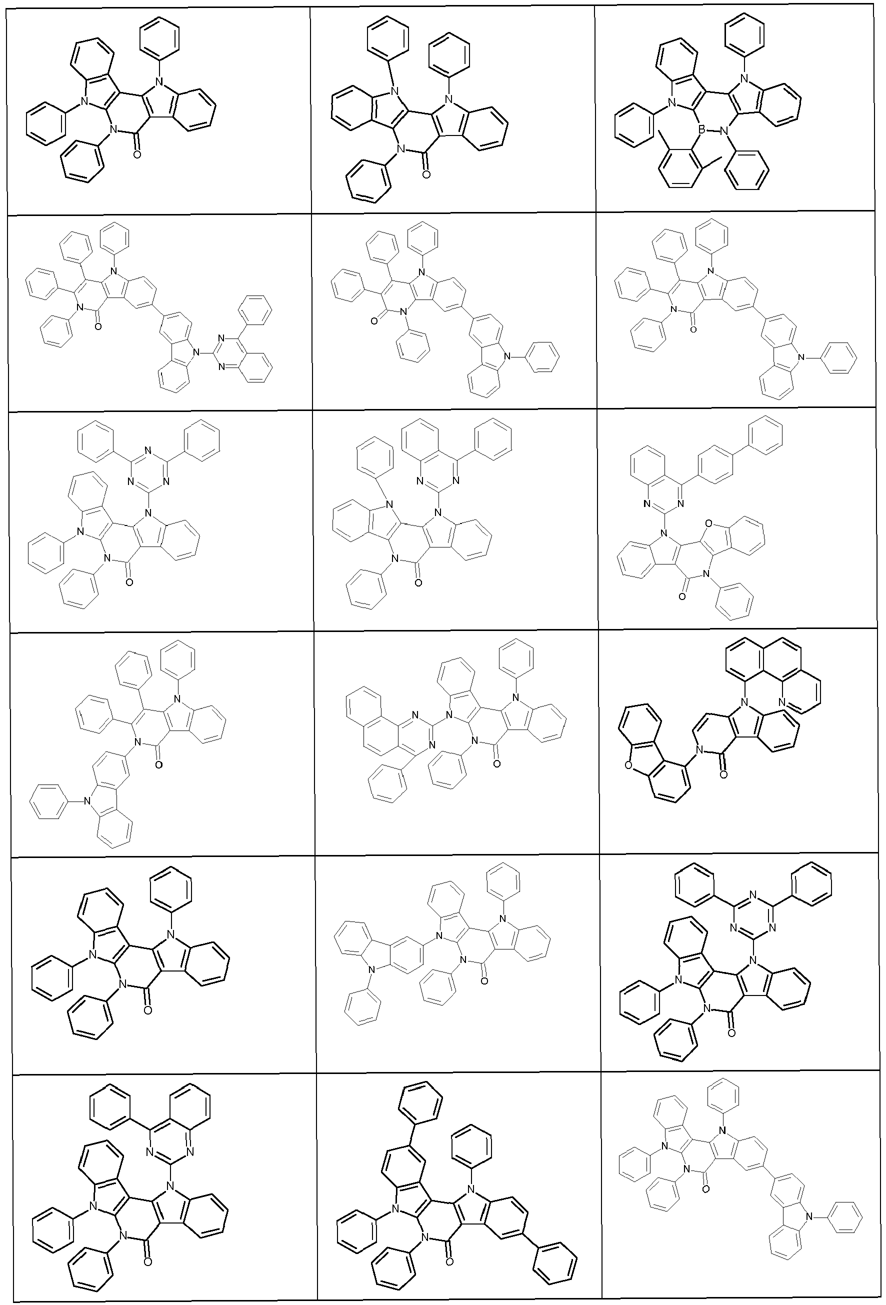

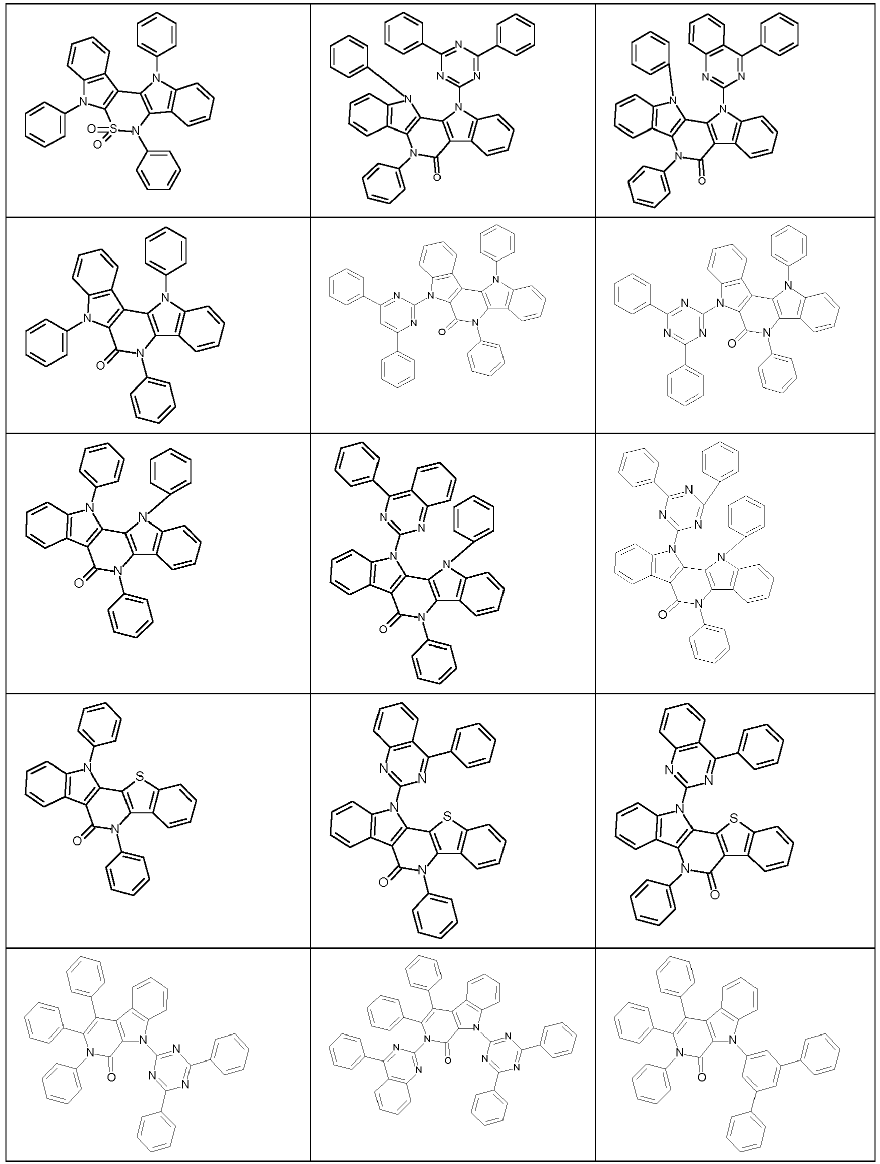

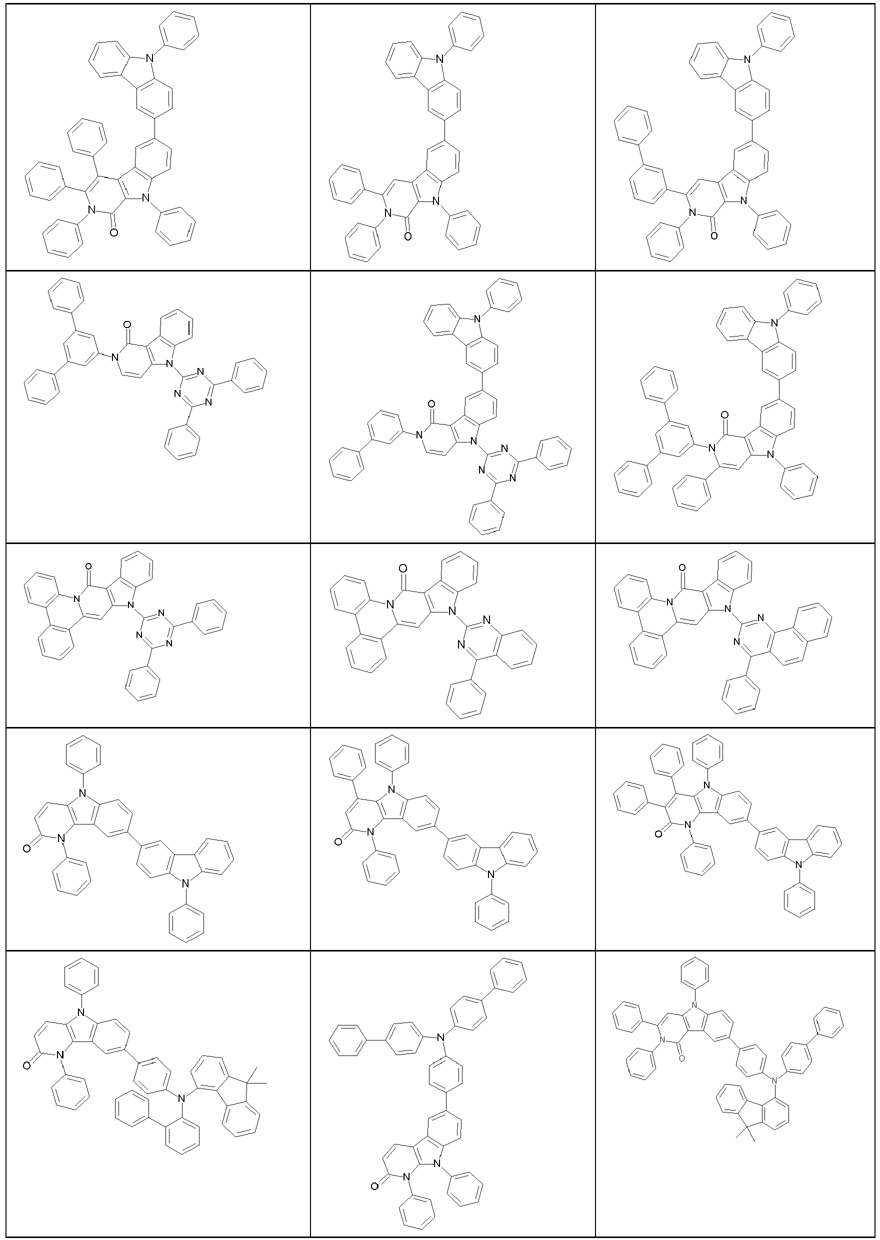

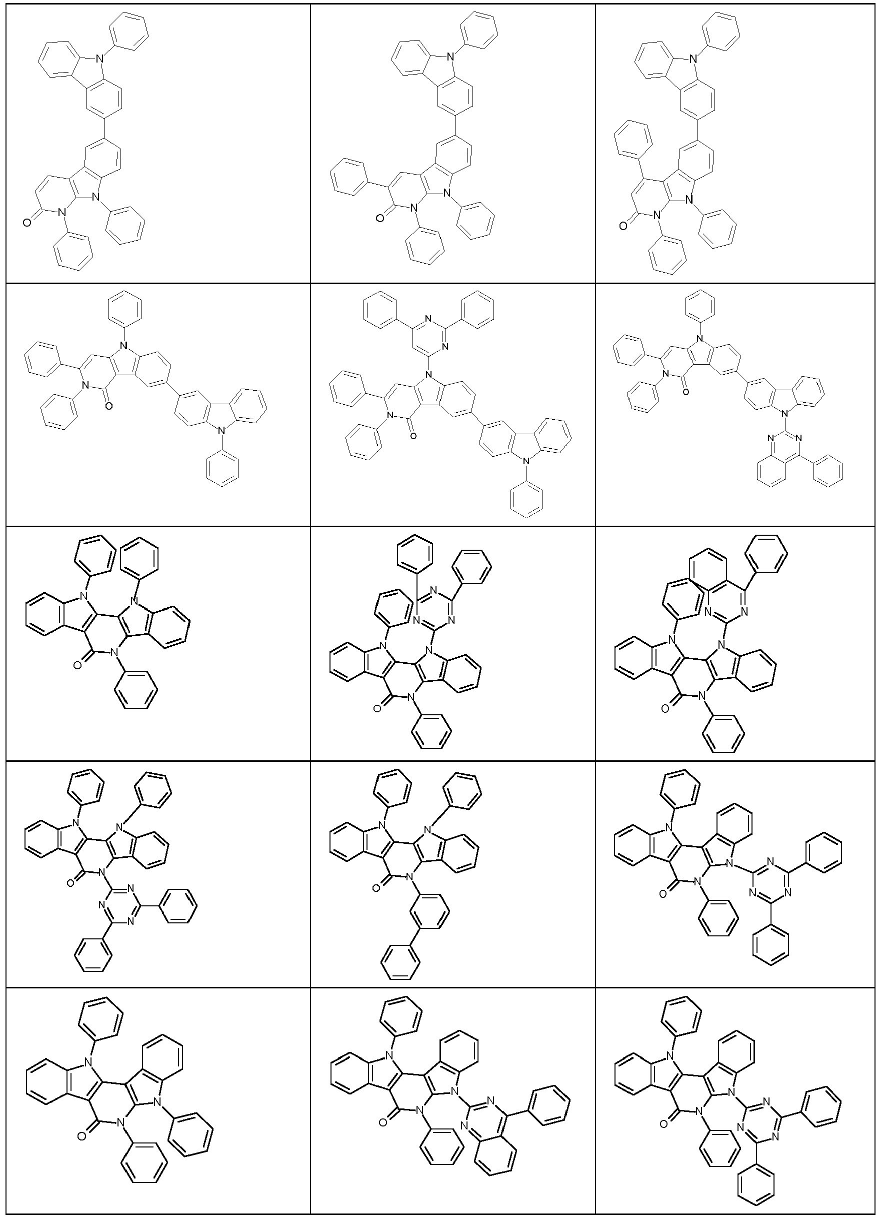

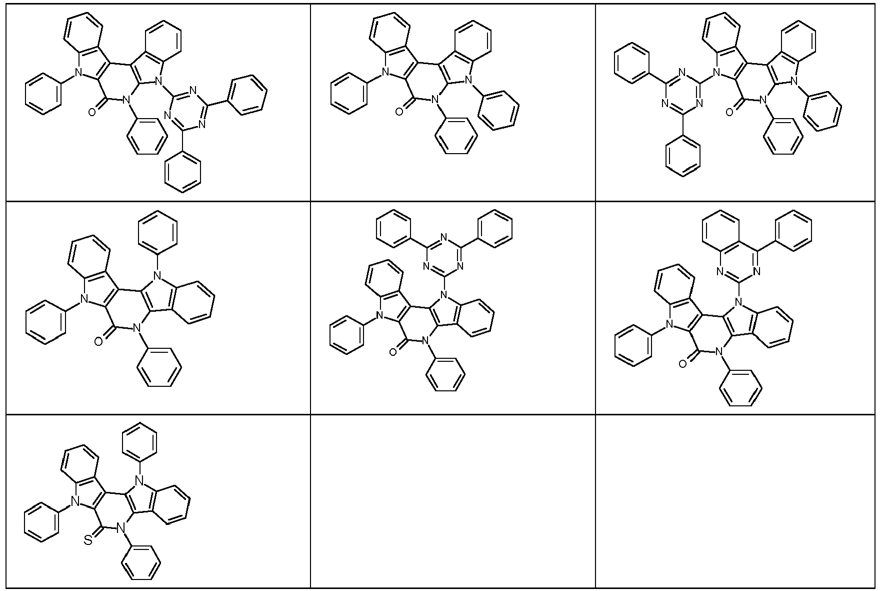

Die Grundstruktur der erfindungsgemäßen Verbindungen, die keine Gruppe gemäß Formel (3) enthalten und die noch nicht durch Ar¹ und Ar² substituiert sind, ist in der Literatur bekannt. Die Synthese der erfindungsgemäßen Verbindungen aus diesen Grundstrukturen ist in Schema 1 dargestellt. Dabei können die Gruppen Ar¹ und Ar² durch eine C-N-Kupplungsreaktion eingeführt werden, beispielsweise durch eine Buchwald-Kupplung oder eine Ullmann-Kupplung. Die Synthese von erfindungsgemäßen Verbindungen, welche eine Gruppe der Formel (3) aufweisen, ist in den in Schema 2 skizzierten Wegen dargestellt. Dabei wird zunächst das Grundgerüst, welches noch keine Gruppen Ar¹, Ar² und Ar³ trägt, synthetisiert. Danach können analog zu Schema 1 das Indol-Stickstoffatom und das Lactam-Stickstoffatom substituiert werden, beispielsweise durch Buchwald-Kupplung bzw. durch Ullmann-Kupplung.

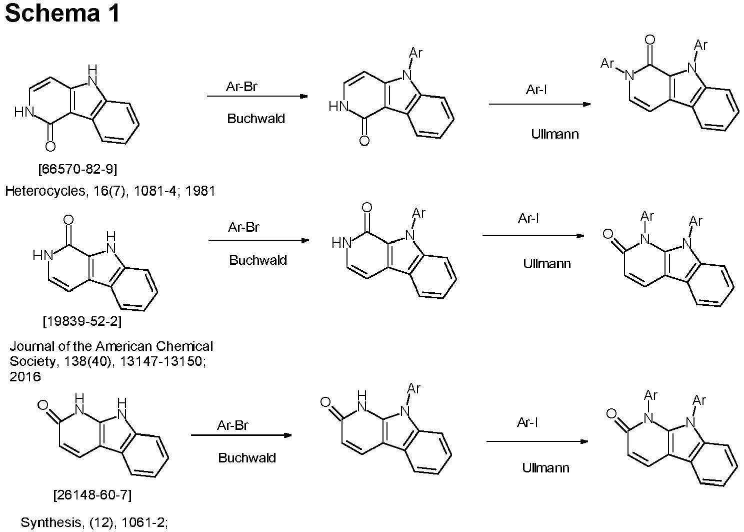

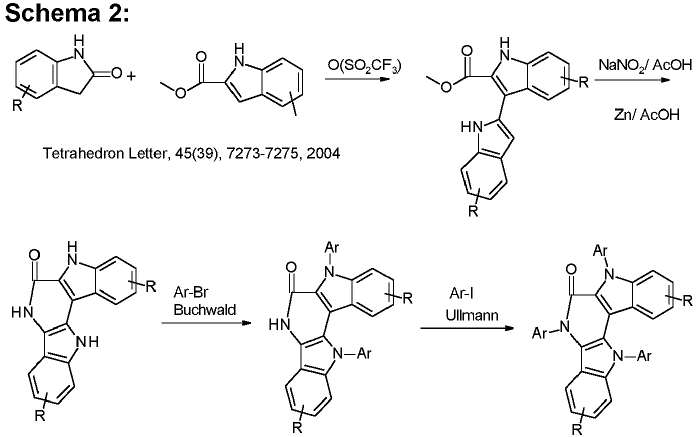

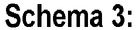

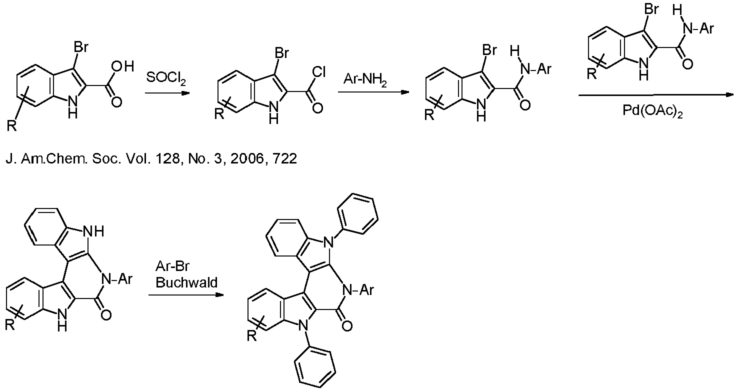

Ein weiterer Gegenstand der Erfindung ist daher ein Verfahren zur Herstellung der erfindungsgemäßen Verbindungen, gekennzeichnet durch die folgenden Schritte:
(1) Synthese des entsprechenden Grundgerüsts, welches noch nicht durch die Gruppen Ar¹, Ar² und gegebenenfalls Ar³ substituiert ist; und
(2) Einführung der Gruppen Ar¹, Ar² und gegebenenfalls Ar³ durch eine C-N-Kupplungsreaktion.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrix-materialien sind hinten im Zusammenhang mit der organischen Elektrolumineszenzvorrichtung aufgeführt.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochblockierschicht und/oder in einer Lochtransportschicht und/oder in einer Exzitonenblockierschicht eingesetzt werden.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

In einer Ausführungsform der Erfindung wird die erfindungsgemäße Verbindung als einziges Matrixmaterial für einen phosphoreszierenden Emitter eingesetzt. Eine weitere Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

In einer bevorzugten Ausführungsform der Erfindung werden die Materialien in Kombination mit einem weiteren Matrixmaterial eingesetzt. Wenn die erfindungsgemäße Verbindung mit einem elektronenarmen heteroaromatischen Ringsystem substituiert ist, sind bevorzugte Co-Matrixmaterialien gewählt aus der Gruppe der Biscarbazole, der verbrückten Carbazole, der Triarylamine, der Dibenzofuran-Carbazol-Derivate bzw. Dibenzofuran-Amin-Derivate und der Carbazolamine.

Bevorzugte Biscarbazole sind die Strukturen der folgenden Formeln (12) und (13), 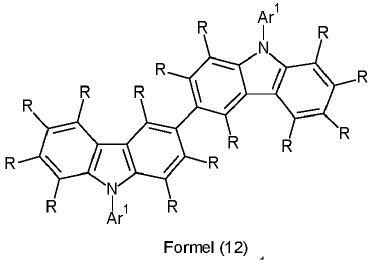 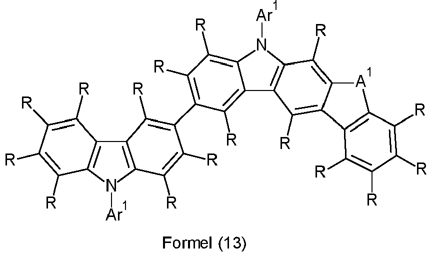 wobei Ar¹ und A¹ die oben genannten Bedeutungen aufweisen und R die oben genannten Bedeutungen aufweist. In einer bevorzugten Ausführungsform der Erfindung steht A¹ für CR₂.

Bevorzugte Ausführungsformen der Verbindungen der Formeln (12) bzw. (13) sind die Verbindungen der folgenden Formeln (12a) bzw. (13a), 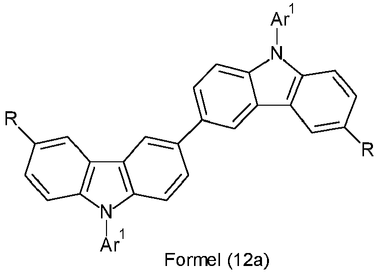 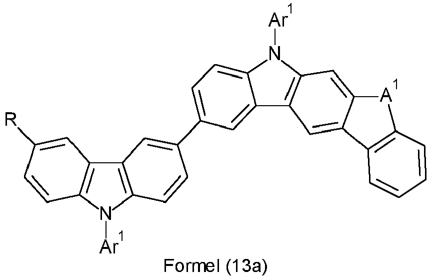 wobei die verwendeten Symbole die oben genannten Bedeutungen aufweisen.

Beispiele für geeignete Verbindungen gemäß Formel (12) oder (13) sind die nachfolgend abgebildeten Verbindungen.

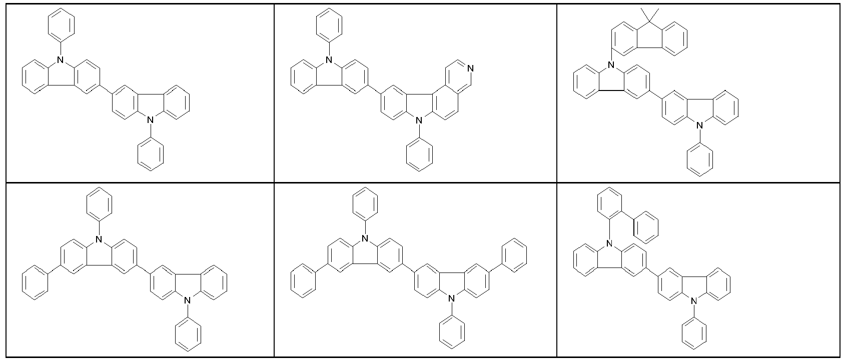

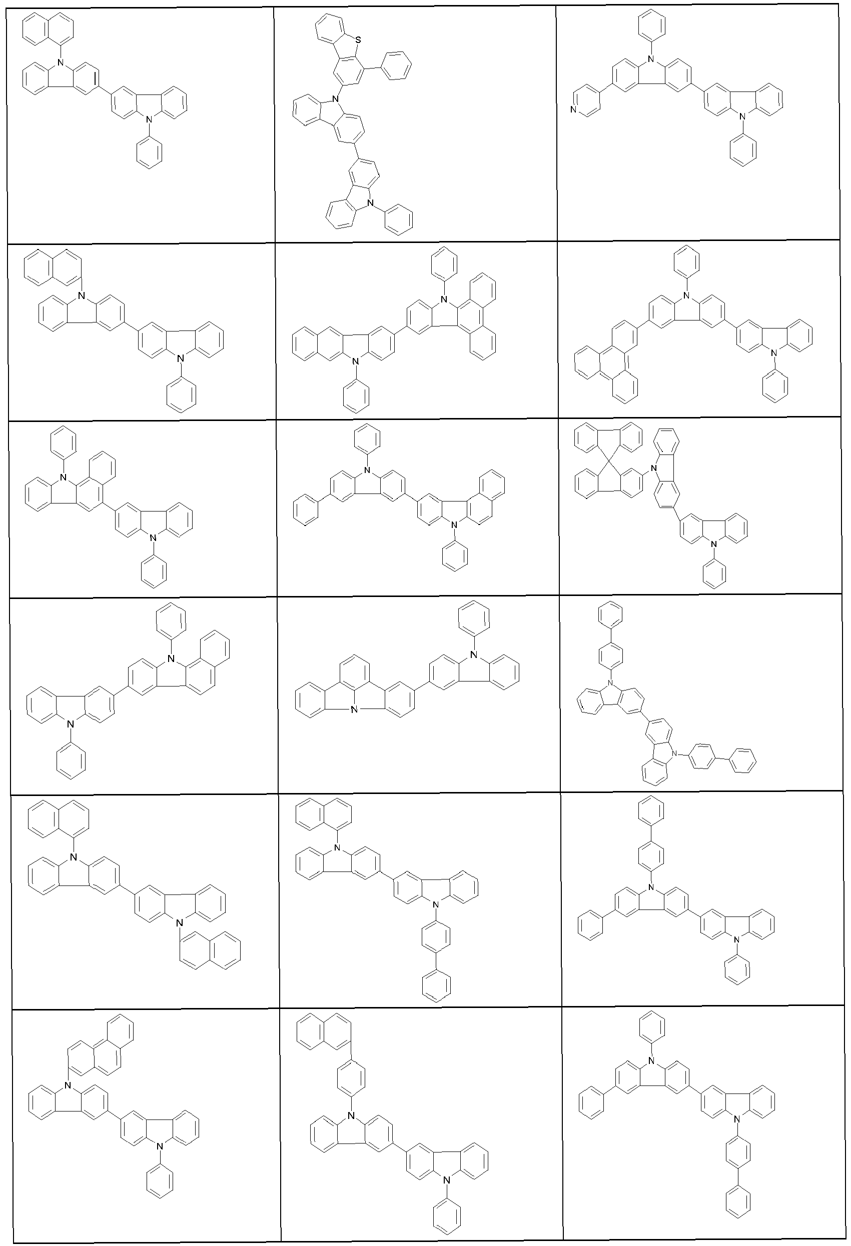

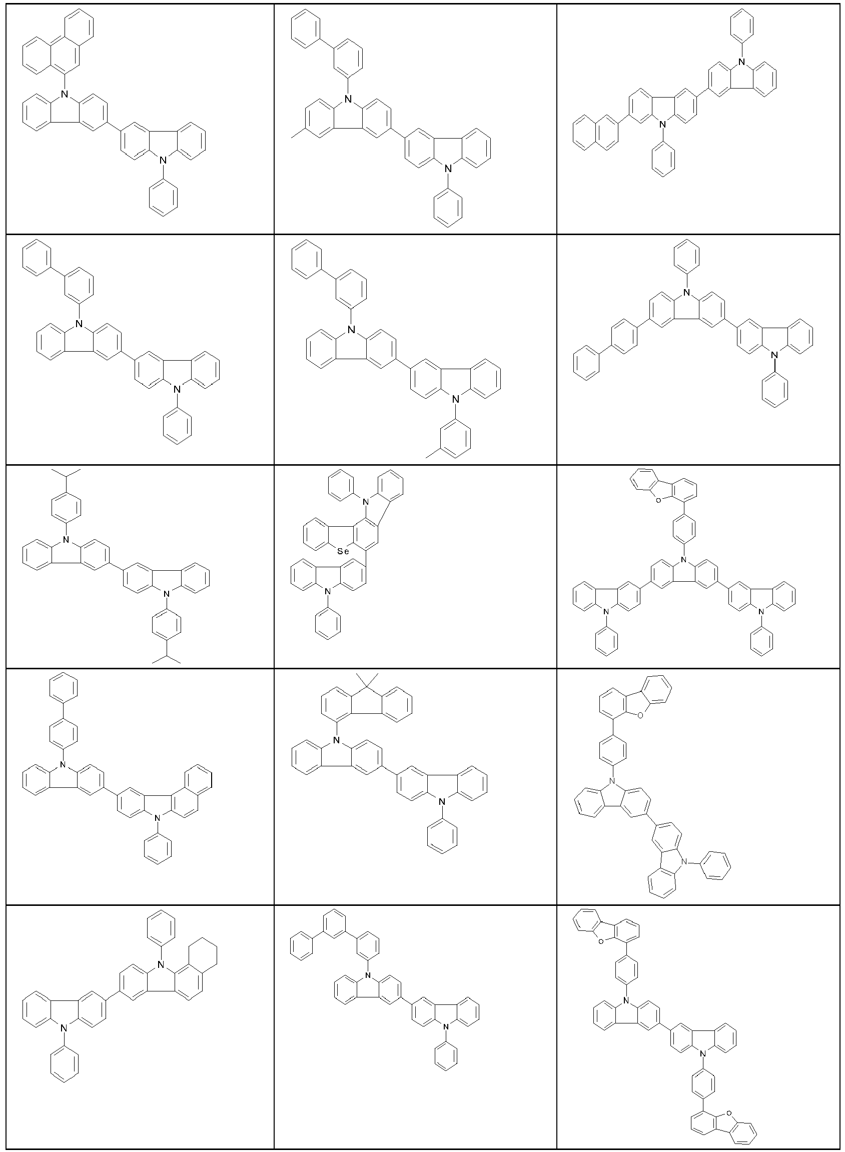

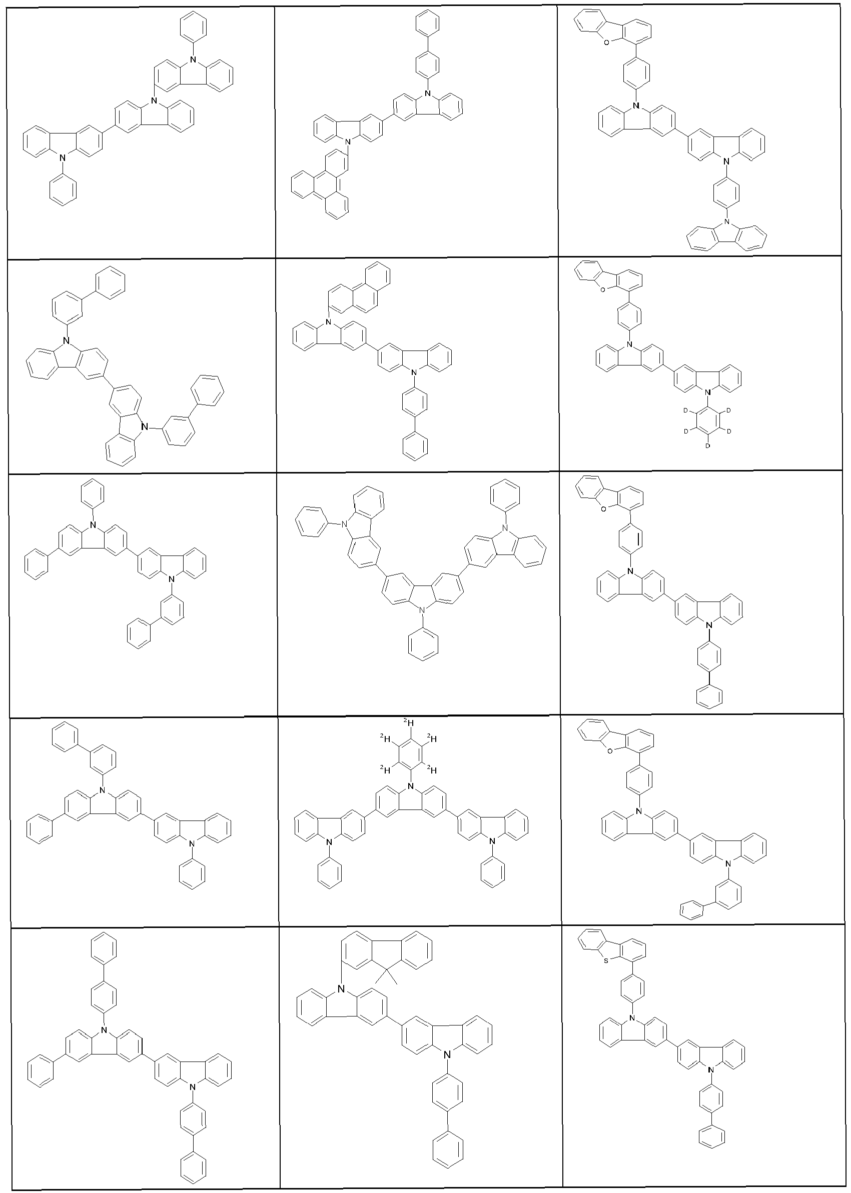

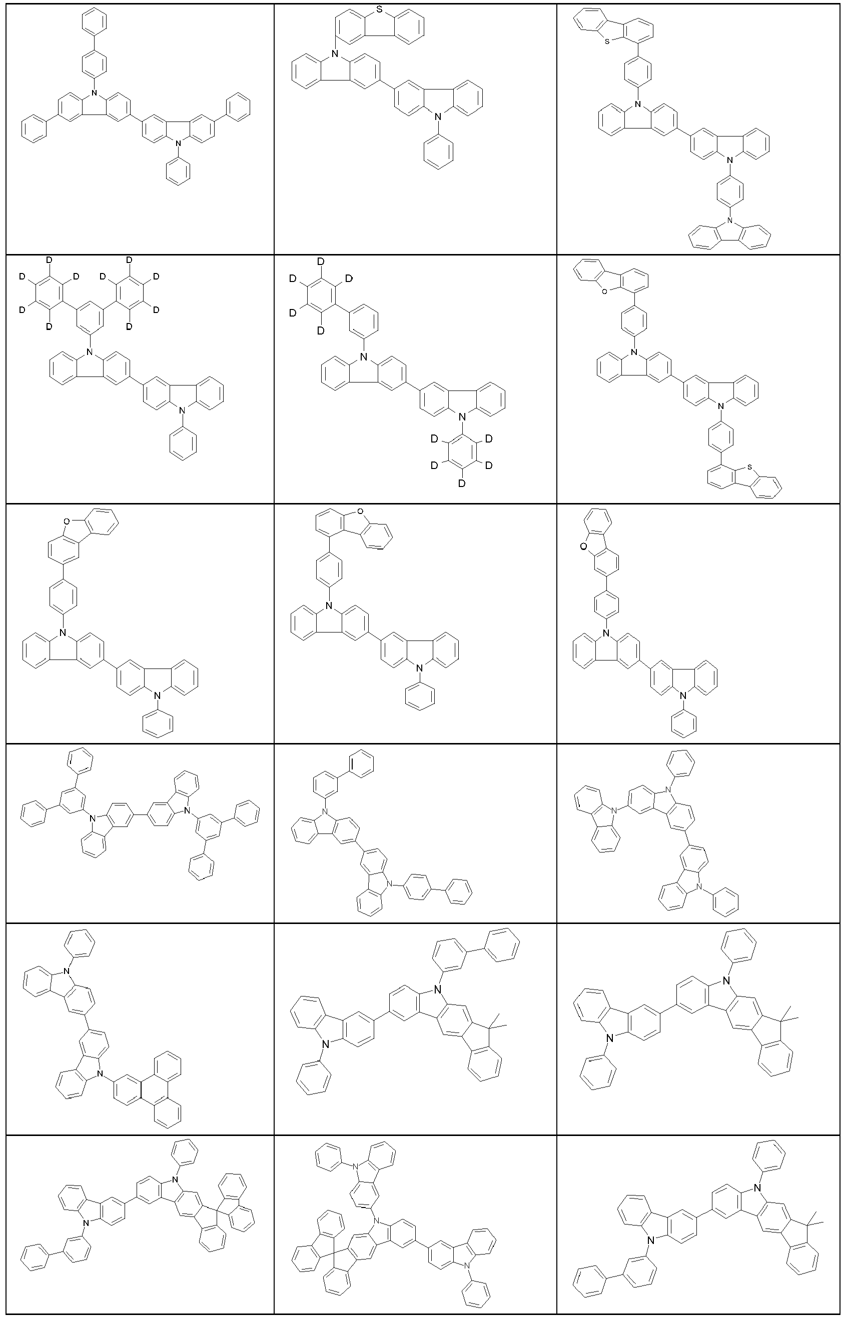

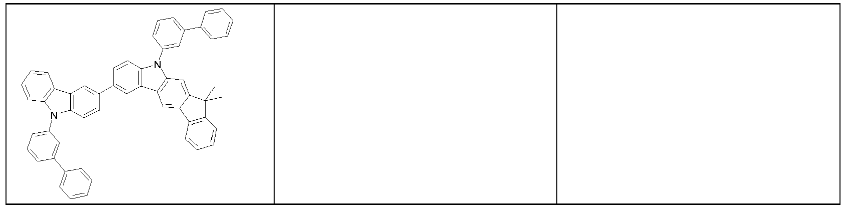

Bevorzugte verbrückte Carbazole sind die Strukturen der folgenden Formel (14), 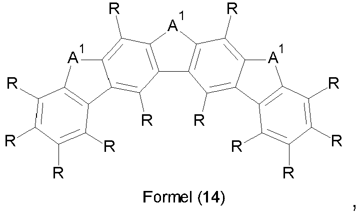 wobei A¹ und R die oben genannten Bedeutungen aufweisen und A¹ bevorzugt gleich oder verschieden bei jedem Auftreten ausgewählt ist aus der Gruppe bestehend aus NAr¹ und CR₂.

Bevorzugte Dibenzofuran-Derivate sind die Verbindungen der folgenden Formel (15), 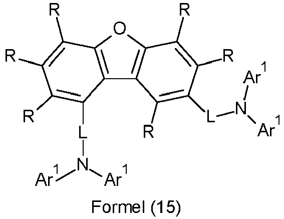 wobei der Sauerstoff auch durch Schwefel ersetzt sein kann, so dass ein Dibenzothiophen entsteht, L für eine Einfachbindung oder ein aromatisches oder heteroaromatisches Ringsystem mit 5 bis 30 aromatischen Ringatomen steht, welches auch durch eine oder mehrere Reste R substituiert sein kann, und R und Ar¹ die oben genannten Bedeutungen aufweisen. Dabei können die beiden Gruppen Ar¹, die an dasselbe Stickstoffatom binden, oder eine Gruppe Ar¹ und eine Gruppe L, die an dasselbe Stickstoffatom binden, auch miteinander verbunden sein, beispielsweise zu einem Carbazol.

Beispiele für geeignete Dibenzofuran-Derivate sind die nachfolgend abgebildeten Verbindungen.

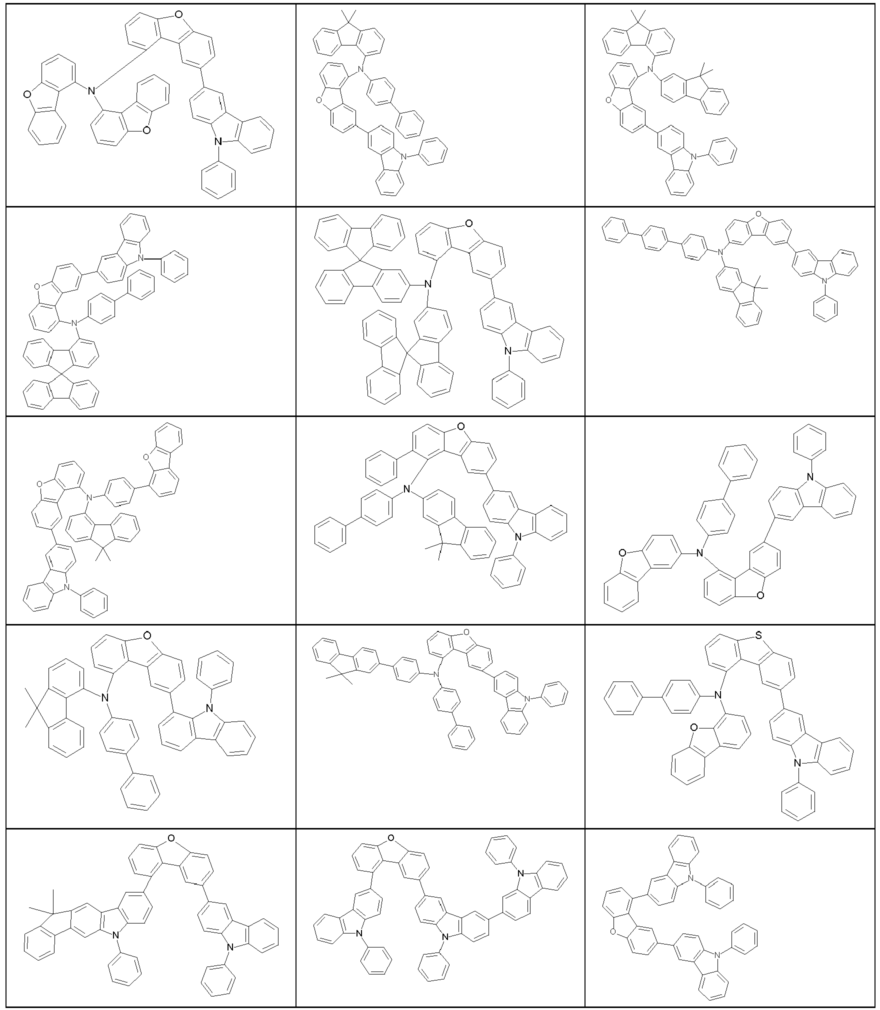

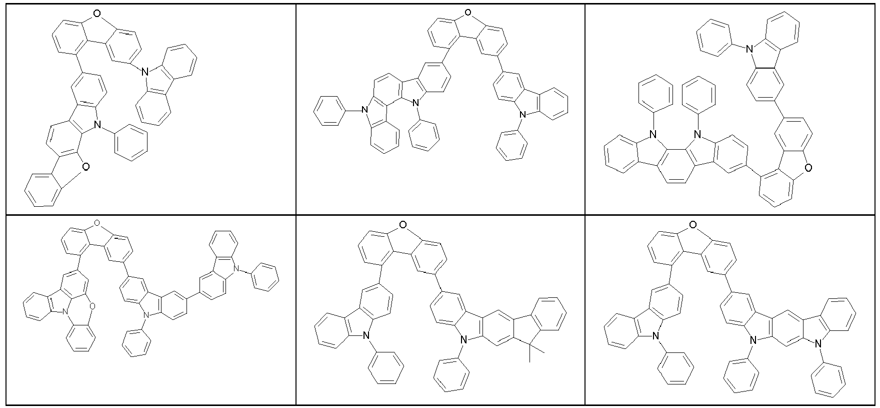

Bevorzugte Carbazolamine sind die Strukturen der folgenden Formeln (16), (17) und (18), 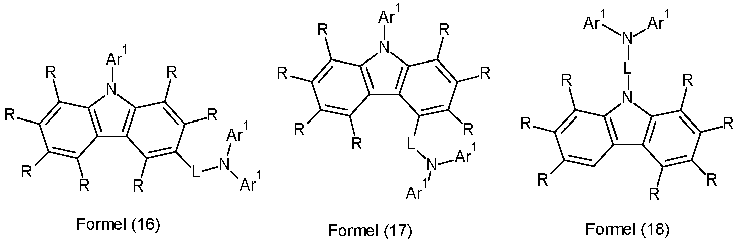 wobei L für ein aromatisches oder heteroaromatisches Ringsystem mit 5 bis 30 aromatischen Ringatomen steht, welches mit einem oder mehreren Resten R substituiert sein kann, und R und Ar¹ die oben genannten Bedeutungen aufweisen.

Beispiele für geeignete Carbazolamin-Derivate sind die nachfolgend abgebildeten Verbindungen.

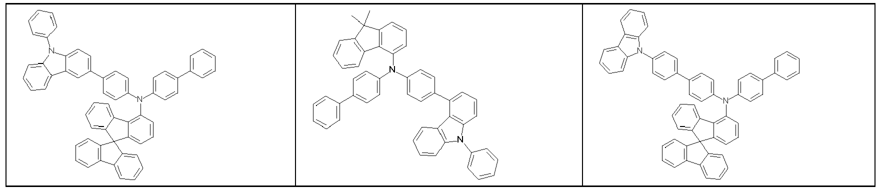

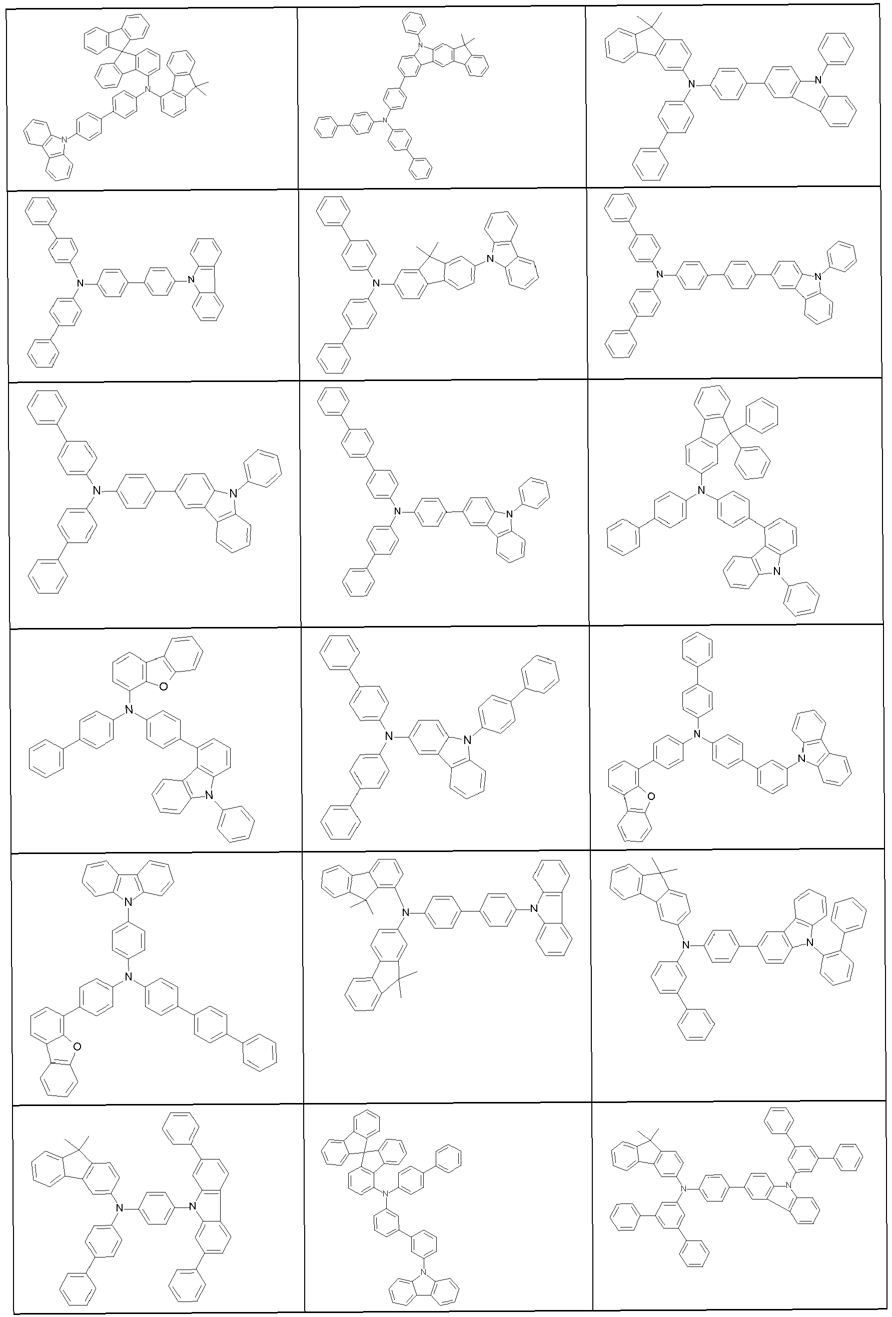

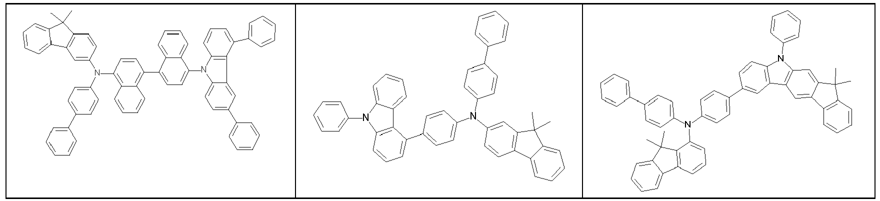

Wenn die erfindungsgemäße Verbindung mit einem elektronenreichen heteroaromatischen Ringsystem, beispielsweise einer Carbazolgruppe, substituiert ist, sind bevorzugte Co-Matrix-Materialien ausgewählt aus der Gruppe bestehend aus Triazin-Derivaten, Pyrimidin-Derivaten und Chinazolin-Derivaten. Bevorzugte Triazin-, Chinazolin- bzw. Pyrimidinderivate, welche als Mischung zusammen mit den erfindungsgemäßen Verbindungen eingesetzt werden können, sind die Verbindungen der folgenden Formeln (19), (20) und (21), 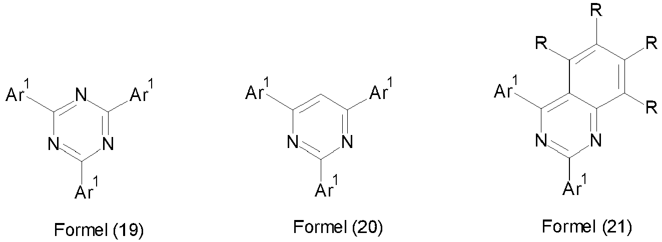 wobei Ar¹ und R die oben genannten Bedeutungen aufweist.

Besonders bevorzugt sind die Triazinderivate der Formel (19) und die Chinazolinderivate der Formel (21), insbesondere die Triazinderivate der Formel (19).

In einer bevorzugten Ausführungsform der Erfindung ist Ar¹ in den Formeln (19), (20) und (21) bei jedem Auftreten gleich oder verschieden ein aromatisches oder heteroaromatisches Ringsystem mit 6 bis 30 aromatischen Ringatomen, insbesondere mit 6 bis 24 aromatischen Ringatomen, das durch einen oder mehrere Reste R substituiert sein kann. Dabei sind geeignete aromatische bzw. heteroaromatische Ringsysteme Ar¹ die gleichen, wie sie oben als Ausführungsformen für Ar¹, Ar² und Ar³ ausgeführt sind, insbesondere die Strukturen Ar-1 bis Ar-83.

Beispiele für geeignete Triazinverbindungen, welche als Matrixmaterialien zusammen mit den erfindungsgemäßen Verbindungen eingesetzt werden können, sind die in der folgenden Tabelle abgebildeten Verbindungen.

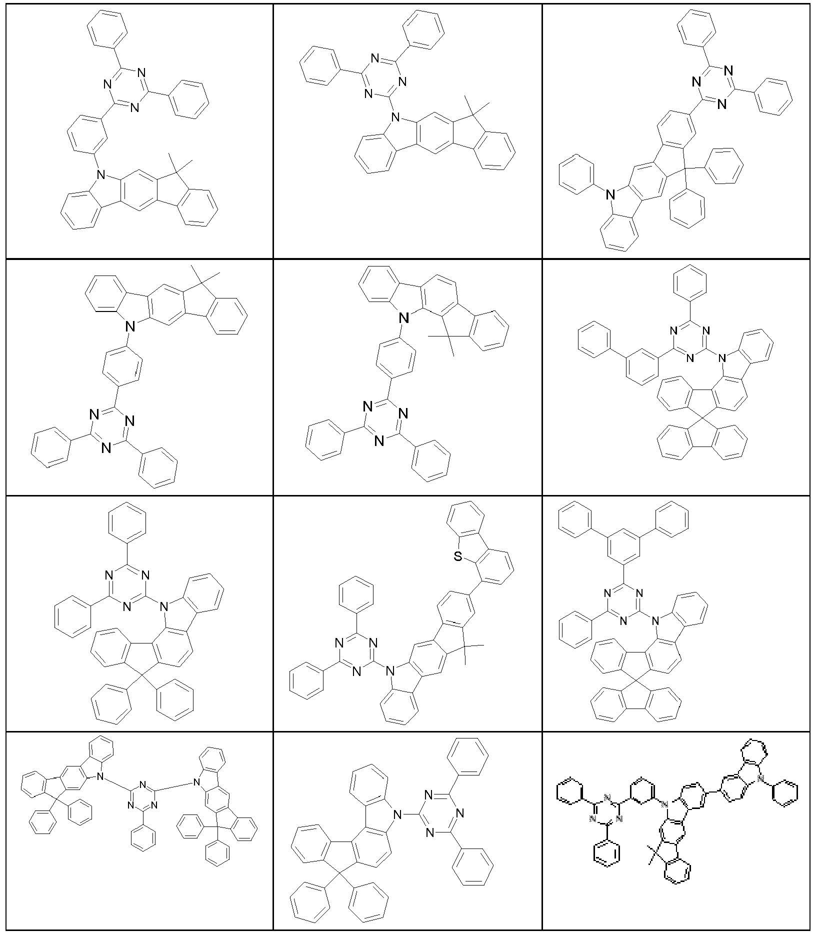

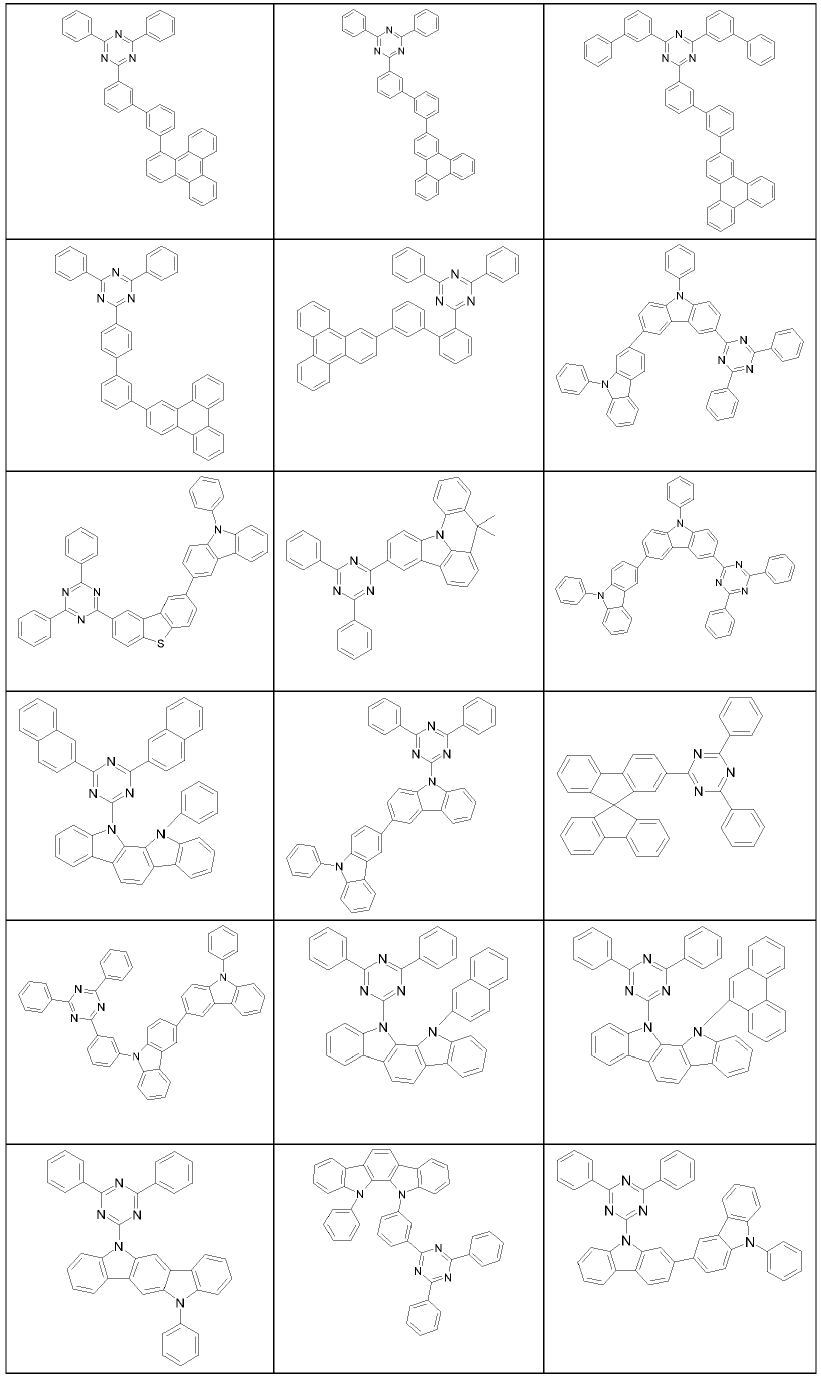

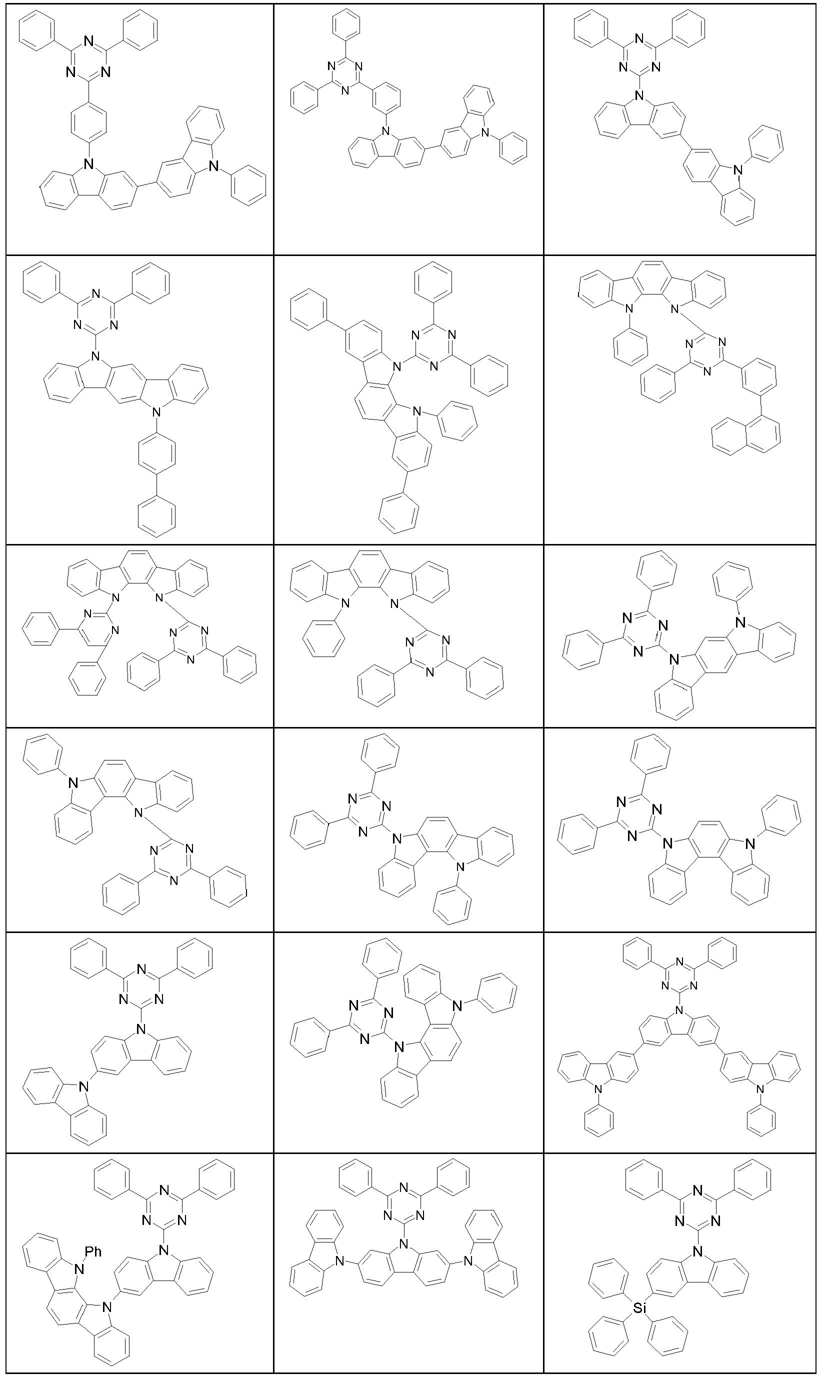

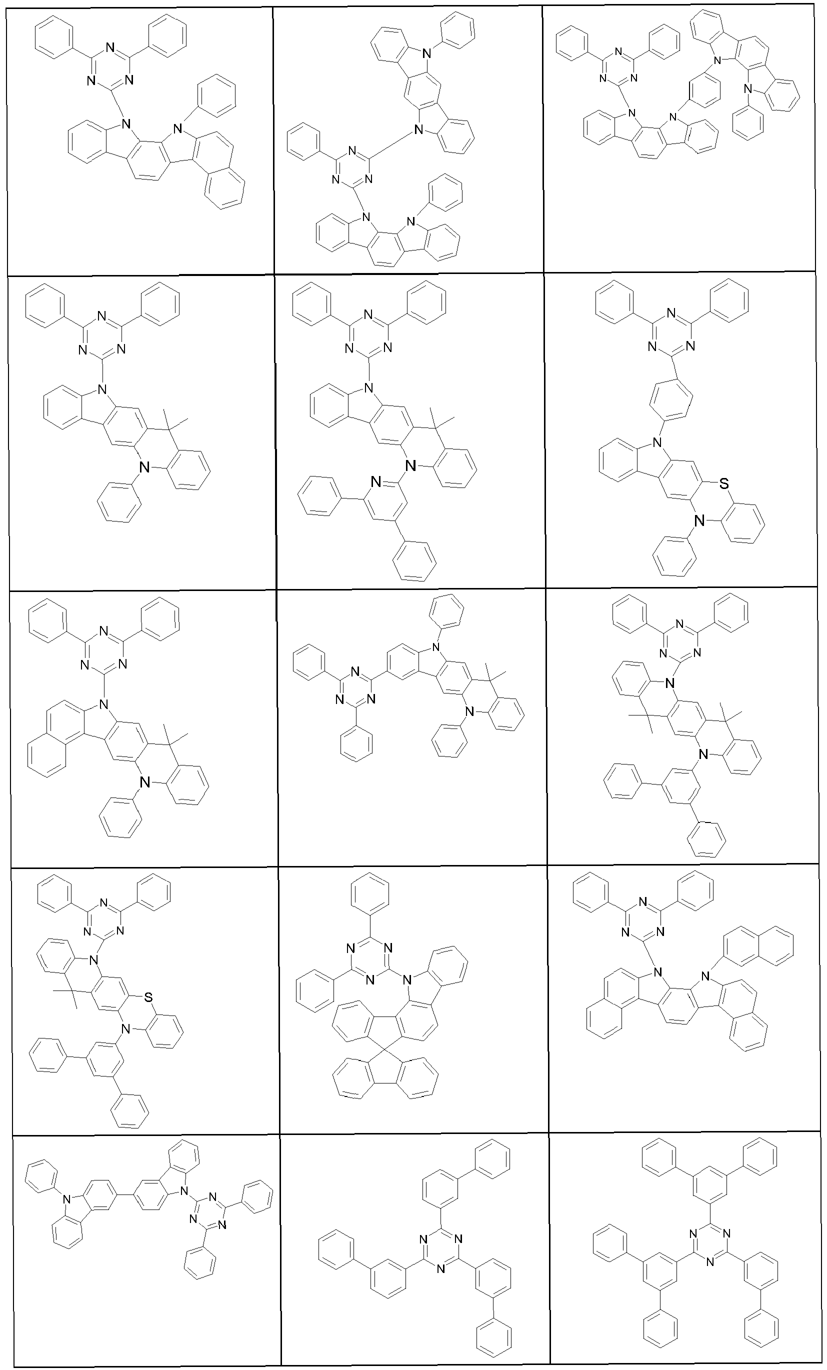

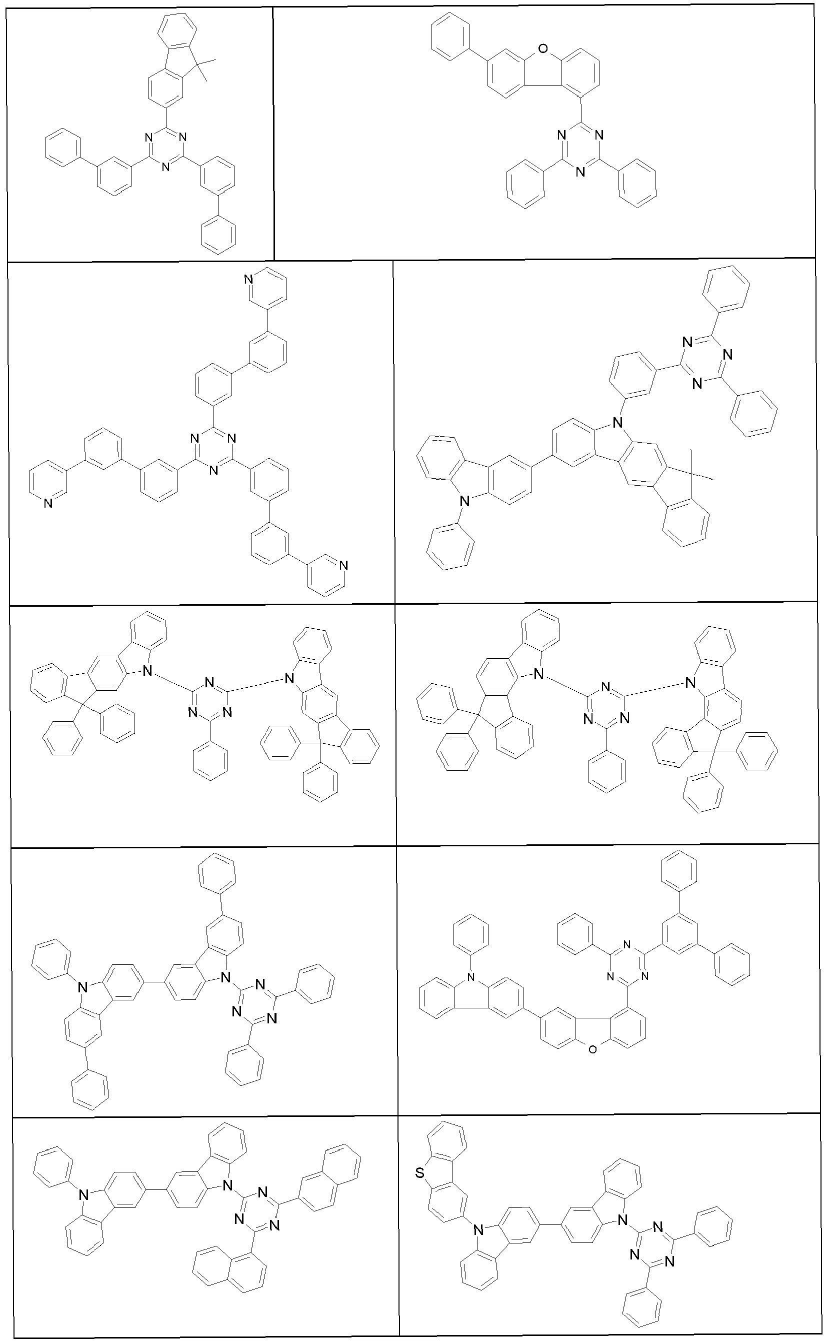

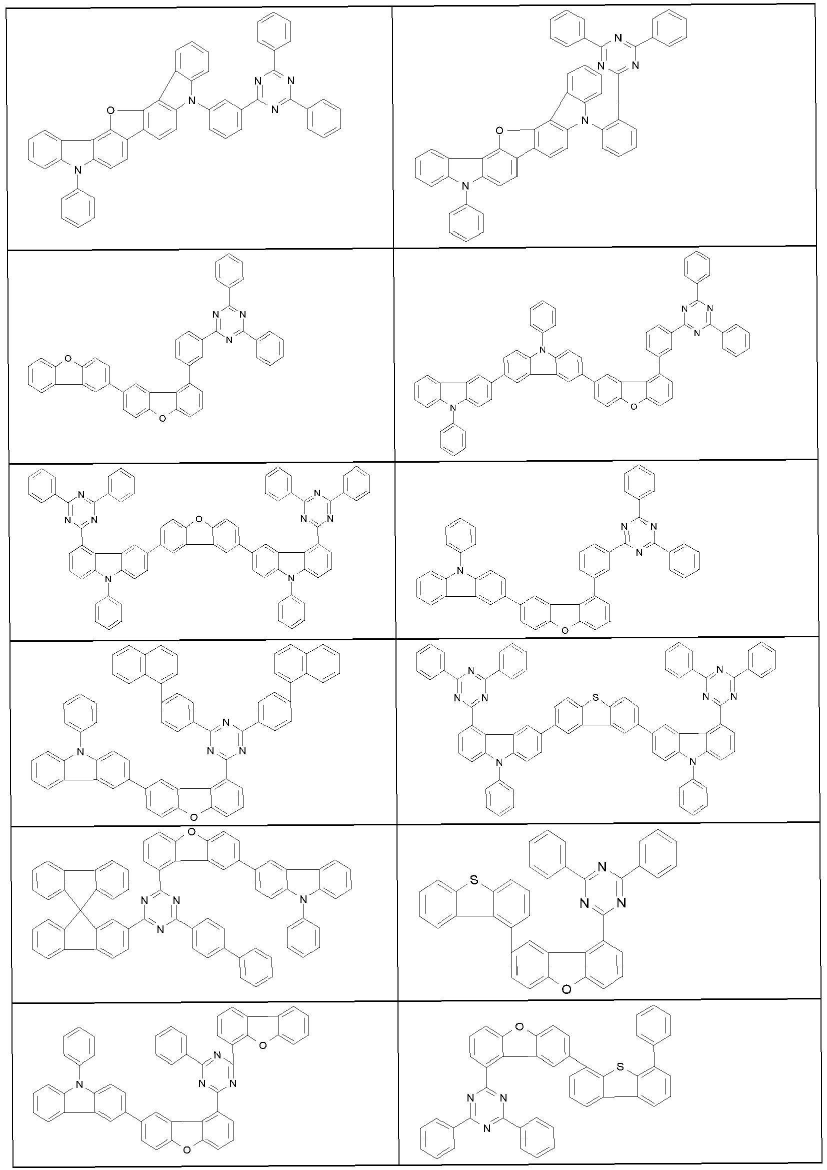

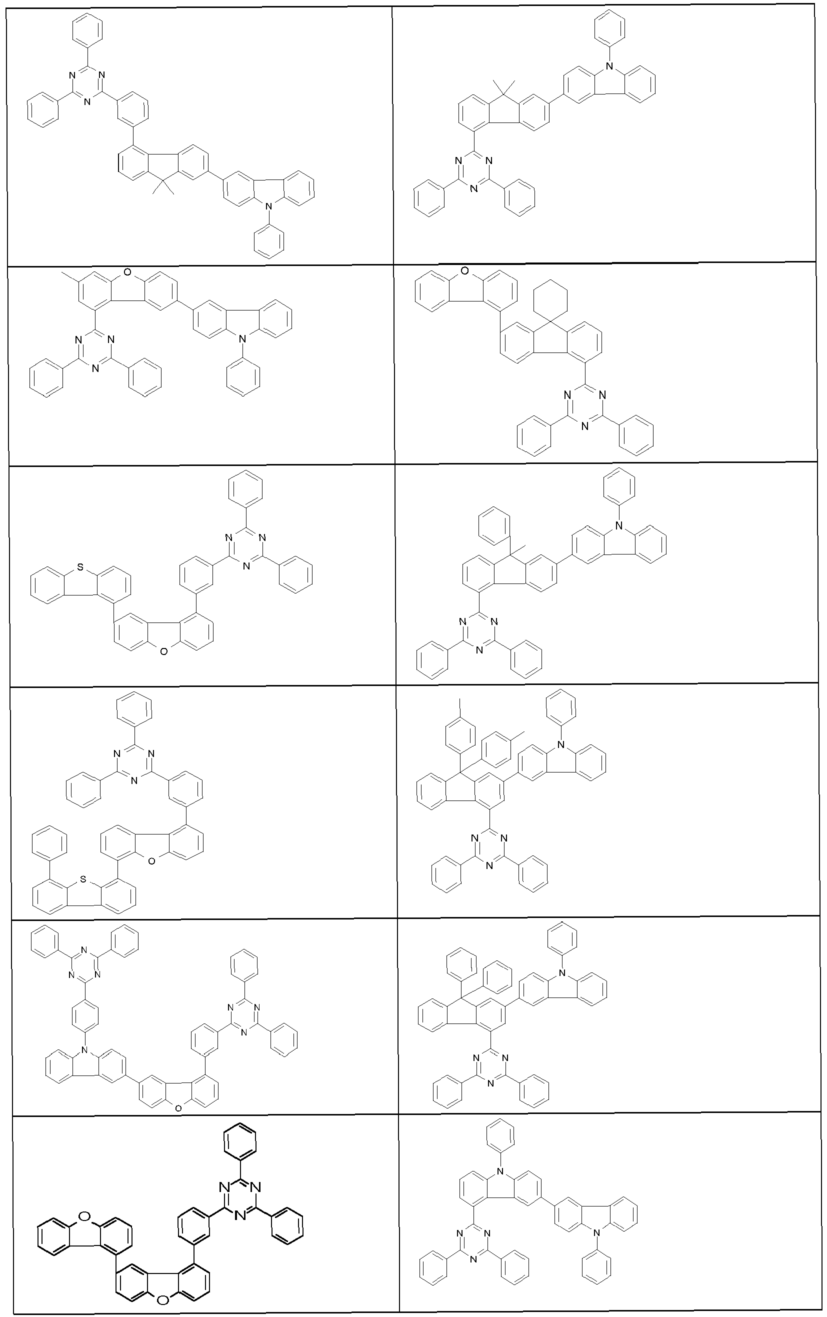

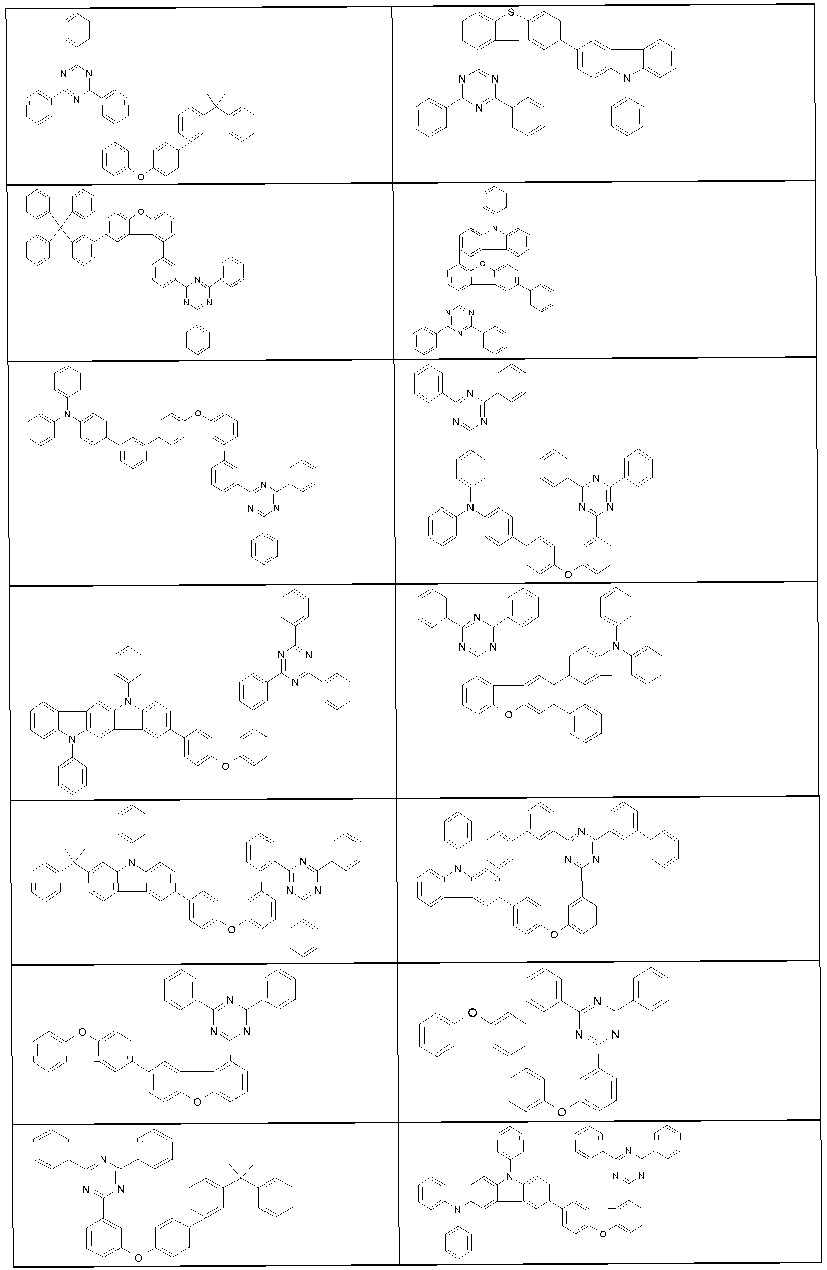

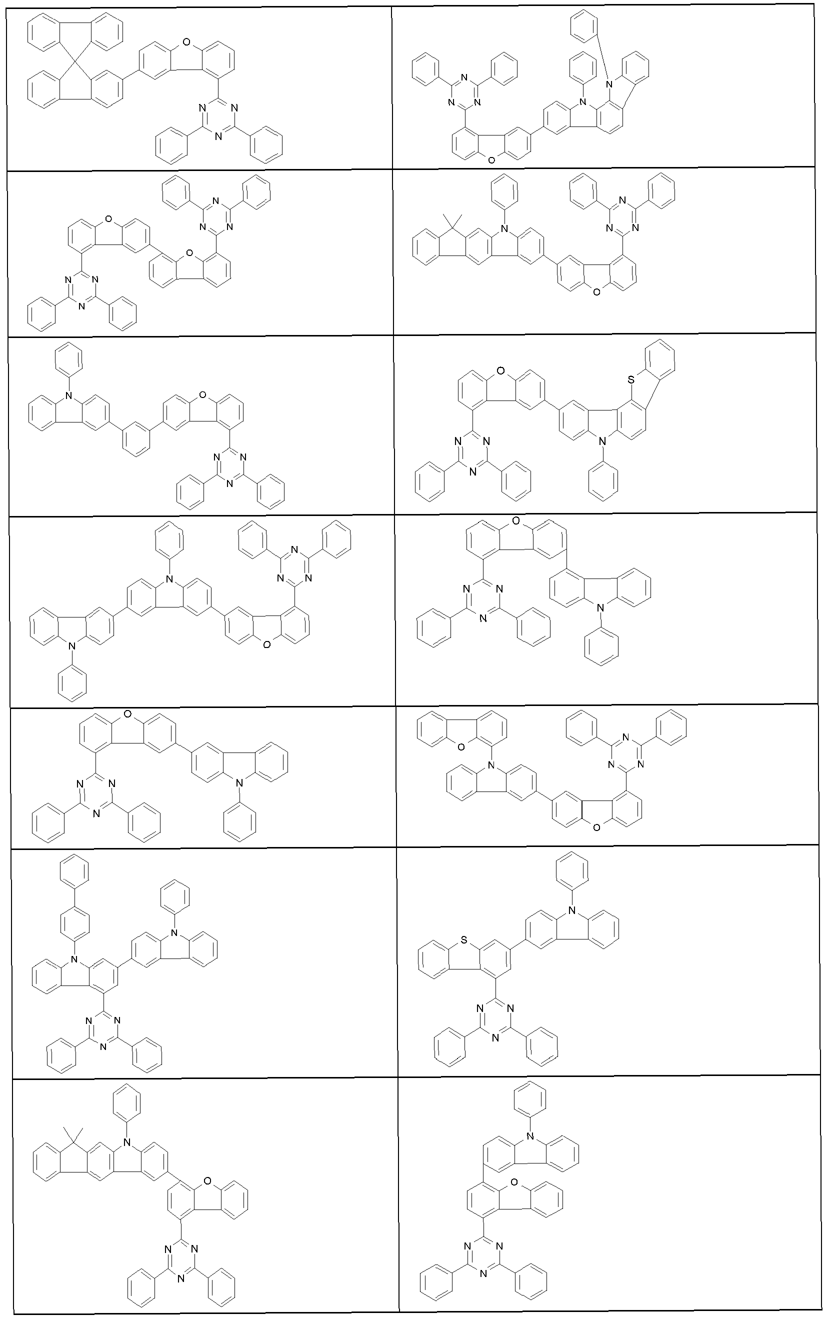

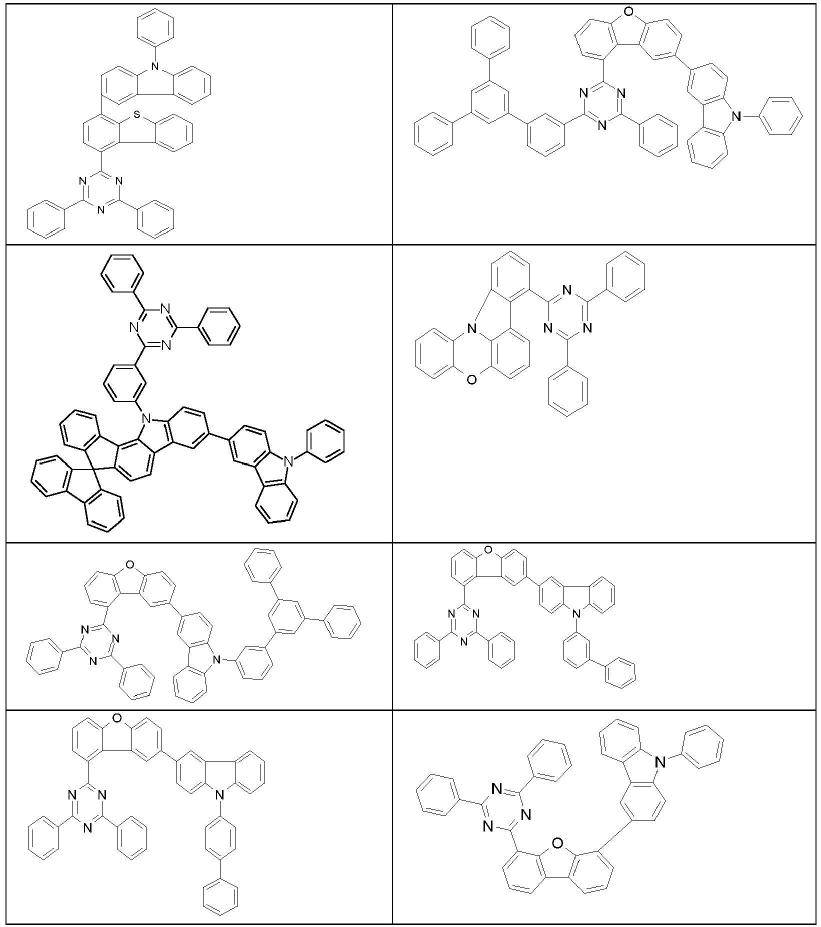

Beispiele für geeignete Chinazolinverbindungen sind die in der folgenden Tabelle abgebildeten Verbindungen:

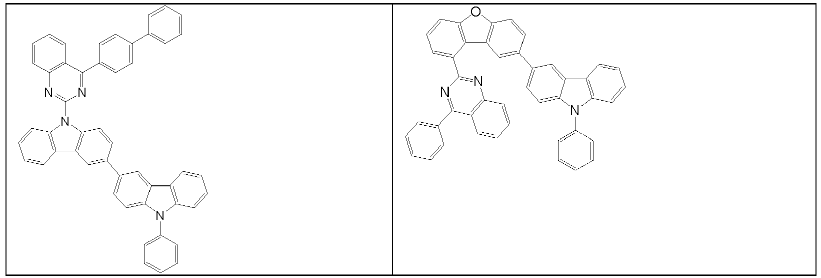

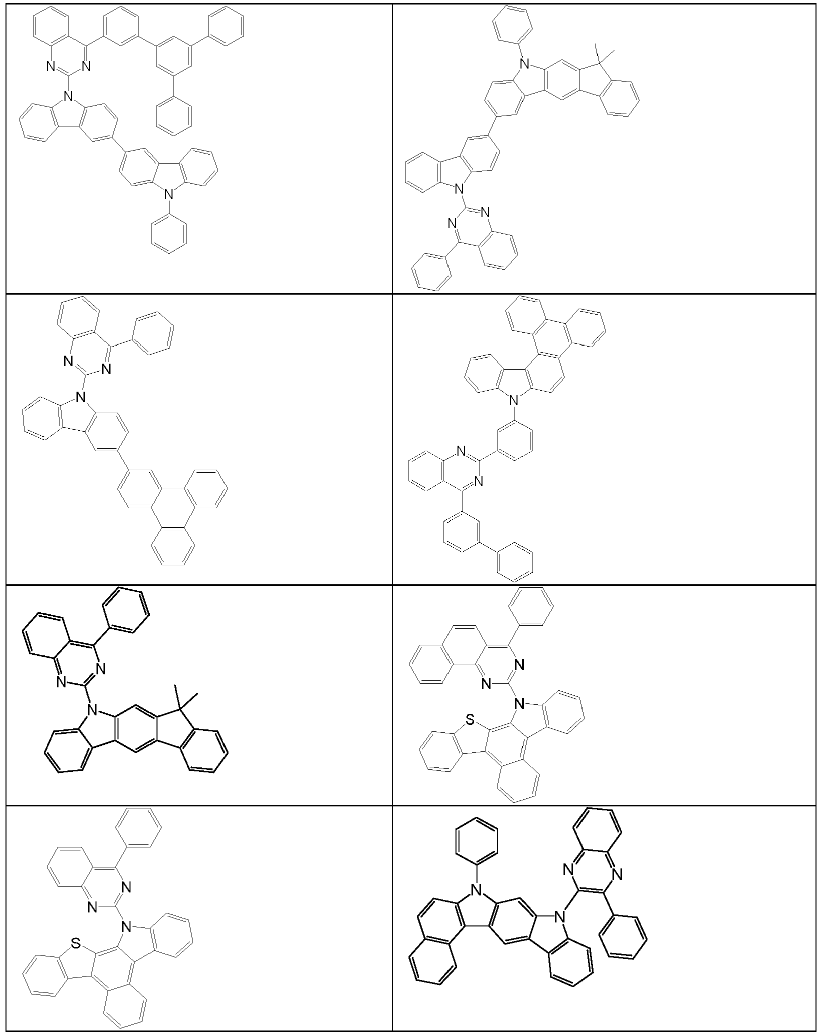

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und WO 2018/041769, WO 2019/020538, WO 2018/178001 sowie den noch nicht offen gelegten Patentanmeldungen EP 17206950.2 und EP 18156388.3 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

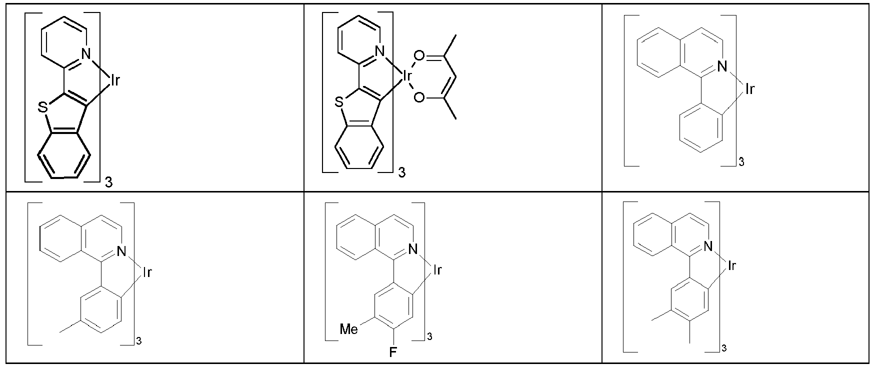

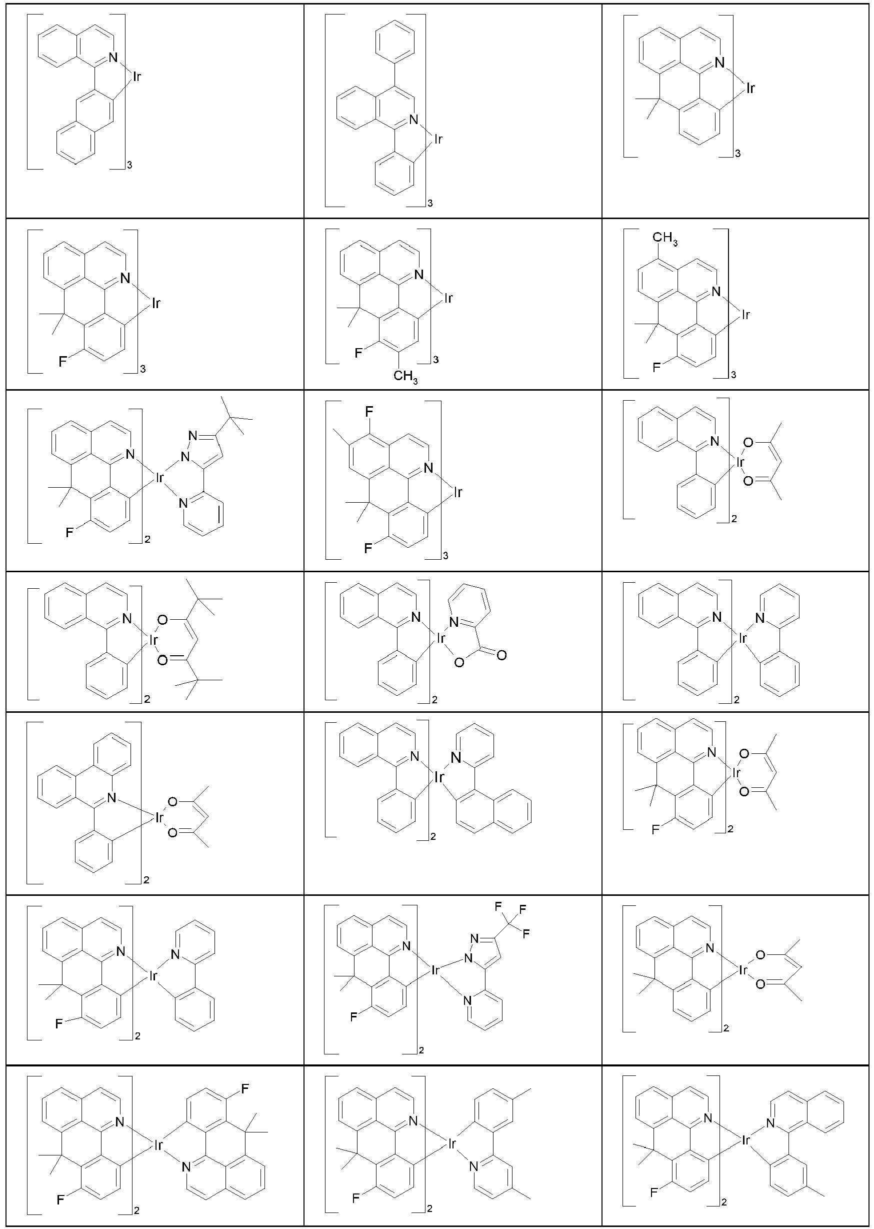

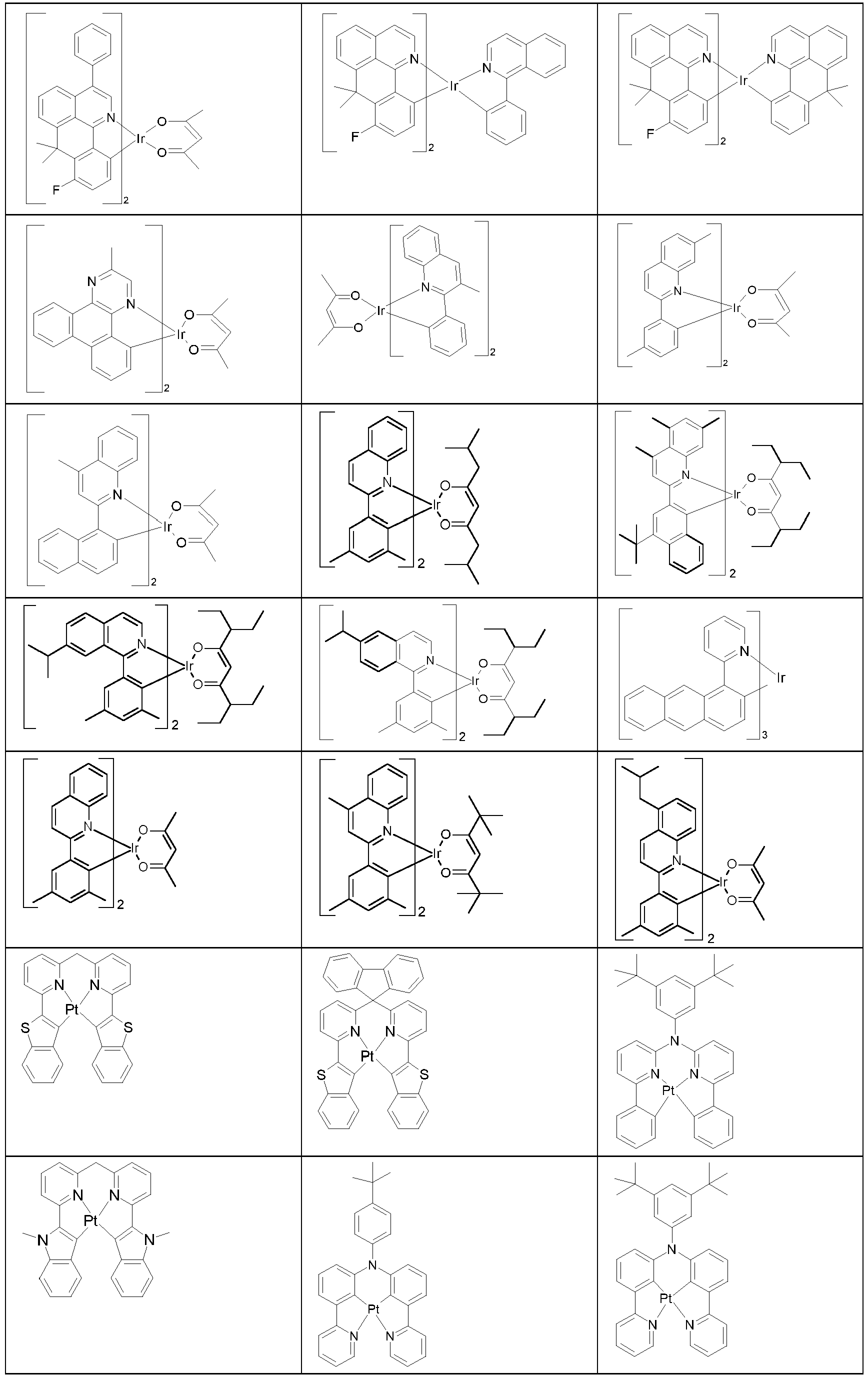

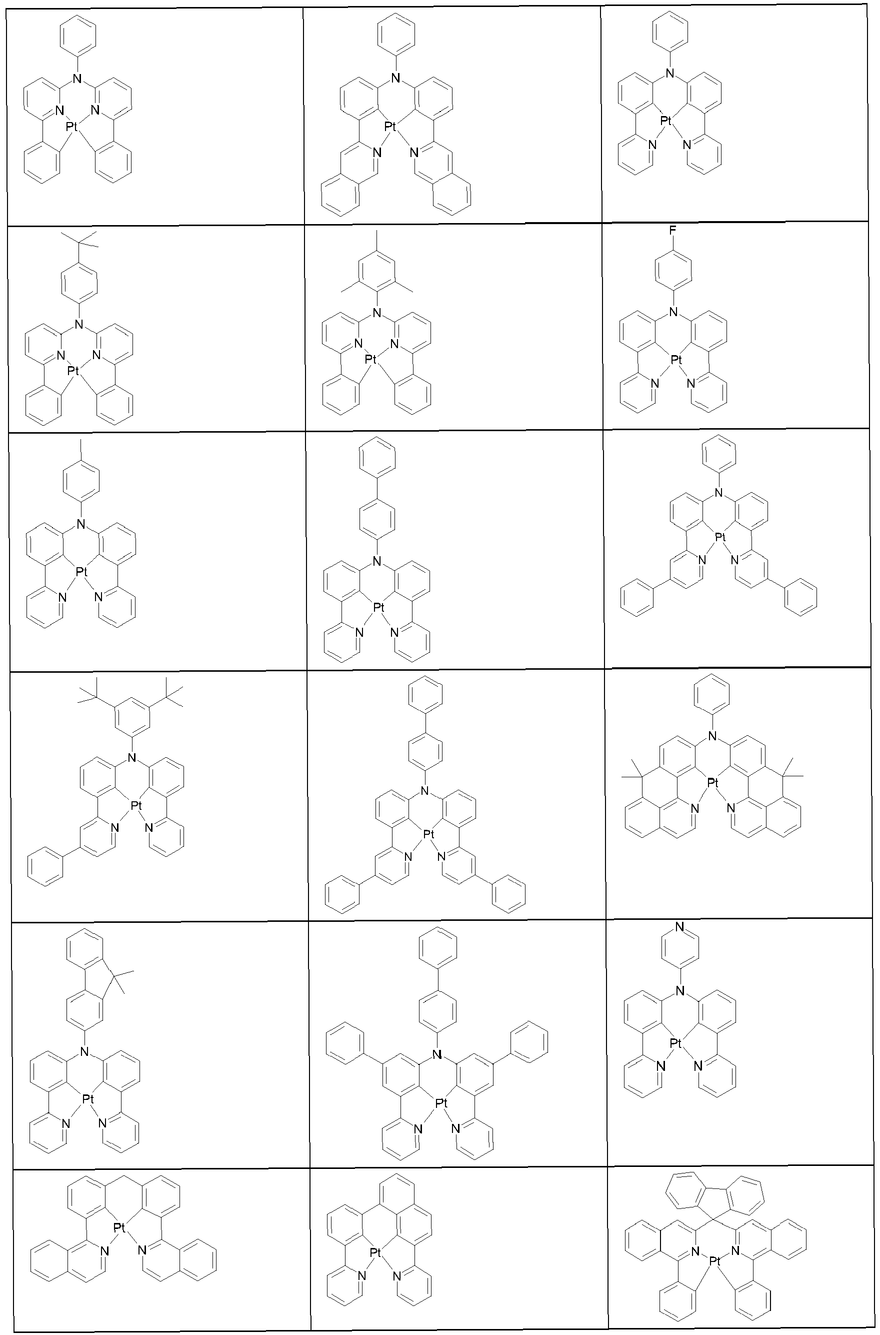

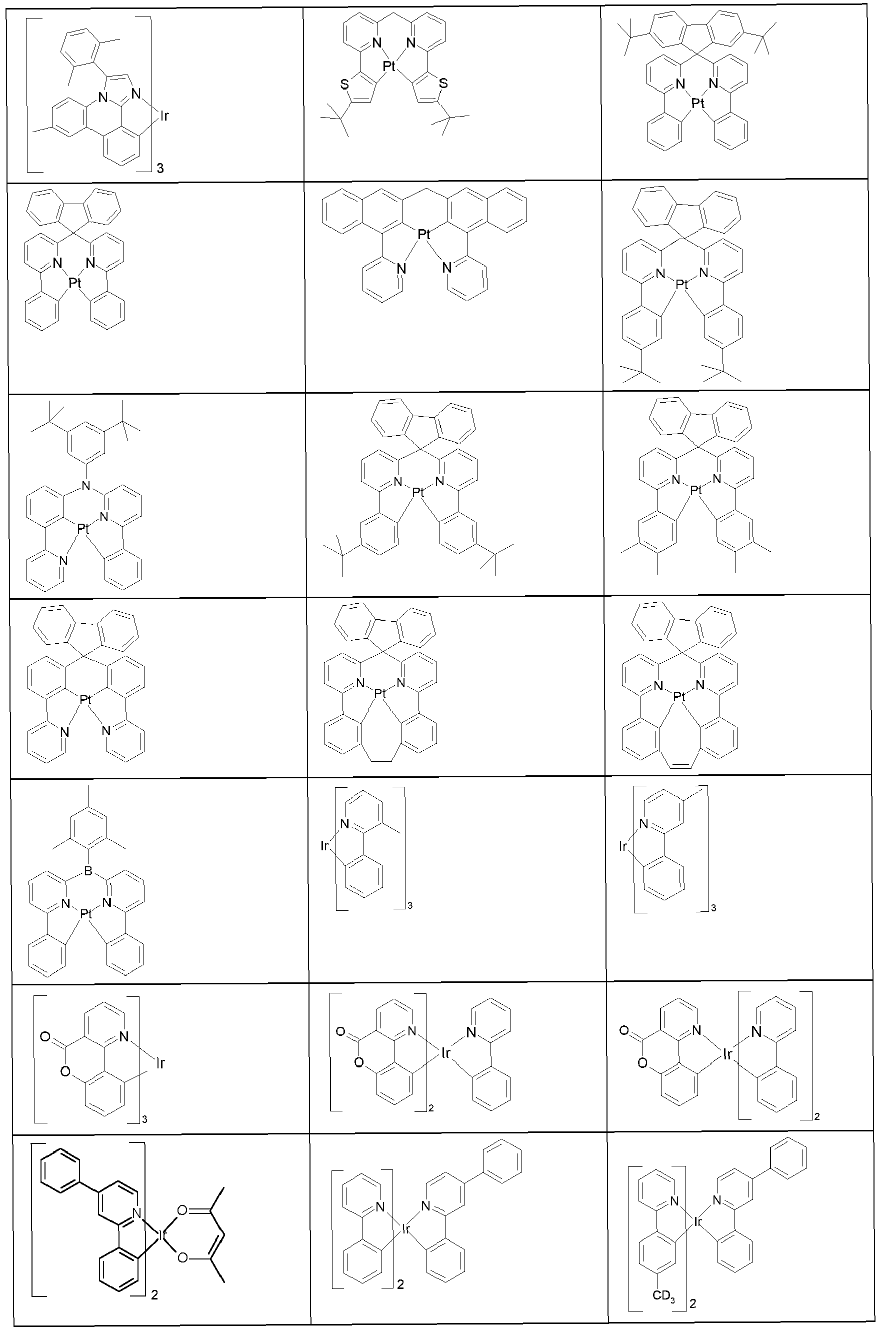

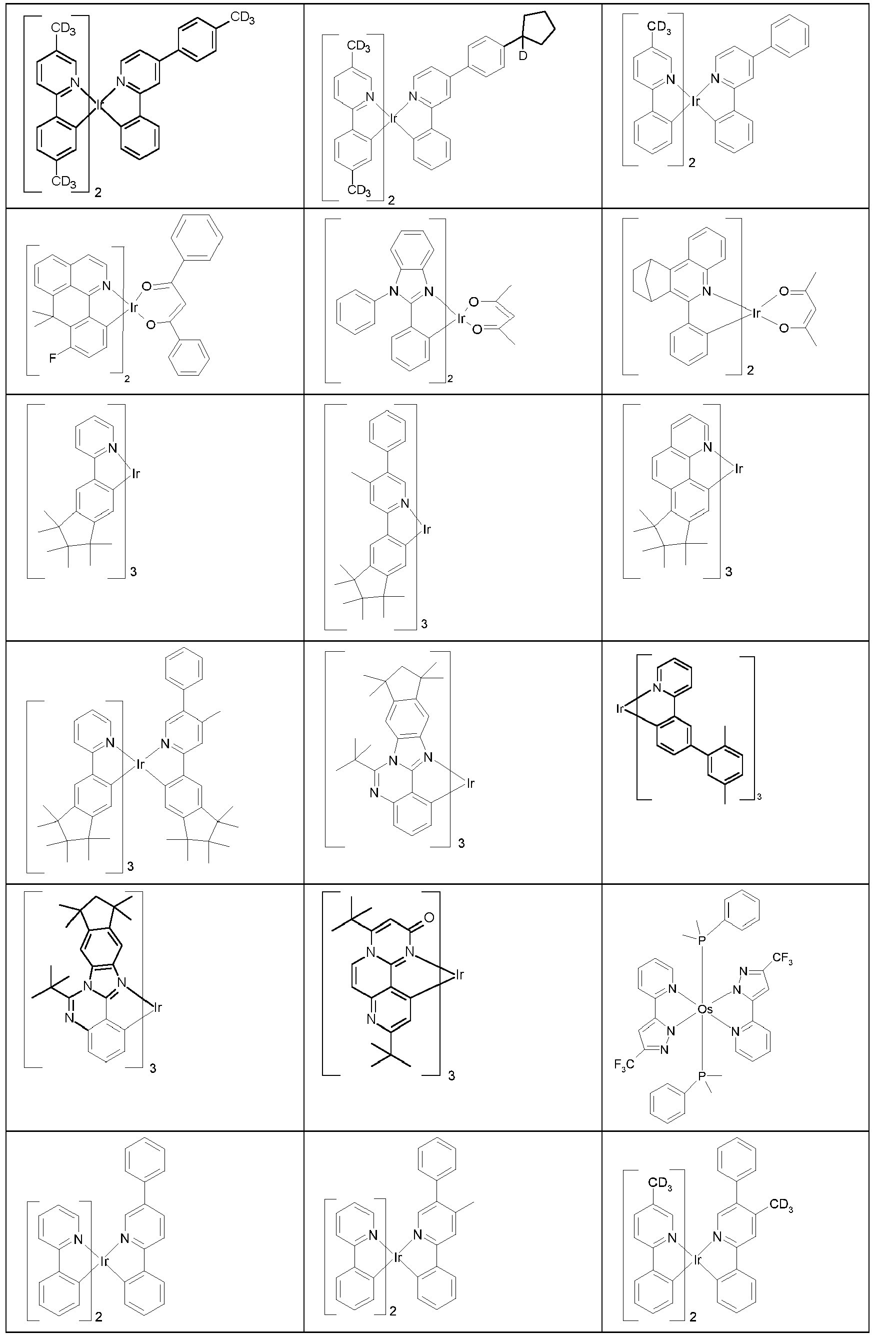

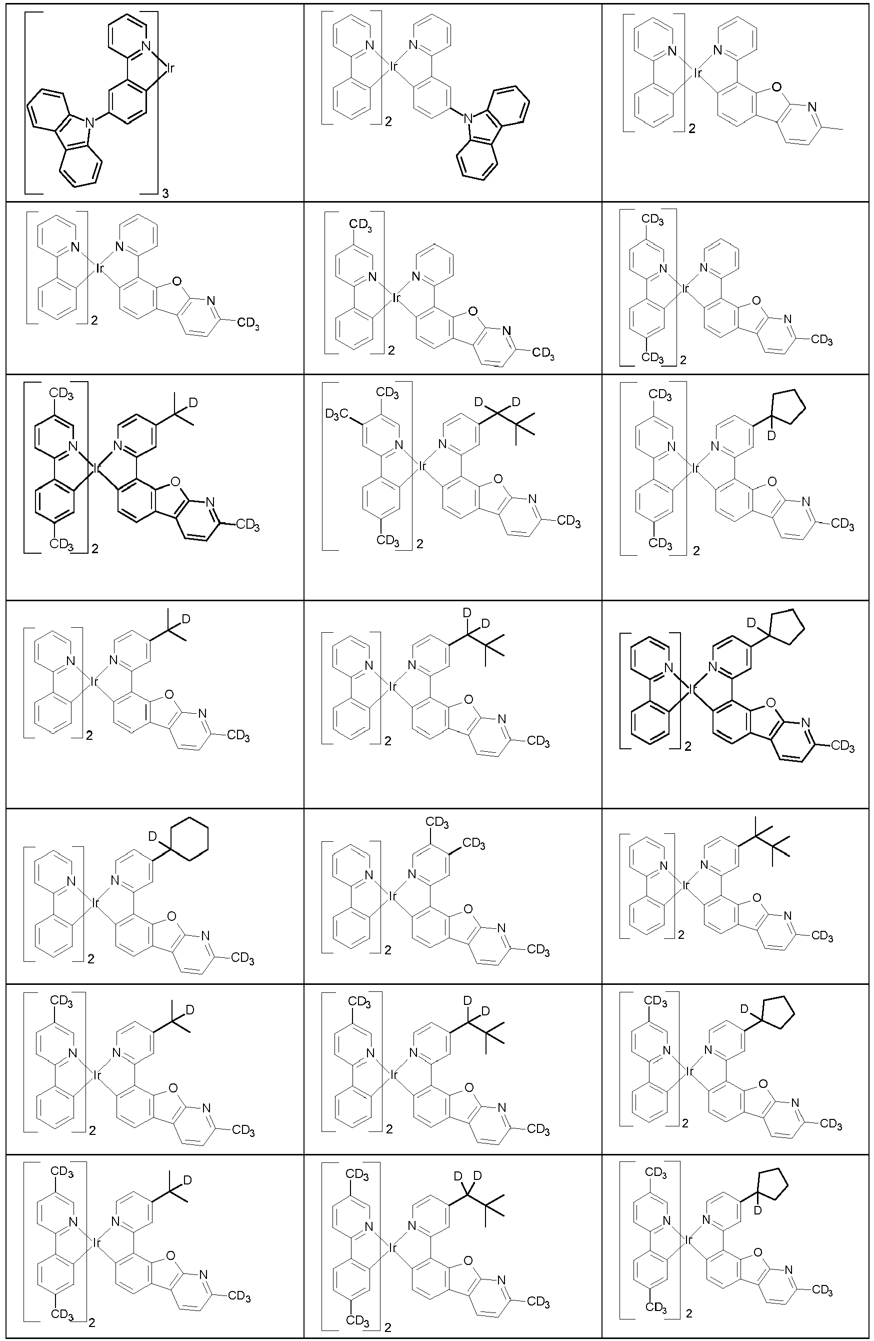

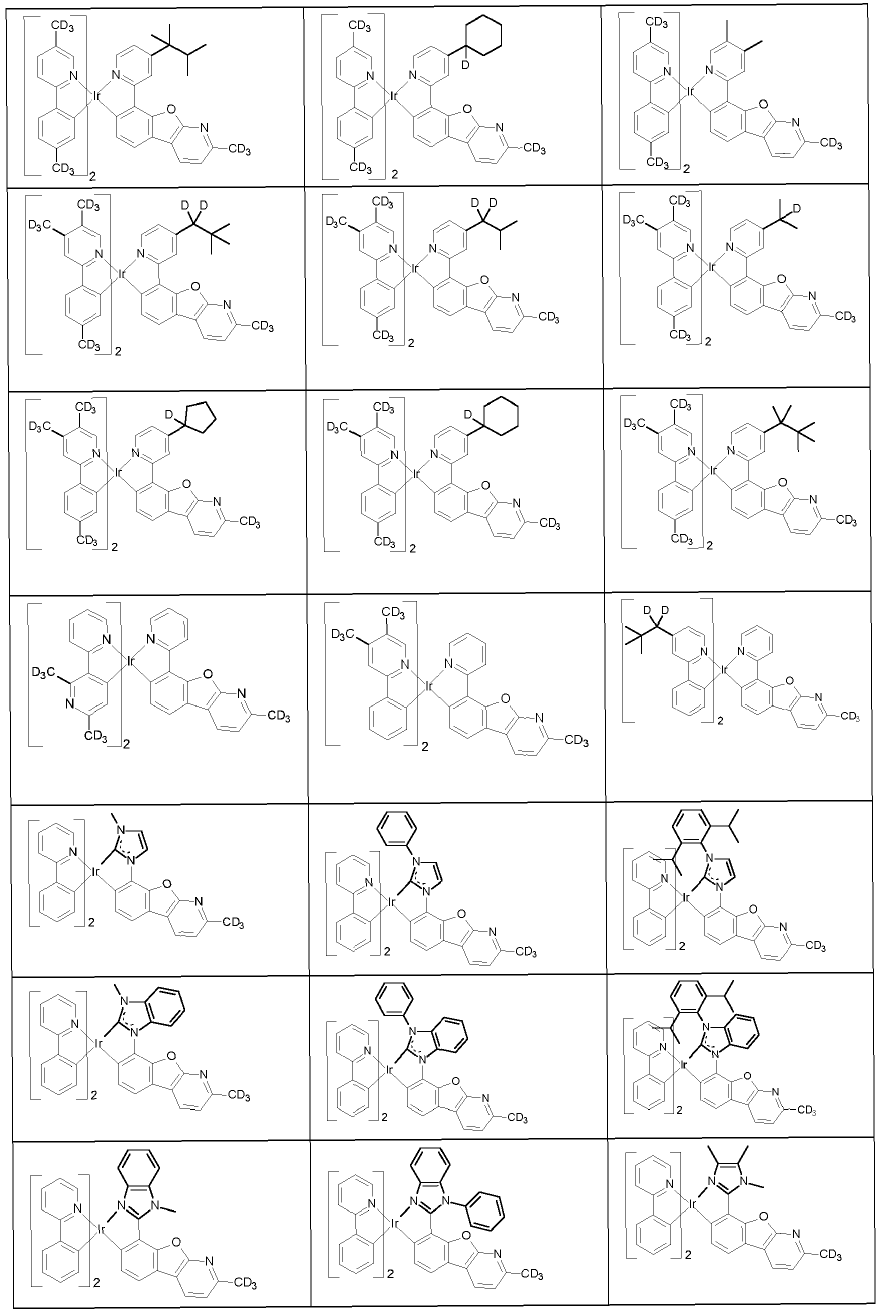

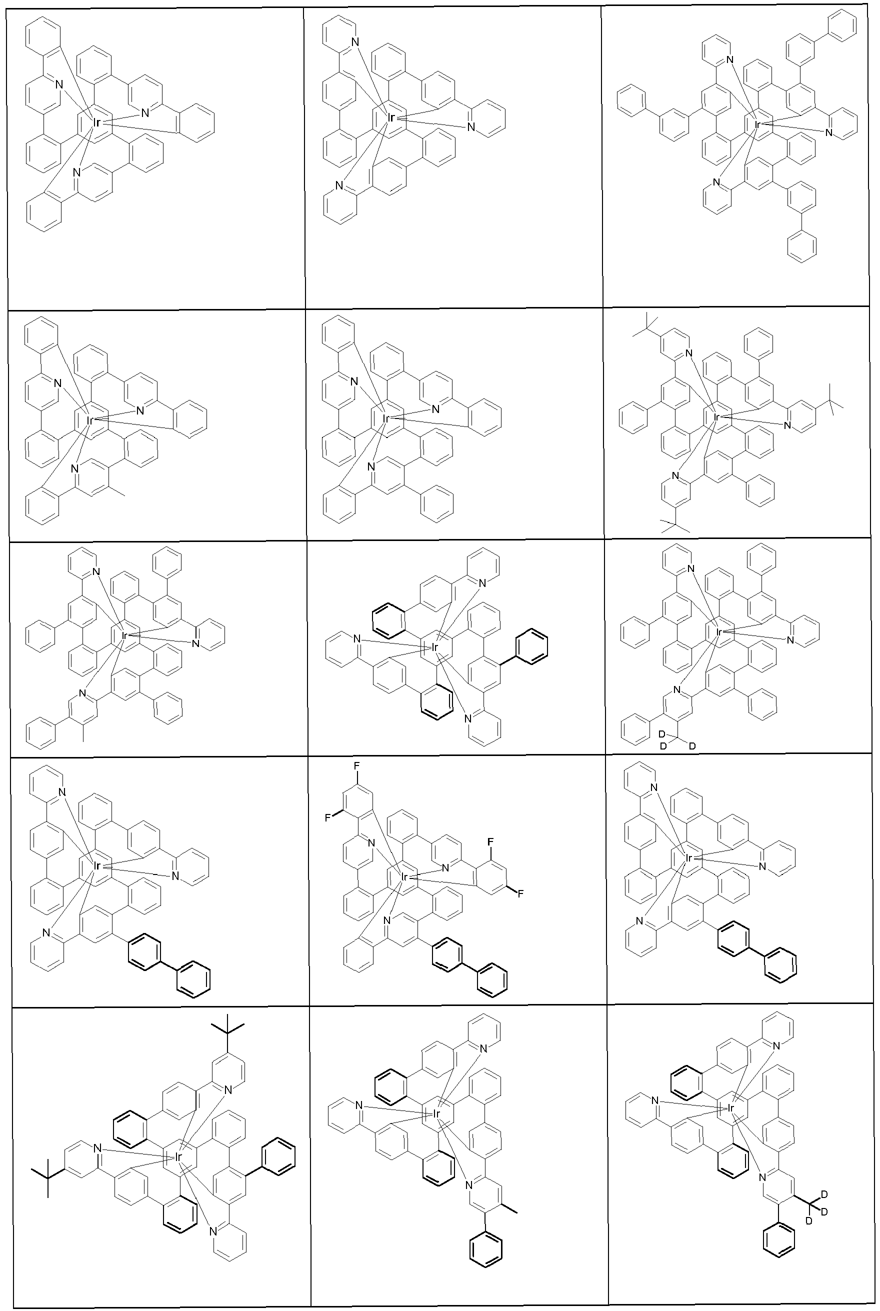

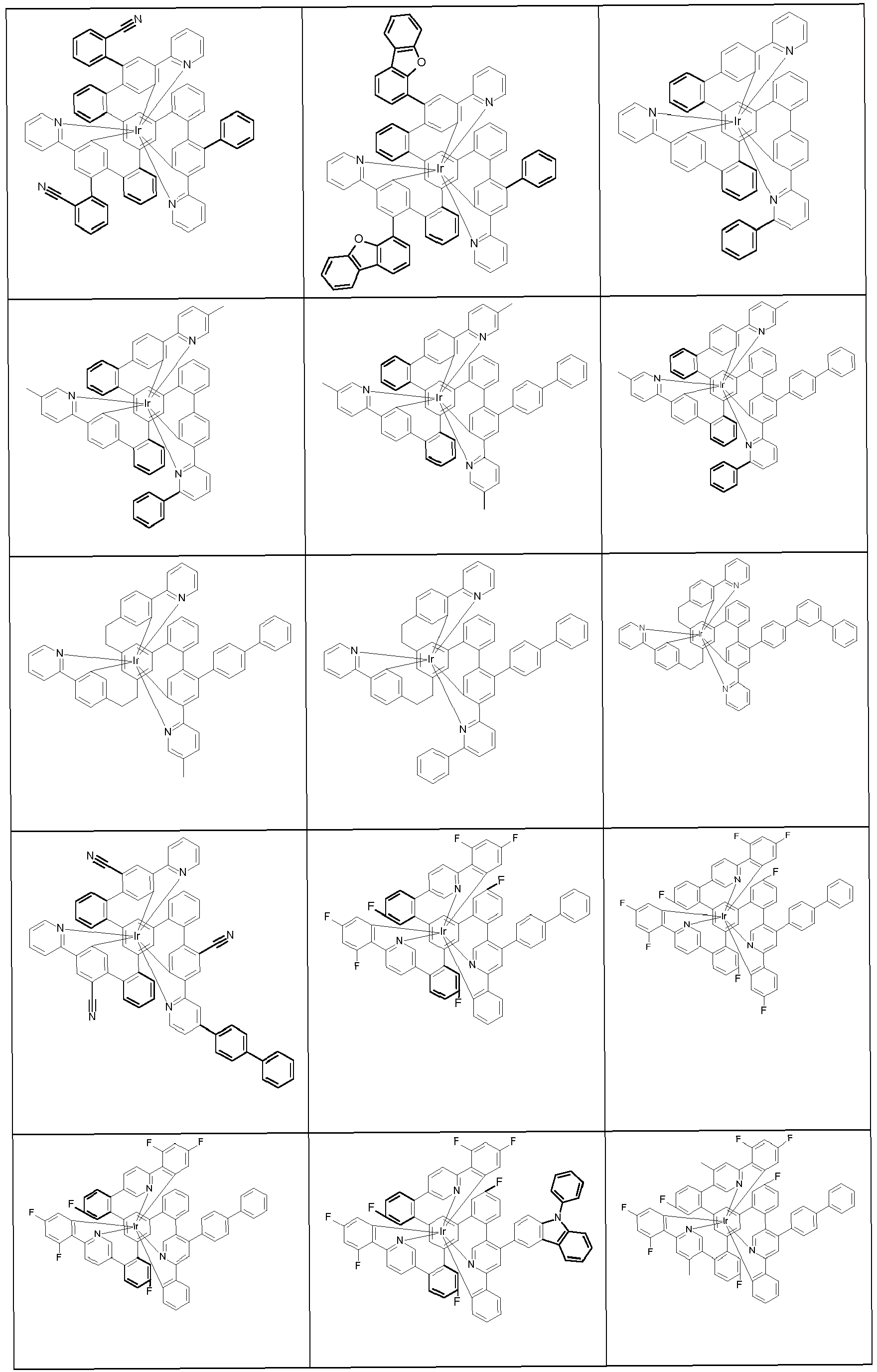

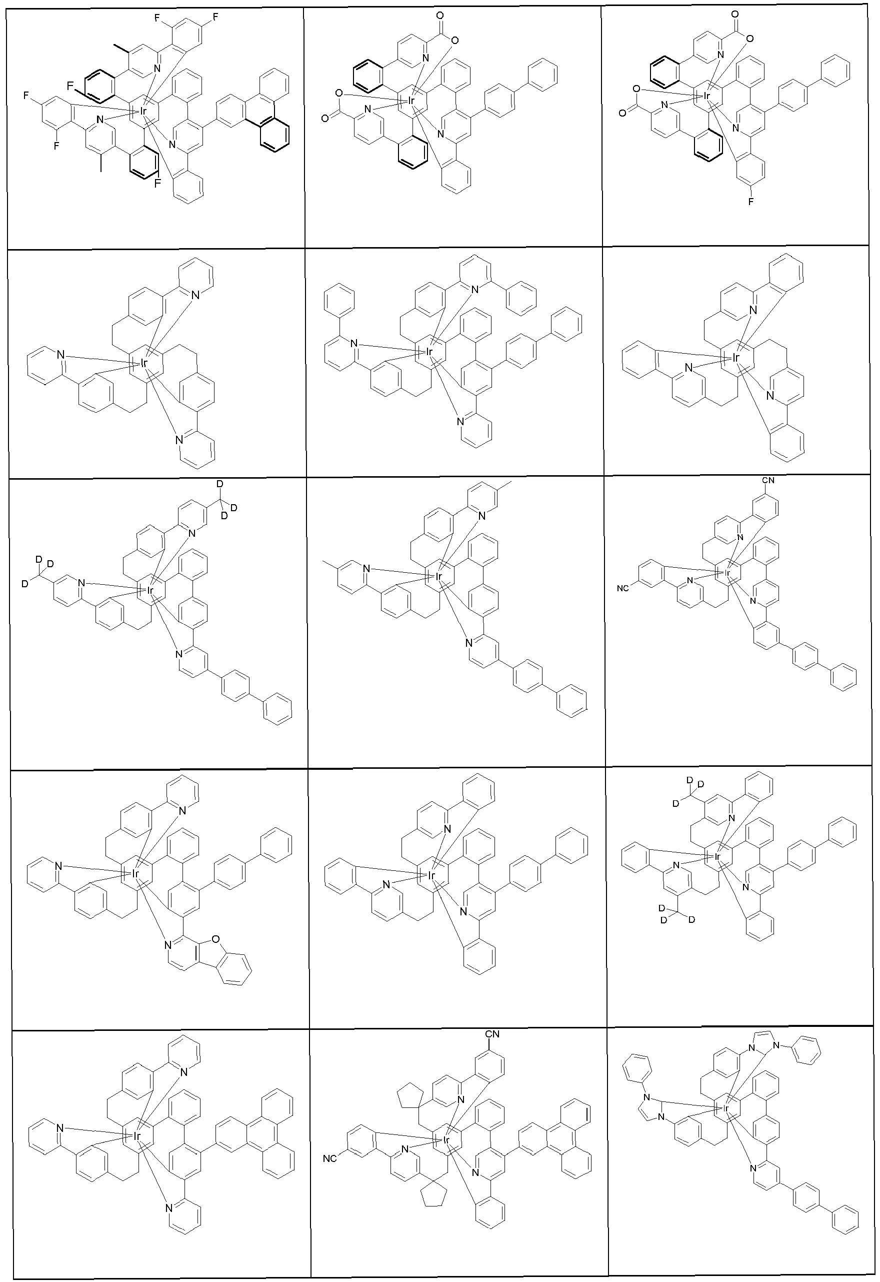

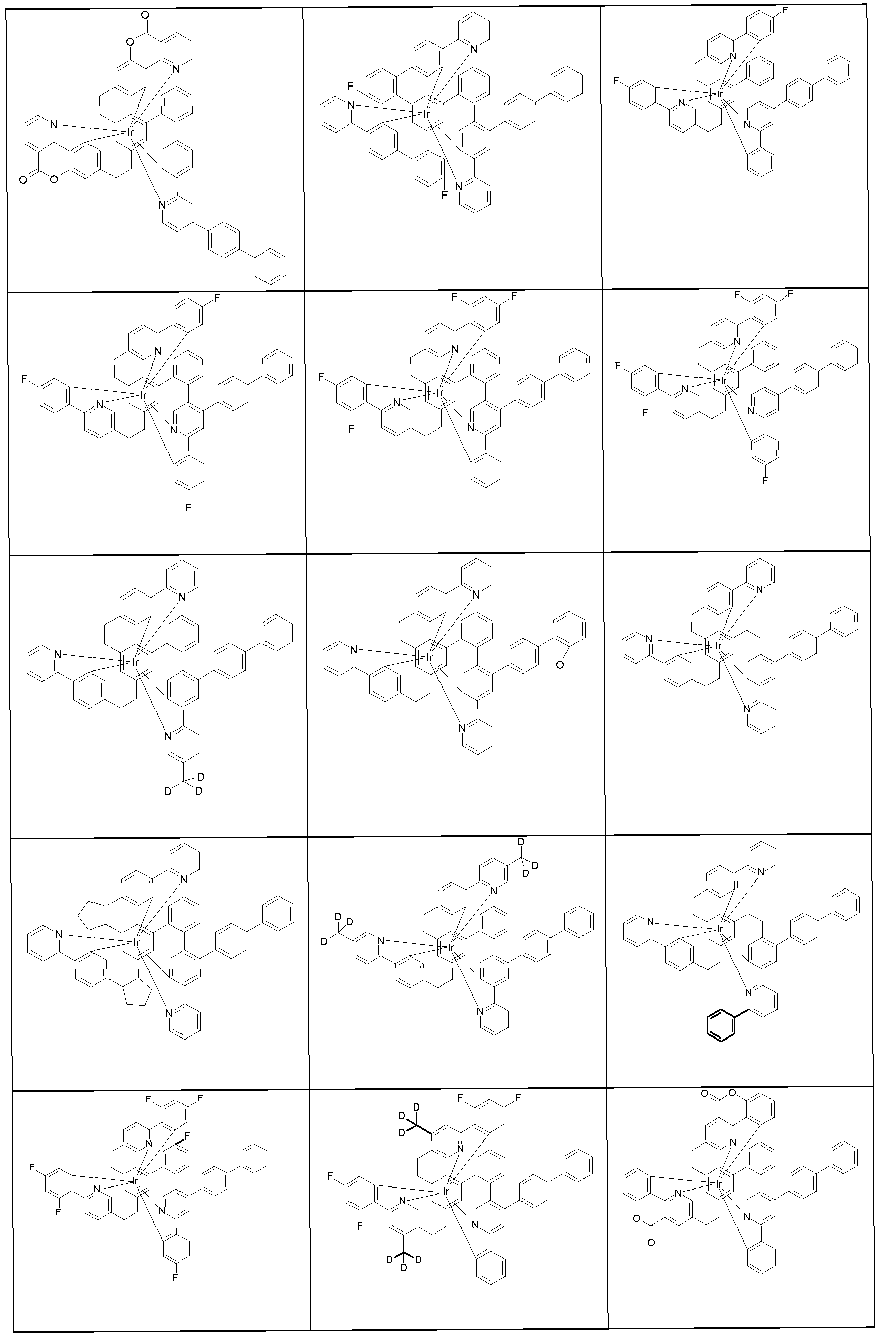

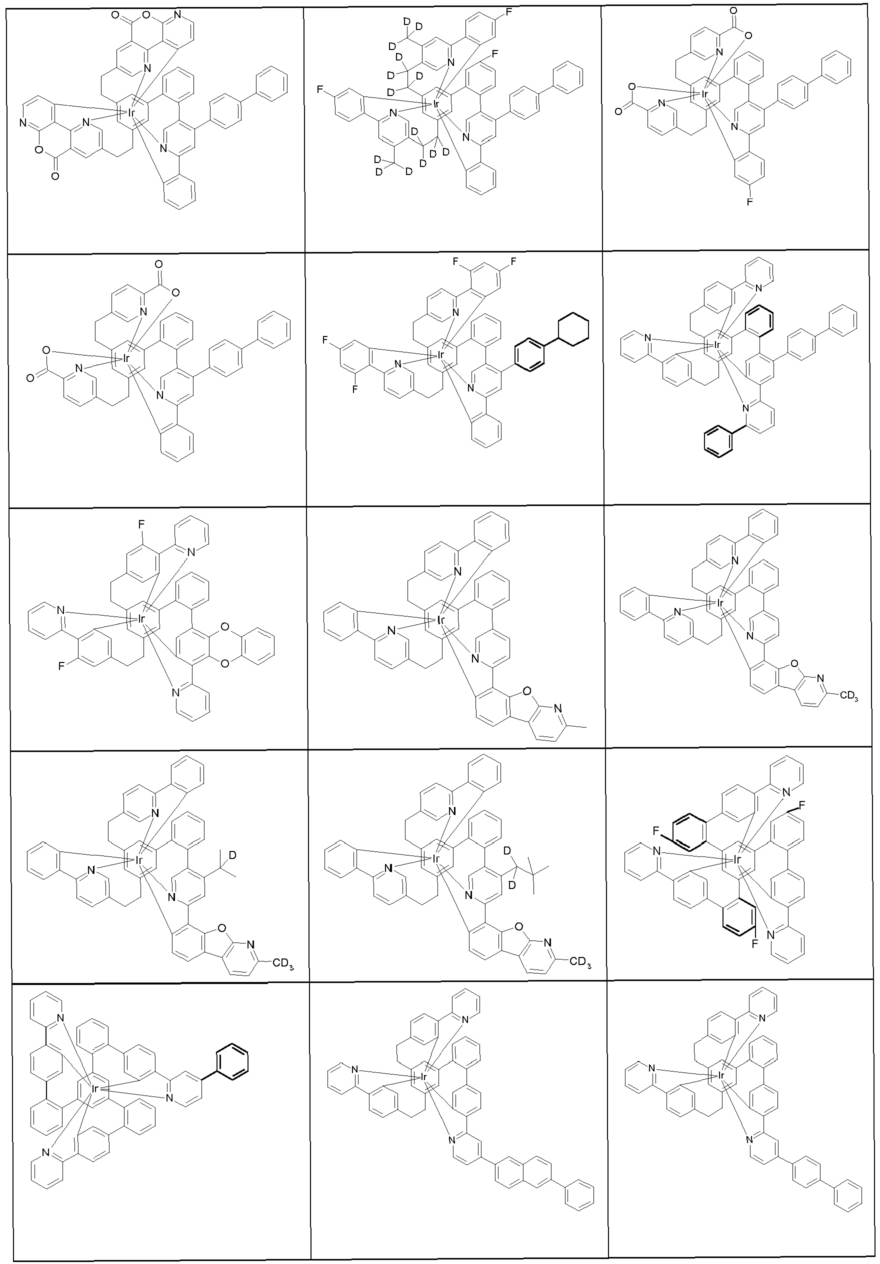

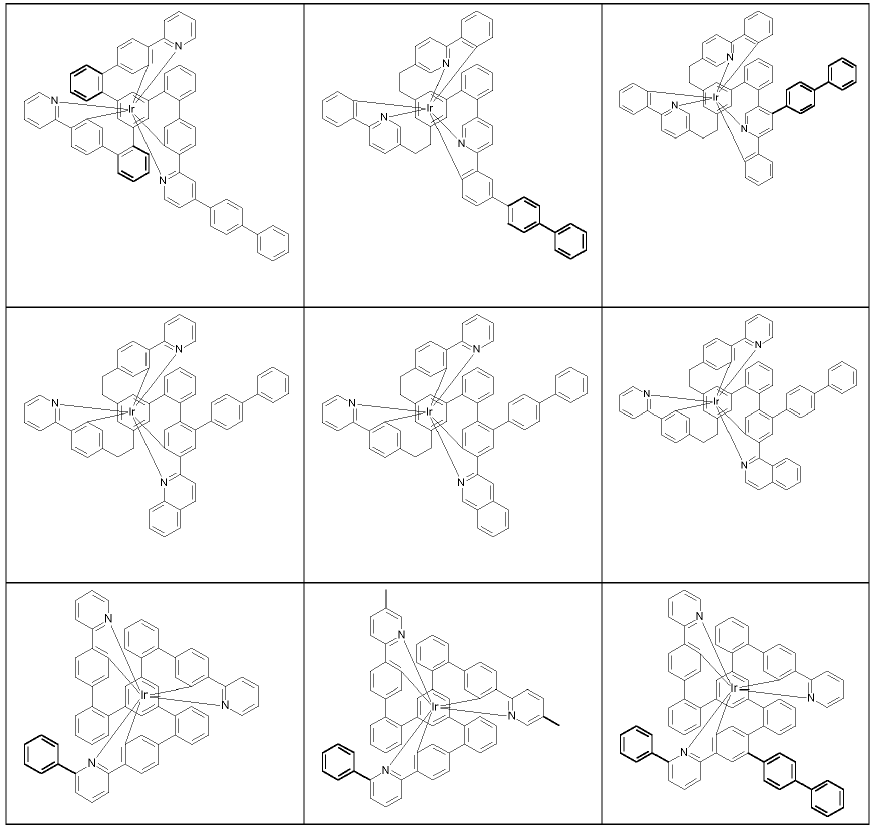

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden Eigenschaften aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

### Synthesebeispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### a) Ethyl-2-(1H-indol-3-yl)-1H-indol-3-carbonsäureester

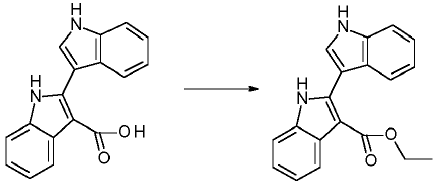

10 g Kaliumhydroxid werden in 20 ml Wasser gelöst, mit 80 ml 95% Ethanol verdünnt und in einem Reaktionskolben vorgelegt. Der Kolben wird in einem Eisbad gekühlt. Mit einem Tropftrichter wird eine Lösung von 10 g (362 mmol) N-Methyl-N-nitroso-p-toluolsulfonamid in 150 ml Diethylether zugegeben. Danach wird destilliert, wobei ein Eisbad unter den Auffangkolben und ein Wasserbad (65 °C) unter den Reaktionskolben platziert wird. Die gelbe Diazomethan/Diethylether-Mischung geht über. Wenn die Gelbfärbung im Reaktionskolben verschwunden ist, ist die Destillation abgeschlossen. Anschließend fügt man noch weitere 50 ml Diethylether hinzu. Das gelbe Destillat enthält Diazomethan (etwa 30 mmol).

### Veresterung:

Zu einer eisgekühlten Lösung von 8.2 g (30 mmol) 2-(1H-Indol-3-yl)-1H-indol-3-carbonsäure (Synthese: siehe Beispiel 1d) in einem Ether/ Ethanol-Gemisch 1:1 (200 ml) gibt man langsam die etherische Diazomethan-Lösung, bis keine Gasentwicklung mehr zu beobachten ist und die schwache Gelbfärbung bestehen bleibt. Man gibt gerade so viel Essigsäure zu, dass die gelbe Farbe verschwindet, und entfernt dann das Lösungsmittel. Ausbeute: 8.2 g (26 mmol); 90% der Theorie.

### b) Ethyl-3-(3-nitroso-1H-indol-2-yl)-1H-indol-2-carboxylat

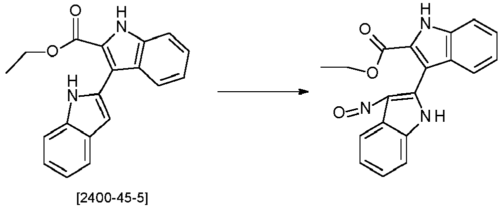

7.9 g (26 mmol) Ethyl-3-(1H-indol-2-yl)-1H-indol-2-carboxylat werden in 50 mL Essigsäure vorgelegt und auf 18 °C gekühlt. Dazu gibt man tropfenweise eine Lösung aus 1.6 g (23.19 mmol) Natriumnitrit, gelöst in 3 ml Wasser, wobei die Temperatur 20 °C nicht übersteigen sollte. Danach rührt man 30 min. bei Raumtemperatur und gibt die Mischung dann auf Eiswasser. Der Feststoff wird abgesaugt und mit Methanol gewaschen. Die Ausbeute beträgt 6.2 g (25.8 mmol); 73% der Theorie.

Analog können folgende Verbindungen erhalten werden:

| | **Edukt** | **Produkt** | **Ausbeute** |
|---|---|---|---|
| 1b | 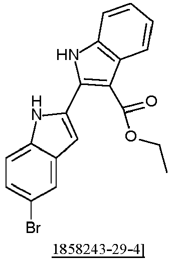 | 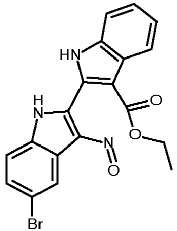 | 68% |
| 2b | 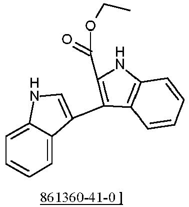 | 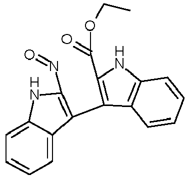 | 65% |
| 3b | 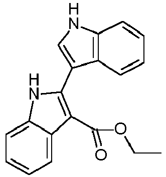 | 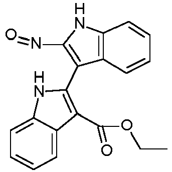 | 73% |

### c) Cyclisierung

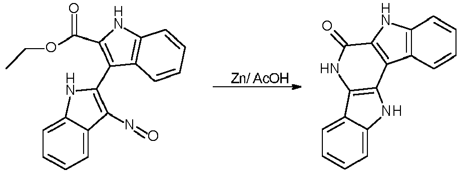

43 g (129 mmol) Ethyl-3-(3-nitroso-1H-indol-2-yl)-1H-indol-2-carboxylat und 60 g (931 mmol) Zinkpulver werden in 500 ml Essigsäure bei 80 °C für 12 h gerührt. Der Ansatz wird abgekühlt und der ausgefallene Feststoff abgesaugt. Der Rückstand wird aus DMF umkristallisiert. Ausbeute: 24.6 g (90 mmol); 70% der Theorie.

Analog können folgende Verbindungen erhalten werden:

| | **Edukt** | **Produkt** | **Ausbeute** |
|---|---|---|---|
| 1c | 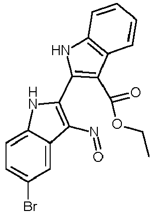 | 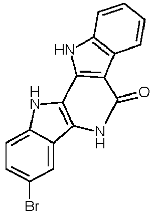 | 68% |
| 2c | 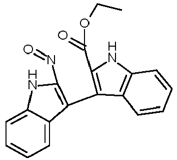 | 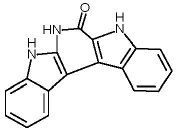 | 65% |
| 3c | 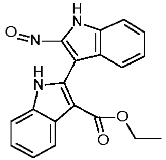 | 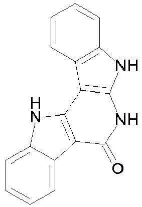 | 71% |

### d) 2-Brom-1-phenyl-indol-3-carbonsäure

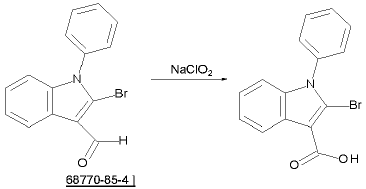

Bei Raumtemperatur werden 49.5 g (165 mmol) 2-Brom-1-phenyl-indol-3-carboxyaldehyd, 200 ml 2-Methyl-2-buten und 600 ml Dioxan vorgelegt. Dazu gibt man tropfenweise eine Lösung aus 81.9 g (902mmol) NaClO₂ und 81.9 g (590 mmol) NaH₂PO₄·H₂O in 410 mL Wasser. Nach 2.5 h gibt man wieder 20 g NaClO₂ and 20 g NaH₂PO₄·H₂O zu und nach 5 h jeweils 10 g von beiden Salzen. Die Lösung wird zweimal mit 300 ml Essigester extrahiert, eingeengt und mit 300 ml 1%iger NaOH extrahiert. Die wässrige Phase wird mit HCl auf pH-Wert 3 gebracht und das ausgefallene Produkt abgetrennt. Die Ausbeute beträgt 36.5 g (121 mmol); 70% der Theorie.

Analog kann die folgende Verbindung erhalten werden:

| | **Edukt** | **Produkt** | **Ausbeute** |
|---|---|---|---|
| 1d | 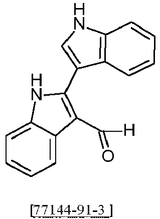 | 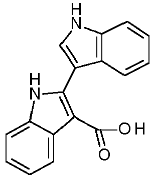 | 78% |

### e) 3-Brom-N,1-diphenyl-indol-2-carboxamid

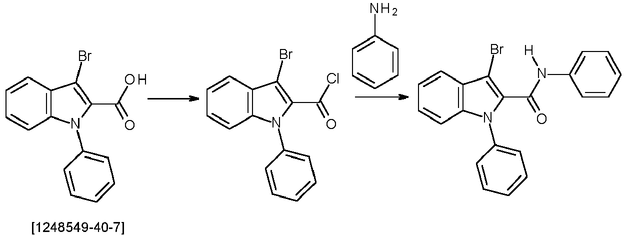

73 g (230 mmol) 3-Brom-1-phenyl-indol-2-carbonsäure werden in 1300 ml Methylenchlorid vorgelegt und mit 10 Tropfen DMF versetzt. Bei Raumtemperatur werden 86.3 ml (1020 mmol) Oxalylchlorid in 400 ml Methylenchlorid zugetropft und 5 h bei 45 °C gerührt. Das Lösungsmittel wird im Vakuum entzogen. Der Feststoff wird in 200 ml CH₂Cl₂ gelöst und auf 0 °C gekühlt. Anschließend tropft man 21 ml (230 mmol) Anilin zu und lässt dann 2 h bei Raumtemperatur ruhen. Nach Zugabe von 100 ml gesättigter NaHCOs-Lösung trennt man die organische Phase ab und kristallisiert den Rückstand aus Toluol um. Ausbeute: 73 g (187 mmol); 81 % der Theorie.

Analog können folgende Verbindungen hergestellt werden:

| | **Edukt 1** | **Edukt 2** | **Produkt** | **Ausbeute** |
|---|---|---|---|---|
| 1e | 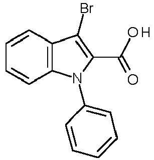 | 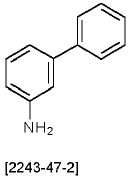 | 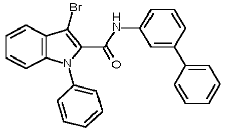 | 65% |
| 2e | 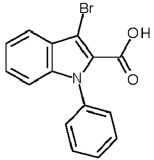 | 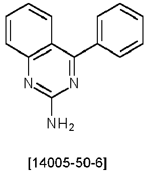 | 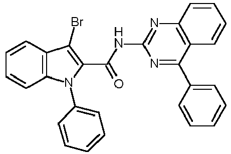 | 54% |
| 3e | 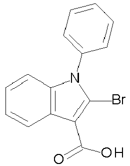 | 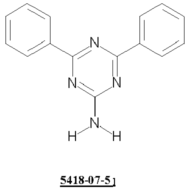 | 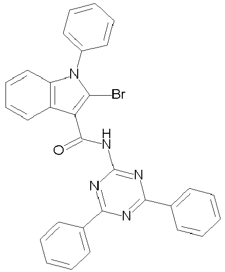 | 46% |

### f) Cyclisierung

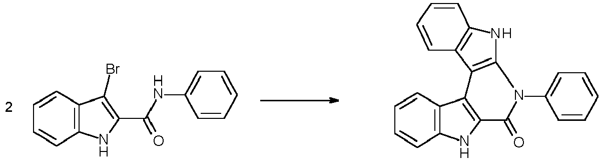

Unter Schutzgas werden 15.7 g (45 mmol) 3-Brom-N-phenyl-1H-indol-2-carboxamid, 5 g (2.2 mmol) Pd(OAc)₂, 10 g (4.5 mmol) Tri-2-furylphosphin und 12 g (90 mmol) K₂CO₃ in 1000 ml DMF bei 150 °C für 30 h gerührt. Die Lösung wird mit Wasser verdünnt und mit Essigester zweimal extrahiert. Die vereinigten organischen Phasen werden über Na₂SO₄ getrocknet und einrotiert. Der Rückstand wird chromatographisch (EtOAc/Hexan: 2/3) gereinigt. Der Rückstand wird aus Toluol umkristallisiert. Die Ausbeute beträgt 8.8 g (25 mmol); 70% der Theorie.

Analog können folgende Verbindungen hergestellt werden:

| | **Edukt** | **Produkt** | **Ausbeute** |
|---|---|---|---|
| 1f | 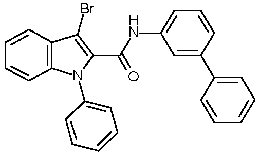 | 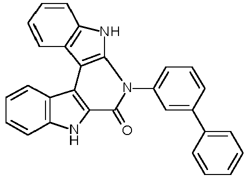 | 65% |
| 2f | 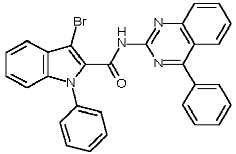 | 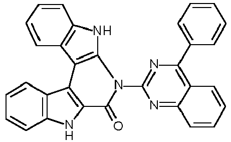 | 68% |
| 3f | 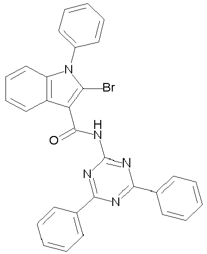 | 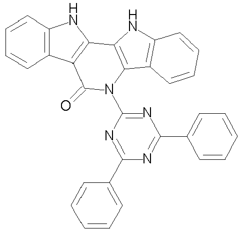 | 56% |

### g) 5-(9-Phenylcarbazol-3-yl)-2,9-dihydropyrido[3,4-b]indol-1-on

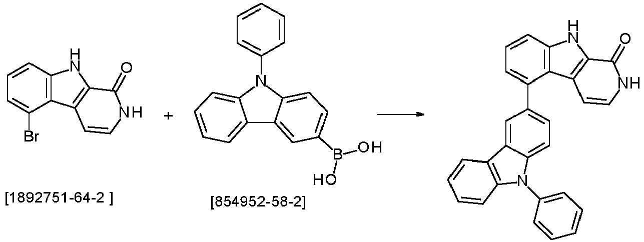

19.1 (73 mmol) 5-Brom-2,9-dihydropyrido[3,4-b]indol-1-on, 20.8 g (75 mmol) Phenylcarbazol-3-boronsäure und 14.7 g (139 mmol) Natriumcarbonat werden in 200 mL Toluol, 52 mL Ethanol und 100 mL Wasser suspendiert. Zu dieser Suspension werden 80 mg (0.69 mmol) Tetrakistriphenylphosphin-palladium(0) gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Heptan / Dichlormethan umkristallisiert. Die Ausbeute beträgt 25 g (60 mmol); 83% der Theorie.

Analog können folgende Verbindungen hergestellt werden:

| | **Edukt 1** | **Edukt 2** | **Produkt** | **Ausbeute** |
|---|---|---|---|---|
| 1g | 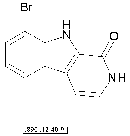 | 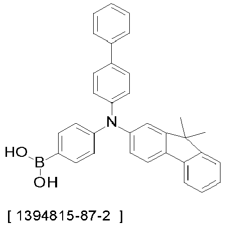 | 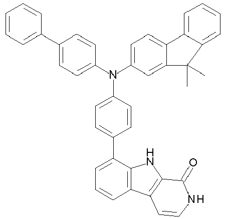 | 72% |
| 2g | 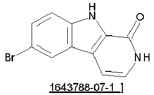 | 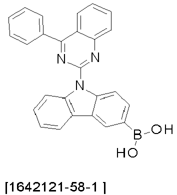 | 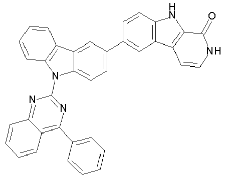 | 70% |
| 3g | 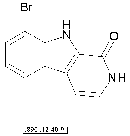 | 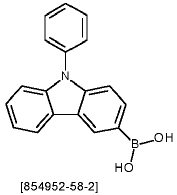 | 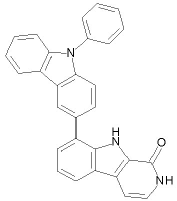 | 72% |
| 4g | 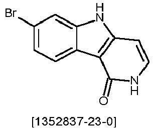 | 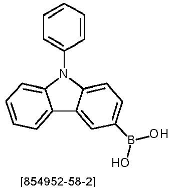 | 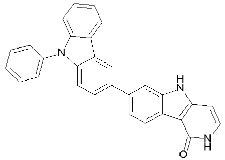 | 71% |
| 5g | 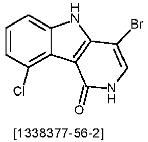 | 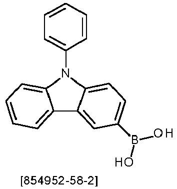 | 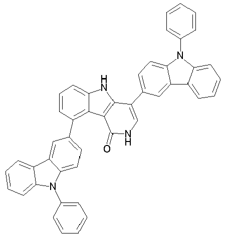 | 68% |

### h) 9-(4,6-Diphenyl-1,3,5-triazin-2-yl)-5-(9-phenylcarbazol-3-yl)-2H-pyrido[3,4-b]indol-1-on

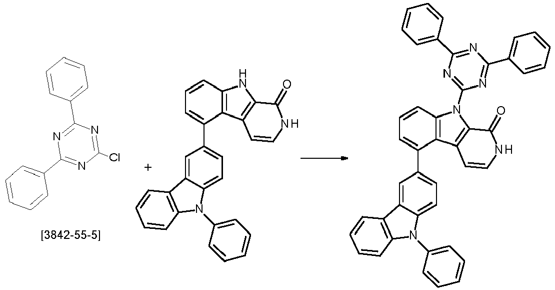

41 g (51 mmol) 5-(9-Phenylcarbazol-3-yl)-2,9-dihydropyrido[3,4-b]indol-1-on und 16 g (60 mmol) 2-Chlor-4,6-diphenyl-[1,3,5]triazin werden in 400 ml Toluol unter Argonatmosphäre gelöst. 1.0 g (5 mmol) Tri-tert-butylphosphin wird zugegeben und unter Argonatmosphäre gerührt. 0.6 g (2 mmol) Pd(OAc)₂ werden zugegeben und unter Argonatmosphäre gerührt, wonach 9.5 g (99 mmol) Natium-tert-butanolat zugegeben werden. Das Reaktionsgemisch wird 24 h unter Rückfluss gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM/Heptan (1:4)) gereinigt. Die Ausbeute beträgt 38 g (58 mmol); 60% der Theorie.

Bei **8h, 14h** und **15h** wird der Rückstand wird aus Toluol umkristallisiert und abschließend im Hochvakuum (p = 5 x 10-5 mbar) sublimiert. Die Reinheit beträgt 99.9%.

Analog können folgende Verbindungen hergestellt werden:

| | **Edukt 1** | **Edukt 2** | **Produkt** | **Ausbeute** |
|---|---|---|---|---|
| 1h | 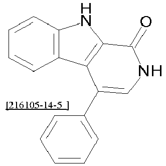 | 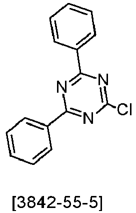 | 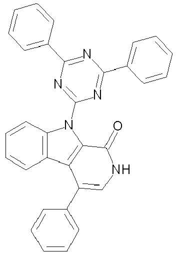 | 61% |
| 2h | 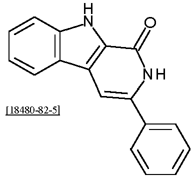 | 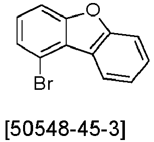 | 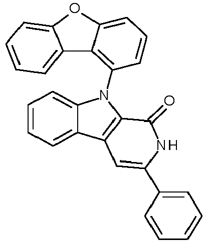 | 64% |
| 3h | 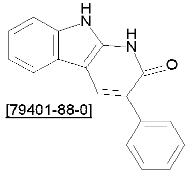 | 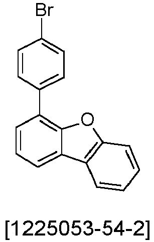 | 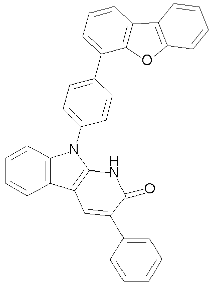 | 67% |
| 4h | 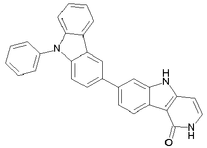 | 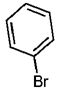 | 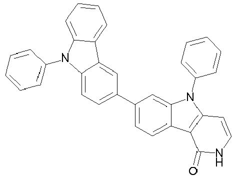 | 67% |
| 5h | 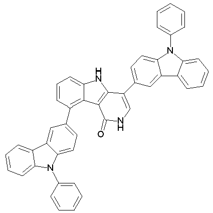 | 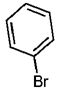 | 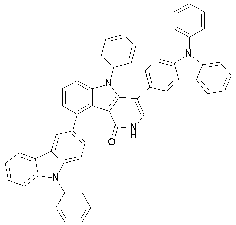 | 70% |
| 6h | 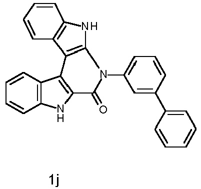 | 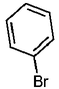 | 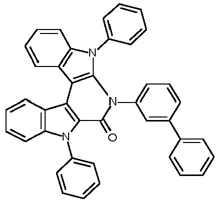 | 68% |
| 7h | 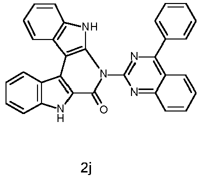 | 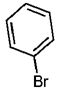 | 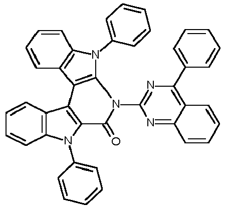 | 66% |
| 8h | 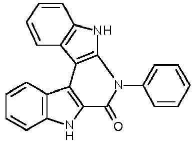 | 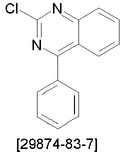 | 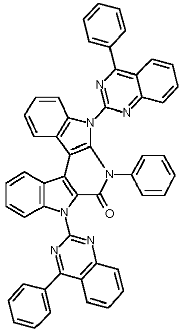 | 68% |
| 9h | 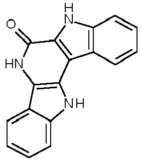 | 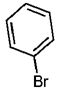 | 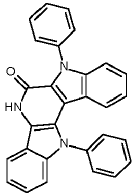 | 71% |
| 10h | 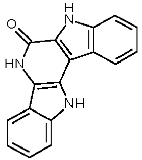 | 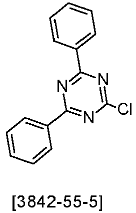 | 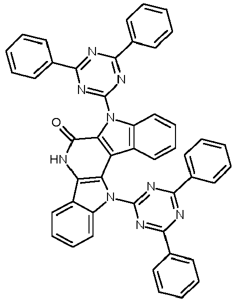 | 65% |
| 11h | 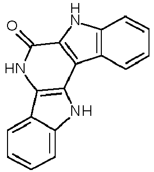 | 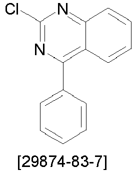 | 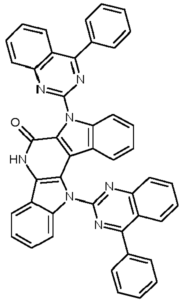 | 59% |
| 12h | 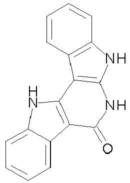 | 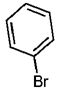 | 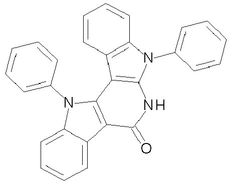 | 70% |
| 13h | 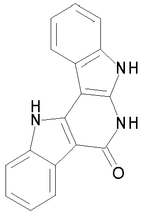 | 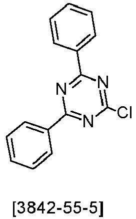 | 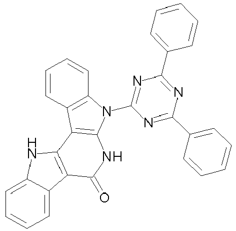 | 56% |
| 14h | 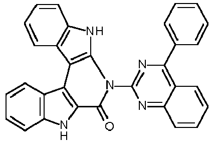 | 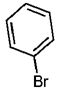 | 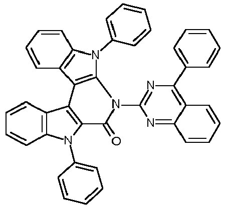 | 71% |
| 15h | 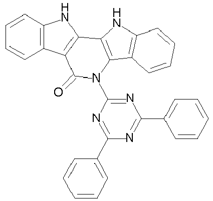 | 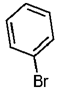 | 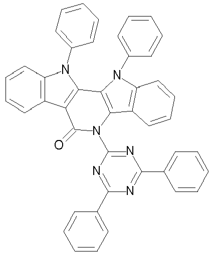 | 77% |
| 16h | 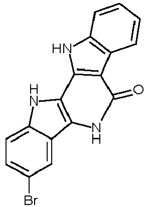 | 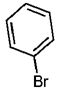 | 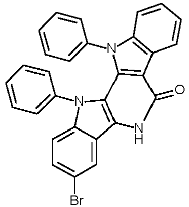 | 70% |
| 17h | 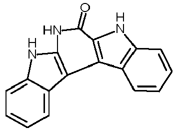 | 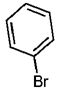 | 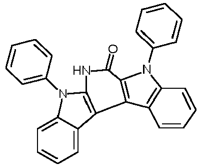 | 76% |
| 18h | 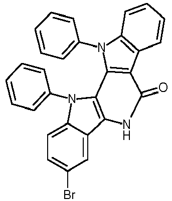 | 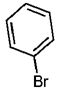 | 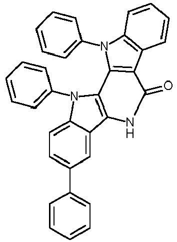 | 80% |

### j) 9-(4,6-Diphenyl-1,3,5-triazin-2-yl)-2-phenyl-5-(9-phenylcarbazol-3-yl)pyrido[3,4-b]indol-1-on

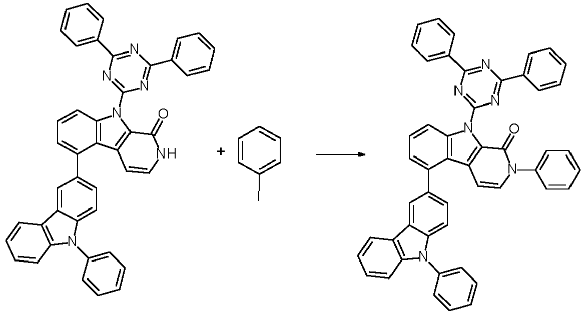

27 g (41 mmol) 9-(4,6-Diphenyl-1,3,5-triazin-2-yl)-5-(9-phenylcarbazol-3-yl)-2H-pyrido[3,4-b]indol-1-on, 61.2 g (85 mmol) 4-lodbenzol, 44.7 g (320 mmol) Kaliumcarbonat, 3 g (16 mmol) Kupfer(I])-iodid und 3.6 g (16 mmol) 1,3-Di-(pyridin-2-yl)-propan-1,3-dion werden in 100 ml DMF 30 h bei 150 °C gerührt. Die Lösung wird mit Wasser verdünnt und zweimal mit Essigester extrahiert. Die vereinigten organischen Phasen werden über Na₂SO₄ getrocknet und einrotiert. Der Rückstand wird chromatographisch (EtOAc/Hexan: 2/3) gereinigt, aus Toluol umkristallisiert und abschließend im Hochvakuum (p = 5 × 10⁻⁵ mbar) sublimiert. Die Reinheit beträgt 99.9%. Die Ausbeute beträgt 21 g (28 mmol); 70% der Theorie.

Analog können folgende Verbindungen erhalten werden:

| | **Edukt 1** | **Edukt 2** | **Produkt** | **Ausbeute** |
|---|---|---|---|---|
| 1j | 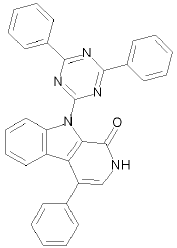 | 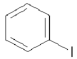 | 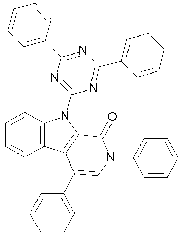 | 70% |
| 2j | 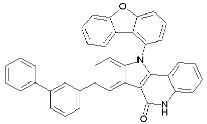 | 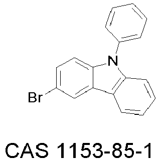 | 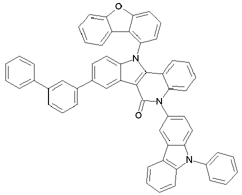 | 74% |
| 3j | 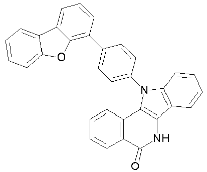 | 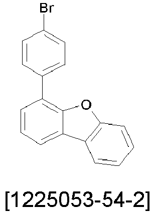 | 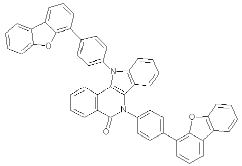 | 73% |
| 4j | 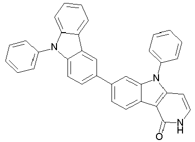 | 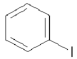 | 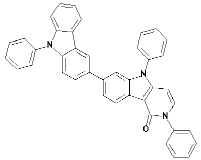 | 75% |
| 5j | 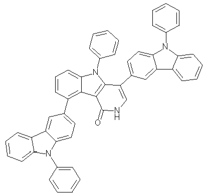 | 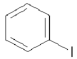 | 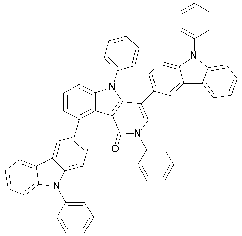 | 79% |
| 6j | 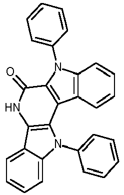 | 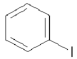 | 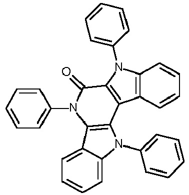 | 79% |
| 7j | 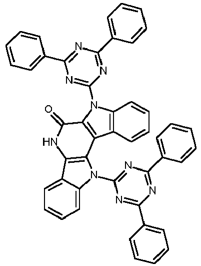 | 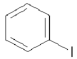 | 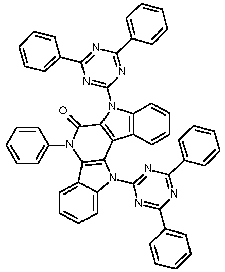 | 69% |
| 8j | 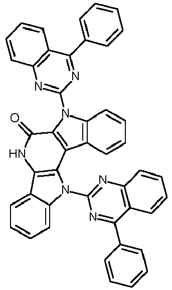 | 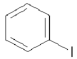 | 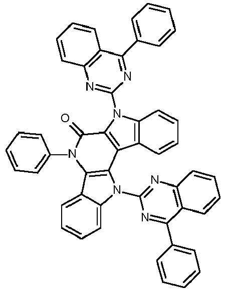 | 78% |
| 9j | 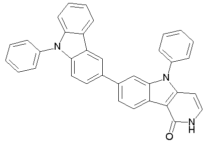 | 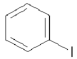 | 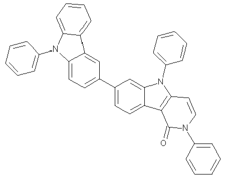 | 76% |
| 10j | 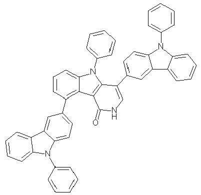 | 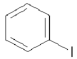 | 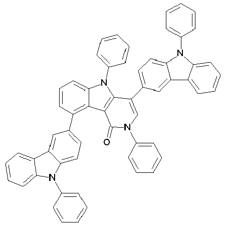 | 68% |
| 11j | 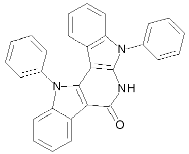 | 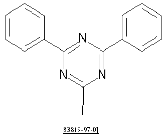 | 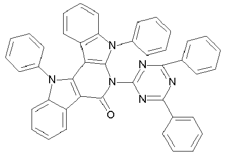 | 71% |
| 12j | 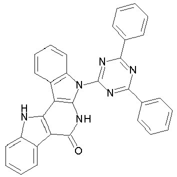 | 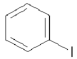 | 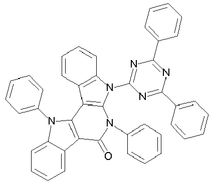 | 69% |
| 13j | 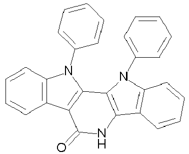 | 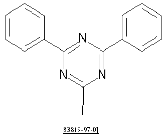 | 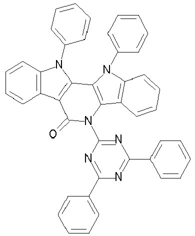 | 80% |
| 14j | 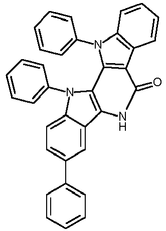 | 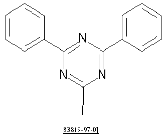 | 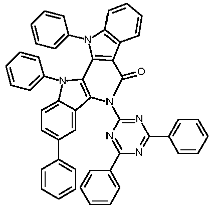 | 61% |
| 15j | 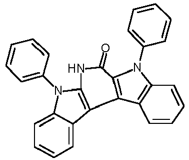 | 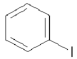 | 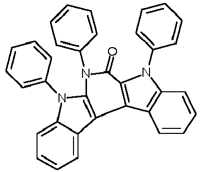 | 79% |
| 16j | 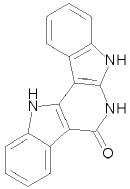 | 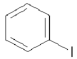 | 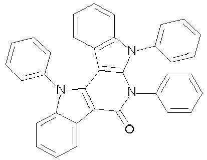 | 76% |

### Herstellung der OLEDs

In den folgenden Beispielen E1 bis E5 (siehe Tabelle 1) wird der Einsatz der erfindungsgemäßen Materialien in OLEDs vorgestellt.

**Vorbehandlung für die Beispiele E1 bis E5:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:EG1:TEG1 (45%:45%:10%) bedeutet hierbei, dass das Material IC1 in einem Volumenanteil von 45%, EG1 in einem Volumenanteil von 45% und TEG1 in einem Volumenanteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet. Die so erhaltenen Ergebnisse sind Tabelle 3 zu entnehmen.

### Verwendung der erfindungsgemäßen Materialien in OLEDs

Die erfindungsgemäßen Verbindungen EG1 bis EG5 werden in den Beispielen E1 bis E5 als Matrixmaterial in der Emissionsschicht von phosphoreszierenden grünen OLEDs eingesetzt.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 230nm | SpMA2 20nm | IC1:EG1:TEG1 (64%:29%:7%) 40nm | ST2 5nm | ST2:LiQ (50%:50%) 30nm | LiQ 1nm |
| E2 | HATCN 5nm | SpMA1 230nm | SpMA2 20nm | EG2:TEG1 (88%:12%) 40nm | ST2 5nm | ST2:LiQ (50%:50%) 30nm | LiQ 1nm |
| E3 | HATCN 5nm | SpMA1 230nm | SpMA2 20nm | IC1:EG3:TEG1 (49%:44%:7%) 40nm | ST2 5nm | ST2:LiQ (50%:50%) 30nm | LiQ 1nm |
| E4 | HATCN 5nm | SpMA1 230nm | SpMA2 20nm | IC1:EG4:TEG1 (49%:44%:7%) 40nm | ST2 5nm | ST2:LiQ (50%:50%) 30nm | LiQ 1nm |
| E5 | HATCN 5nm | SpMA1 230nm | SpMA2 20nm | EG5:IC2:TEG1 (49%:44%:7%) 40nm | ST2 5nm | ST2:LiQ (50%:50%) 30nm | LiQ 1nm |
| E6 | HATCN 5nm | SpMA1 230nm | SpMA2 20nm | EG5:IC3:TEG1 (49%:44%:7%) 40nm | ST2 5nm | ST2:LiQ (50%:50%) 30nm | LiQ 1nm |

**Tabelle 2: Strukturformeln der Materialien für die OLEDs**

| | |
|---|---|
| 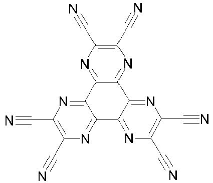 | 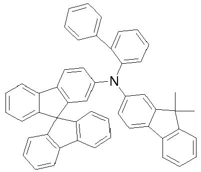 |
| HATCN | SpMA1 |
| 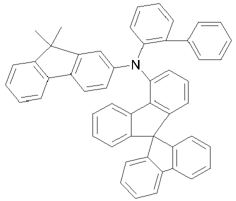 | 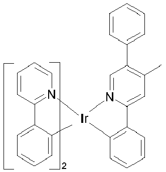 |
| SpMA3 | TEG1 |
| 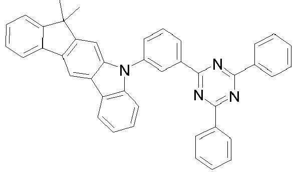 | 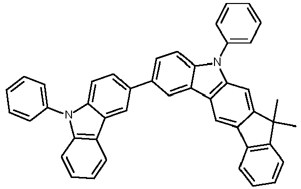 |
| IC1 | IC2 |
| 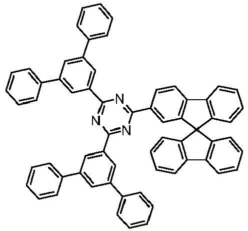 | 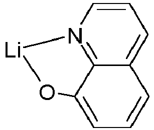 |
| ST2 | LiQ |
| 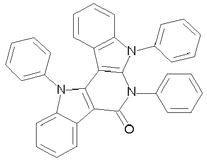 | 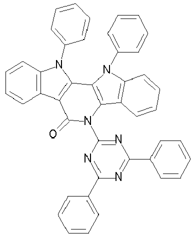 |
| EG1 | EG2 |
| 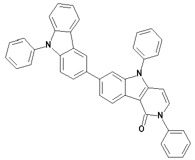 | 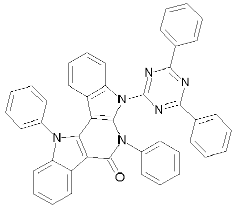 |
| EG3 | EG4 |
| 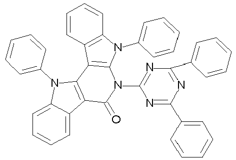 | 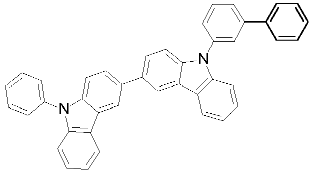 |
| EG5 | IC3 |

**Tabelle 3: Daten der OLEDs**

| Bsp. | U1000 (V) | SE1000 (cd/A) | EQE 1000 (%) | CIE x/y bei 1000 cd/m² |
|---|---|---|---|---|
| E1 | 31 | 69 | 18 | 0.34/0.62 |
| E2 | 3.4 | 72 | 20 | 0.35/0.61 |
| E3 | 33 | 71 | 19 | 0.35/0.62 |
| E4 | 3.2 | 70 | 18 | 0.34/0.61 |
| E5 | 3.6 | 66 | 17 | 0.35/0.61 |
| E6 | 32 | 66 | 21 | 0.35/0.61 |

## Patentansprüche

1. Verbindung gemäß Formel (1), 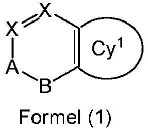 wobei für die verwendeten Symbole gilt:
A, B sind jeweils ausgewählt aus der Gruppe bestehend aus NAr¹, C=O, C=S, C=NR, BR, PR, P(=O)R, SO und SO₂, mit der Maßgabe, dass eines der Symbole A und B für NAr¹ steht und das andere der Symbole A und B für C=O, C=S, C=NR, BR, PR, P(=O)R, SO oder SO₂ steht;
Cy¹ ist zusammen mit den beiden explizit eingezeichneten Kohlenstoffatomen eine Gruppe der folgenden Formel (2), 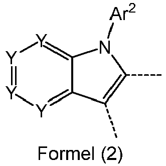 wobei die gestrichelten Bindungen die Verknüpfung dieser Gruppe in der Formel (1) kennzeichnen;
X ist gleich oder verschieden bei jedem Auftreten CR' oder N; oder die beiden Gruppen X stehen für eine Gruppe der folgenden Formel (3), 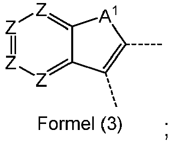
Y, Z ist gleich oder verschieden bei jedem Auftreten CR oder N;
A¹ ist gleich oder verschieden bei jedem Auftreten NAr³, O, S oder C(R)₂;
Ar¹, Ar², Ar³ ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann; dabei kann Ar¹ mit einem benachbarten Rest R` ein Ringsystem bilden;
R, R' ist bei jedem Auftreten gleich oder verschieden H, D, F, Cl, Br, I, N(Ar')₂, N(R¹)₂, OAr', SAr`, CN, NO₂, OR¹, SR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann; dabei können zwei Reste R auch miteinander ein aliphatisches, heteroaliphatisches, aromatisches oder heteroaromatisches Ringsystem bilden; weiterhin können zwei Reste R` auch miteinander ein aliphatisches oder heteroaliphatisches Ringsystem bilden; weiterhin kann R' mit einem benachbarten Rest Ar¹ ein Ringsystem bilden;
Ar' ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
R¹ ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein aliphatisches Ringsystem bilden;
R² ist bei jedem Auftreten gleich oder verschieden H, D, F, CN oder ein aliphatischer, aromatischer oder heteroaromatischer organischer Rest mit 1 bis 20 C-Atomen, in dem auch ein oder mehrere H-Atome durch F ersetzt sein können.

2. Verbindung nach Anspruch 1, ausgewählt aus den Verbindungen der Formeln (4) und (5), 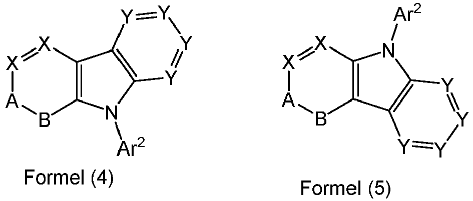 wobei die verwendeten Symbole die in Anspruch 1 genannten Bedeutungen aufweisen.

3. Verbindung nach Anspruch 1 oder 2, **dadurch gekennzeichnet, dass** eine der Gruppen A und B für NAr¹ und die andere der Gruppen A und B für C=O, P(=O)R, BR oder SO₂ steht.

4. Verbindung nach einem oder mehreren der Ansprüche 1 bis 3, ausgewählt aus den Verbindungen der Formeln (4a), (4b), (5a) und (5b), 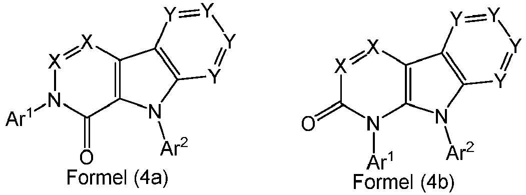 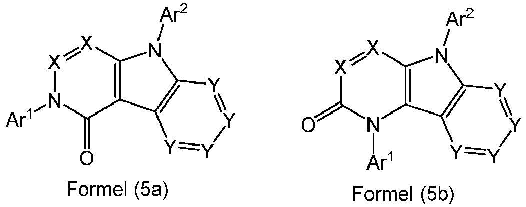 wobei die verwendeten Symbole die in Anspruch 1 genannten Bedeutungen aufweisen.

5. Verbindung nach einem oder mehreren der Ansprüche 1 bis 4, ausgewählt aus den Verbindungen der Formeln (4a-1), (4b-1), (5a-1) und (5b-1), 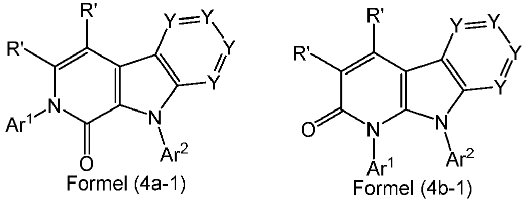 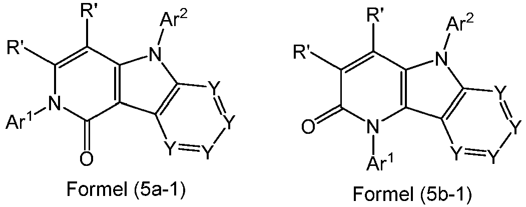 wobei die verwendeten Symbole die in Anspruch 1 genannten Bedeutungen aufweisen.

6. Verbindung nach einem oder mehreren der Ansprüche 1 bis 5, ausgewählt aus den Verbindungen der Formeln (4a-2), (4b-2), (5a-2) und (5b-2), 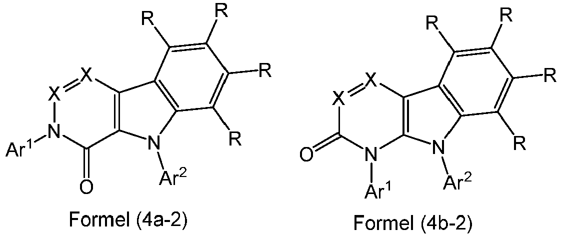 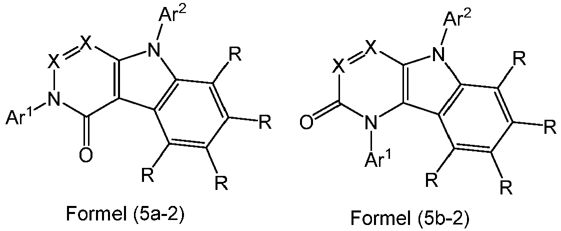 wobei die verwendeten Symbole die Anspruch 1 genannten Bedeutungen aufweisen.

7. Verbindung nach einem oder mehreren der Ansprüche 1 bis 6, ausgewählt aus den Verbindungen der Formeln (4a-3), (4b-3), (5a-3) und (5b-3), 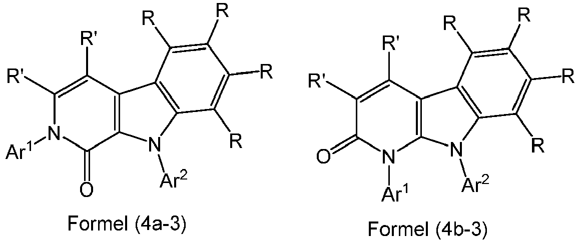 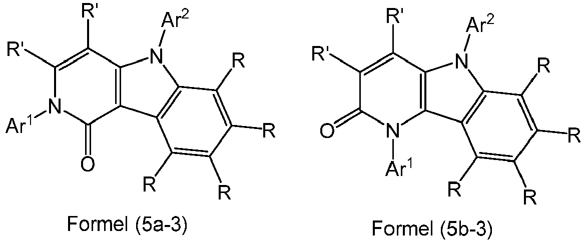 wobei die verwendeten Symbole die in Anspruch 1 genannten Bedeutungen aufweisen.

8. Verbindung nach einem oder mehreren der Ansprüche 1 bis 4, ausgewählt aus den Verbindungen der Formeln (6-2) bis (9-2), 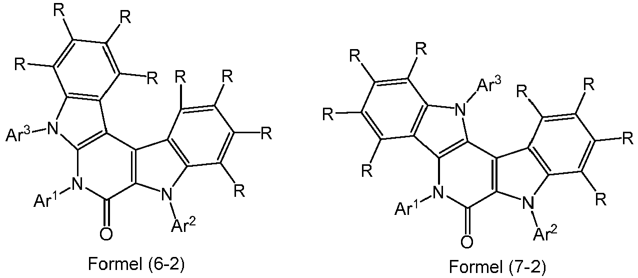 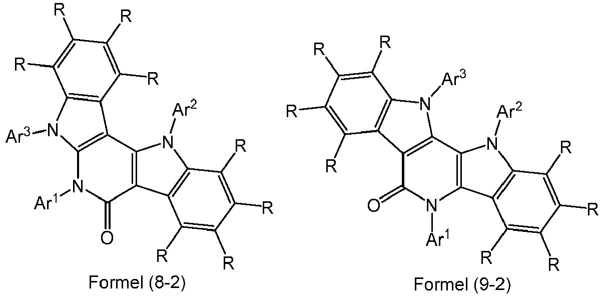 wobei die verwendeten Symbole die in Anspruch 1 genannten Bedeutungen aufweisen.

9. Verbindung nach einem oder mehreren der Ansprüche 1 bis 4, ausgewählt aus den Verbindungen der Formeln (10-1) und (11-1), 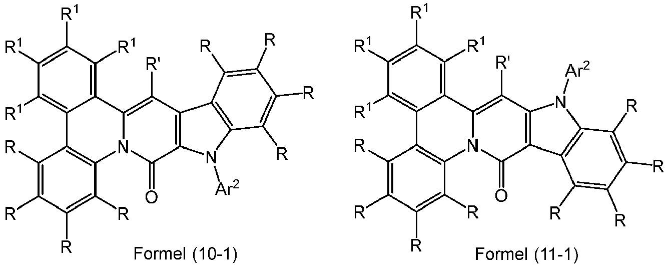 wobei die verwendeten Symbole die in Anspruch 1 genannten Bedeutungen aufweisen.

10. Verfahren zur Herstellung einer Verbindung nach einem oder mehreren der Ansprüche 1 bis 9, **gekennzeichnet durch** die folgenden Schritte:
(1) Synthese des Grundgerüsts, welches noch nicht durch die Gruppen Ar¹, Ar² und gegebenenfalls Ar³ substituiert ist; und
(2) Einführung der Gruppen Ar¹, Ar² und gegebenenfalls Ar³ durch eine C-N-Kupplungsreaktion.

11. Formulierung, enthaltend mindestens eine Verbindung nach einem oder mehreren der Ansprüche 1 bis 9 und mindestens eine weitere Verbindung und/oder mindestens ein Lösemittel.

12. Verwendung einer Verbindung nach einem oder mehreren der Ansprüche 1 bis 9 in einer elektronischen Vorrichtung.

13. Elektronische Vorrichtung enthaltend mindestens eine Verbindung nach einem oder mehreren der Ansprüche 1 bis 9.

14. Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 9 in einer emittierenden Schicht als Matrixmaterial für phosphoreszierende Emitter oder für Emitter, die TADF (thermally activated delayed fluorescence) zeigen, und/oder in einer Elektronentransportschicht und/oder in einer Lochblockierschicht und/oder in einer Lochtransportschicht und/oder in einer Exzitonenblockierschicht eingesetzt wird.

## Claims

1. Compound of the formula (1), 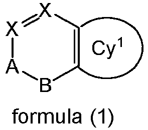 where the following applies to the symbols used:
A, B are in each case selected from the group consisting of NAr¹, C=O, C=S, C=NR, BR, PR, P(=O)R, SO and SO₂, with the proviso that one of the symbols A and B stands for NAr¹ and the other of the symbols A and B stands for C=O, C=S, C=NR, BR, PR, P(=O)R, SO or SO₂;
Cy¹ is, together with the two carbon atoms explicitly drawn in, a group of the following formula (2), 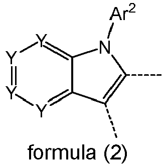 where the dashed bonds denote the linking of this group in the formula (1);
X is, identically or differently on each occurrence, CR' or N; or the two groups X stand for a group of the following formula (3), 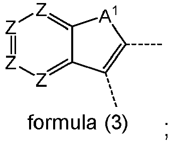
Y, Z are, identically or differently on each occurrence, CR or N;
A¹ is, identically or differently on each occurrence, NAr³, O, S or C(R)₂;
Ar¹, Ar², Ar³ are on each occurrence, identically or differently, an aromatic or heteroaromatic ring system having 5 to 40 aromatic ring atoms, which may be substituted by one or more radicals R; Ar¹ may form a ring system with an adjacent radical R`;
R, R' are on each occurrence, identically or differently, H, D,
F, Cl, Br, I, N(Ar')₂, N(R¹)₂, OAr`, SAr`, CN, NO₂, OR¹, SR¹, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R¹, where one or more non-adjacent CH₂ groups may be replaced by Si(R¹)₂, C=O, NR¹, O, S or CONR¹, or an aromatic or heteroaromatic ring system having 5 to 60 aromatic ring atoms, preferably having 5 to 40 aromatic ring atoms, which may in each case be substituted by one or more radicals R¹; two radicals R may also form an aliphatic, heteroaliphatic, aromatic or heteroaromatic ring system with one another; furthermore, two radicals R' may also form an aliphatic or heteroaliphatic ring system with one another; furthermore, R' may form a ring system with an adjacent radical Ar¹;
Ar' is on each occurrence, identically or differently, an aromatic or heteroaromatic ring system having 5 to 40 aromatic ring atoms, which may be substituted by one or more radicals R¹;
R' is on each occurrence, identically or differently, H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R², where one or more non-adjacent CH₂ groups may be replaced by Si(R²)₂, C=O, NR², O, S or CONR², or an aromatic or heteroaromatic ring system having 5 to 40 aromatic ring atoms, which may in each case be substituted by one or more radicals R²; two or more radicals R' may form an aliphatic ring system with one another;
R² is on each occurrence, identically or differently, H, D, F, CN or an aliphatic, aromatic or heteroaromatic organic radical having 1 to 20 C atoms, in which, in addition, one or more H atoms may be replaced by F.

2. Compound according to Claim 1, selected from the compounds of the formulae (4) and (5), 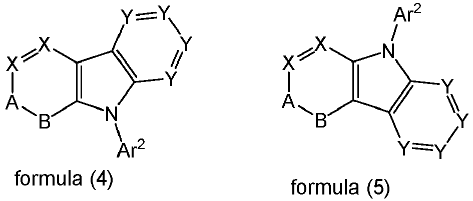 where the symbols used have the meanings given in Claim 1.

3. Compound according to Claim 1 or 2, **characterised in that** one of the groups A and B stands for NAr¹ and the other of the groups A and B stands for C=O, P(=O)R, BR or SO₂.

4. Compound according to one or more of Claims 1 to 3, selected from the compounds of the formulae (4a), (4b), (5a) and (5b), 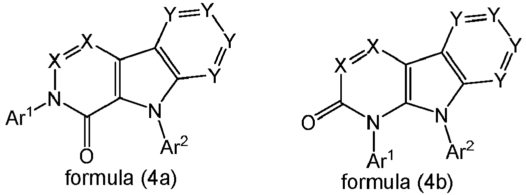 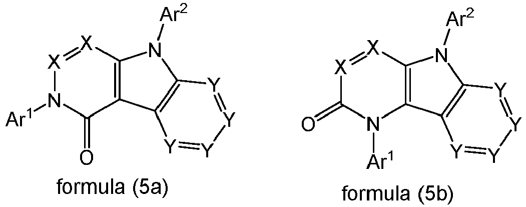 where the symbols used have the meanings given in Claim 1.

5. Compound according to one or more of Claims 1 to 4, selected from the compounds of the formulae (4a-1), (4b-1), (5a-1) and (5b-1), 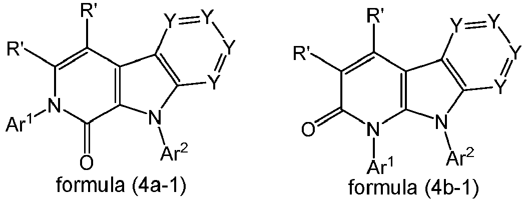 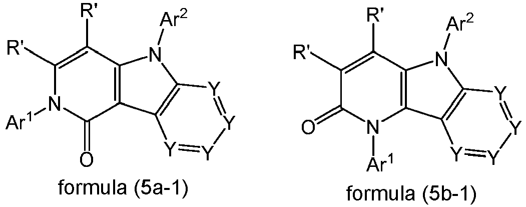 where the symbols used have the meanings given in Claim 1.

6. Compound according to one or more of Claims 1 to 5, selected from the compounds of the formulae (4a-2), (4b-2), (5a-2) and (5b-2), 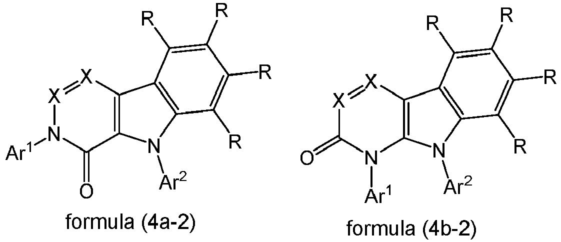 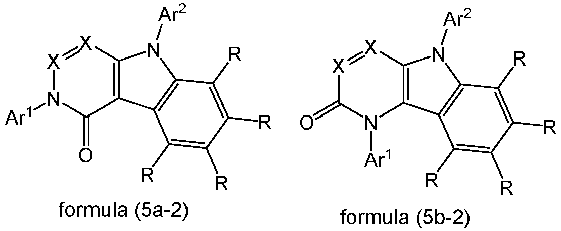 where the symbols used have the meanings given in Claim 1.

7. Compound according to one or more of Claims 1 to 6, selected from the compounds of the formulae (4a-3), (4b-3), (5a-3) and (5b-3), 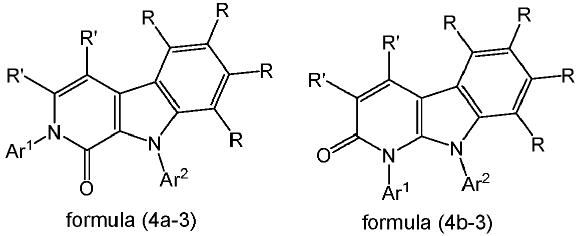 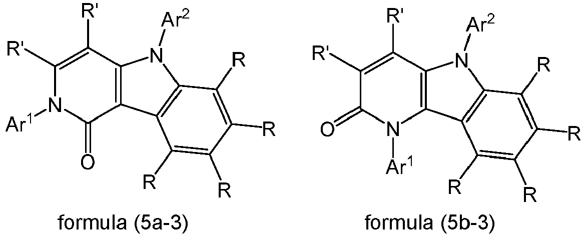 where the symbols used have the meanings given in Claim 1.

8. Compound according to one or more of Claims 1 to 4, selected from the compounds of the formulae (6-2) to (9-2), 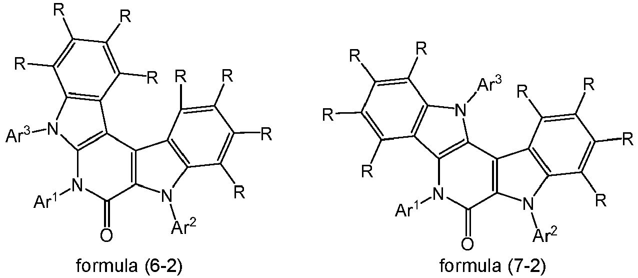 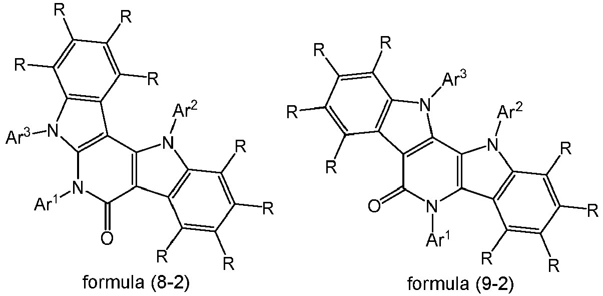 where the symbols used have the meanings given in Claim 1.

9. Compound according to one or more of Claims 1 to 4, selected from the compounds of the formulae (10-1) and (11-1), 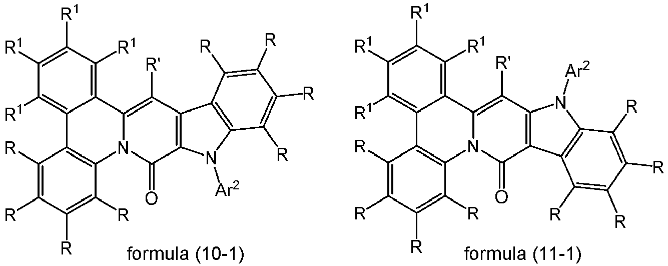 where the symbols used have the meanings given in Claim 1.

10. Process for the preparation of a compound according to one or more of Claims 1 to 9, **characterised by** the following steps:
(1) synthesis of the basic skeleton which is not yet substituted by the groups Ar¹, Ar² and optionally Ar³; and
(2) introduction of the groups Ar¹, Ar² and optionally Ar³ by a C-N coupling reaction.

11. Formulation comprising at least one compound according to one or more of Claims 1 to 9 and at least one further compound and/or at least one solvent.

12. Use of a compound according to one or more of Claims 1 to 9 in an electronic device.

13. Electronic device containing at least one compound according to one or more of Claims 1 to 9.

14. Electronic device according to Claim 13, which is an organic electroluminescent device, **characterised in that** the compound according to one or more of Claims 1 to 9 is employed in an emitting layer as matrix material for phosphorescent emitters or for emitters which exhibit TADF (thermally activated delayed fluorescence), and/or in an electron-transport layer and/or in a hole-blocking layer and/or in a hole-transport layer and/or in an exciton-blocking layer.

## Revendications

1. Composé de formule (1), 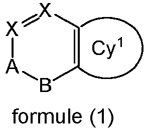 dans laquelle ce qui suit s'applique aux symboles utilisés :
A, B sont, dans chaque cas, choisis dans le groupe constitué par NAr¹, C=O, C=S, C=NR, BR, PR, P(=O)R, SO et SO₂, à condition que l'un parmi les symboles A et B représente NAr¹ et l'autre parmi les symboles A et B représente C=O, C=S, C=NR, BR, PR, P(=O)R, SO ou SO₂ ;
Cy¹ est, conjointement avec les deux atomes de carbone explicitement dessinés, un groupement de formule (2) suivante, 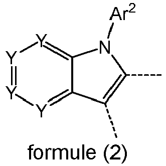 dans laquelle les lignes en pointillés représentent la liaison de ce groupement dans la formule (1) ;
X est, de manière identique ou différente à chaque occurrence, CR' ou N ; ou les deux groupements X représentent un groupement de formule (3) suivante, 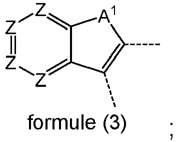
Y, Z sont, de manière identique ou différente à chaque occurrence, CR ou N ;
A' est, de manière identique ou différente à chaque occurrence, NAr³, O, S ou C(R)₂ ;
Ar¹, Ar², Ar³ sont à chaque occurrence, de manière identique ou différente, un noyau aromatique ou hétéroaromatique ayant de 5 à 40 atomes de cycle aromatique, qui peut être substitué par un ou plusieurs radicaux R ; Ar¹ peut former un noyau avec un radical R' adjacent ;
R, R' sont à chaque occurrence, de manière identique ou différente, H, D, F, Cl, Br, I, N(Ar')₂, N(R¹)₂, OAr`, SAr`, CN, NO₂, OR¹, SR¹, C(=O)N(R¹)₂, Si(R¹)₃, B(OR¹)₂, C(=O)R¹, P(=O)(R¹)₂, S(=O)R¹, S(=O)₂R¹, OSO₂R¹, un groupement alkyle à chaîne linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut dans chaque cas être substitué par un ou plusieurs radicaux R¹, où un ou plusieurs groupements CH₂ non adjacents peuvent être remplacés par Si(R¹)₂, C=O, NR¹, O, S ou CONR¹, ou un noyau aromatique ou hétéroaromatique ayant de 5 à 60 atomes de cycle aromatique, préférablement ayant de 5 à 40 atomes de cycle aromatique, qui peut dans chaque cas être substitué par un ou plusieurs radicaux R' ; deux radicaux R peuvent également former un noyau aliphatique, hétéroaliphatique, aromatique ou hétéroaromatique l'un avec l'autre ; en outre, deux radicaux R' peuvent également former un noyau aliphatique ou hétéroaliphatique l'un avec l'autre ; en outre, R' peut former un noyau avec un radical Ar¹ adjacent ;
Ar' est à chaque occurrence, de manière identique ou différente, un noyau aromatique ou hétéroaromatique ayant de 5 à 40 atomes de cycle aromatique, qui peut être substitué par un ou plusieurs radicaux R' ;
R' est à chaque occurrence, de manière identique ou différente, H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², un groupement alkyle à chaîne linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut dans chaque cas être substitué par un ou plusieurs radicaux R², où un ou plusieurs groupements CH₂ non adjacents peuvent être remplacés par Si(R²)₂, C=O, NR², O, S ou CONR², ou un noyau aromatique ou hétéroaromatique ayant de 5 à 40 atomes de cycle aromatique, qui peut dans chaque cas être substitué par un ou plusieurs radicaux R² ; deux, ou plus, radicaux R' peuvent former un noyau aliphatique l'un avec l'autre ;
R² est à chaque occurrence, de manière identique ou différente, H, D, F, CN ou un radical organique aliphatique, aromatique ou hétéroaromatique ayant de 1 à 20 atomes de C, où, de plus, un ou plusieurs atomes de H peuvent être remplacés par F.

2. Composé selon la revendication 1, choisi parmi les composés de formules (4) et (5), 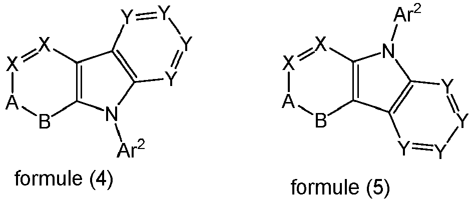 dans lesquelles les symboles utilisés revêtent les significations données selon la revendication 1.

3. Composé selon la revendication 1 ou 2, **caractérisé en ce que** l'un parmi les groupements A et B représente NAr¹ et l'autre parmi les groupements A et B représente C=O, P(=O)R, BR ou SO₂.

4. Composé selon l'une ou plusieurs parmi les revendications 1 à 3, choisi parmi les composés de formules (4a), (4b), (5a) et (5b), 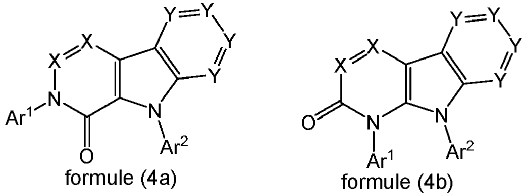 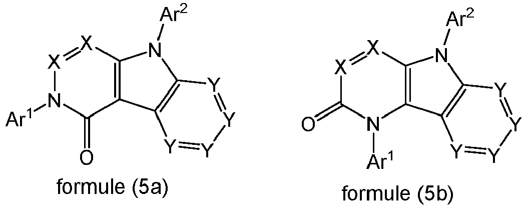 dans lesquelles les symboles utilisés revêtent les significations données selon la revendication 1.

5. Composé selon l'une ou plusieurs parmi les revendications 1 à 4, choisi parmi les composés de formules (4a-1), (4b-1), (5a-1) et (5b-1), 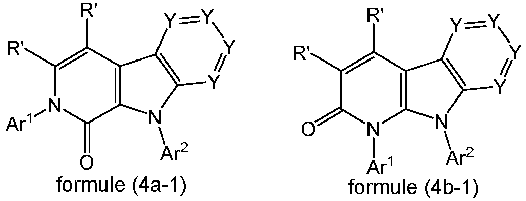 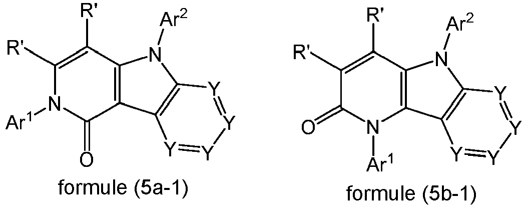 dans lesquelles les symboles utilisés revêtent les significations données selon la revendication 1.

6. Composé selon l'une ou plusieurs parmi les revendications 1 à 5, choisi parmi les composés de formules (4a-2), (4b-2), (5a-2) et (5b-2), 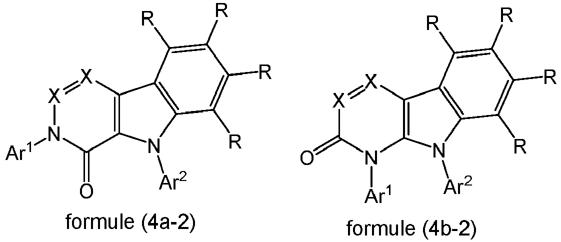 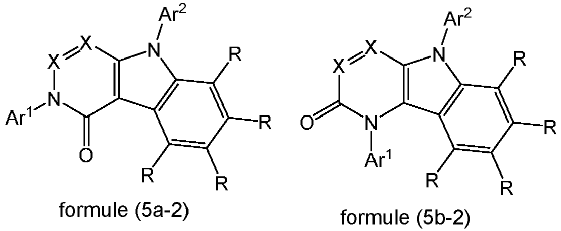 dans lesquelles les symboles utilisés revêtent les significations données selon la revendication 1.

7. Composé selon l'une ou plusieurs parmi les revendications 1 à 6, choisi parmi les composés de formules (4a-3), (4b-3), (5a-3) et (5b-3), 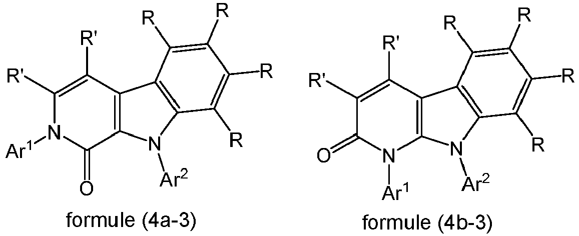 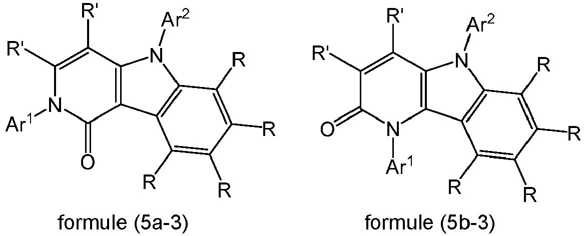 dans lesquelles les symboles utilisés revêtent les significations données selon la revendication 1.

8. Composé selon l'une ou plusieurs parmi les revendications 1 à 4, choisi parmi les composés de formules (6-2) à (9-2), 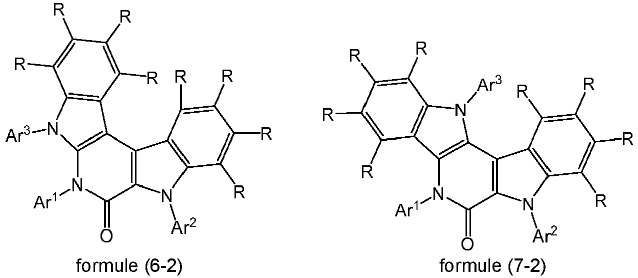 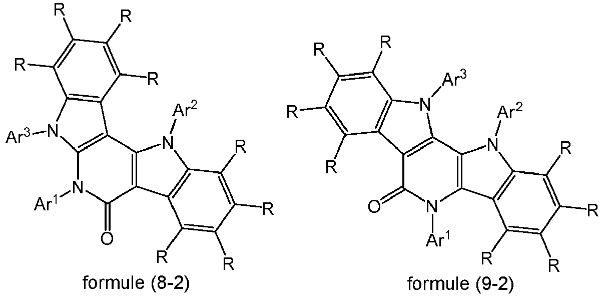 dans lesquelles les symboles utilisés revêtent les significations données selon la revendication 1.

9. Composé selon l'une ou plusieurs parmi les revendications 1 à 4, choisi parmi les composés de formules (10-1) et (11-1), 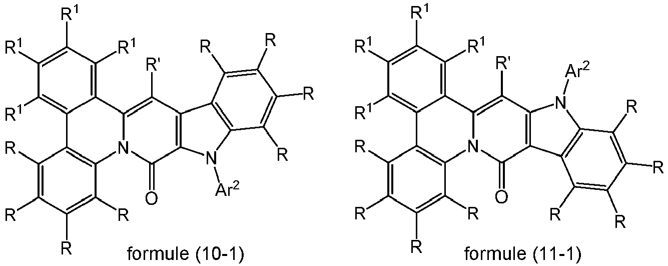 dans lesquelles les symboles utilisés revêtent les significations données selon la revendication 1.

10. Procédé de préparation d'un composé selon l'une ou plusieurs parmi les revendications 1 à 9, **caractérisé par** les étapes suivantes :
(1) la synthèse du squelette basique qui n'est pas encore substitué par les groupements Ar¹, Ar² et éventuellement Ar³ ; et
(2) l'introduction des groupements Ar¹, Ar² et éventuellement Ar³ par une réaction de couplage C-N.

11. Formulation comprenant au moins un composé selon l'une ou plusieurs parmi les revendications 1 à 9 et au moins un autre composé et/ou au moins un solvant.

12. Utilisation d'un composé selon l'une ou plusieurs parmi les revendications 1 à 9, dans un dispositif électronique.

13. Dispositif électronique contenant au moins un composé selon l'une ou plusieurs parmi les revendications 1 à 9.

14. Dispositif électronique selon la revendication 13, qui est un dispositif électroluminescent organique, **caractérisé en ce que** le composé selon l'une ou plusieurs parmi les revendications 1 à 9 est employé dans une couche émettrice comme matériau de matrice pour les émetteurs phosphorescents ou pour les émetteurs présentant une TADF (fluorescence retardée activée thermiquement), et/ou dans une couche de transport d'électrons et/ou dans une couche de blocage de trous et/ou dans une couche de transport de trous et/ou dans une couche de blocage d'excitons.
